# EUROPEAN PATENT APPLICATION

(11) **EP 3 904 331 A1**
(43) Date of publication of application: **03.11.2021**
(21) Application number: 19903140.2
(22) Date of filing: 27.12.2019
(51) Int. Cl.: C07C 211/63, C07C 211/64, C07C 309/13, C07D 207/06, C07D 211/34, C07D 211/62, C07D 217/10, B82Y 15/00, A61K 49/06, C07F 9/38

(54) **NANOPARTICLE, CONTRAST AGENT FOR MAGNETIC RESONANCE IMAGING COMPRISING SAME AND ZWITTERIONIC LIGAND COMPOUND**

(30) Priority: 27.12.2018 JP 2018245927
(71) Applicant: Astellas Pharma Inc., Chuo-ku Tokyo 103-8411 (JP); RIKEN, Wako-shi Saitama 351-0198 (JP); NATIONAL INSTITUTES FOR QUANTUM AND RADIOLOGICAL SCIENCE AND TECHNOLOGY, Inage-ku Chiba-shi Chiba 263-8555 (JP)
(72) Inventor: MIZUTANI, Tsuyoshi, Tokyo 103-8411 (JP); YAMADA, Hiroyoshi, Tokyo 103-8411 (JP); TOYA, Hiroki, Tokyo 103-8411 (JP); FUJIKAWA, Akihiko, Tokyo 103-8411 (JP); YOSHIMURA, Seiji, Tokyo 103-8411 (JP); KIKUCHI, Shigetoshi, Tokyo 103-8411 (JP); MIYAJIMA, Daigo, Wako-shi, Saitama 351-0198 (JP); TAKEUCHI, Toshiaki, Wako-shi, Saitama 351-0198 (JP); AIDA, Takuzo, Wako-shi, Saitama 351-0198 (JP); AOKI, Ichio, Chiba-shi, Chiba 263-8555 (JP)
(74) Representative: Hoffmann Eitle
(86) International application number: PCT/JP2019/051354
(87) International publication number: WO 2020/138389

(57) **Abstract**

Provided is a novel nanoparticle, a contrast agent for magnetic resonance imaging containing the same, and a zwitterionic ligand compound used in production of the nanoparticle. The contrast agent for MRI of the present invention can be suitably used as a contrast agent for MRI in a medical field. The nanoparticle and the zwitterionic ligand compound of the present invention are applicable to various pharmaceutical compositions and the like, including a contrast agent for MRI, and can be used widely in the fields of pharmaceuticals, biotechnology, and the like, including various diagnosis methods and examination reagents.

## Description

### Technical Field

The present invention relates to a novel nanoparticle, a contrast agent for magnetic resonance imaging containing the same, and a zwitterionic ligand compound used for production of the nanoparticle.

### Background Art

Magnetic resonance imaging (MRI), which plays an important role in clinical diagnostic imaging, is an important tool also in the field of biomedical research.

Diagnostic imaging and a contrast agent used for the diagnostic imaging are a technology used for examination of a living organ and tissue. MRI, in particular, is a technology which, on the basis of magnetic properties of atoms, creates an elaborate cross-sectional image and an elaborate three-dimensional image of a tissue and an organ of a living organism with use of high magnetic field strength and a high-frequency radio signal.

MRI is an effective technique for obtaining a two- or three-dimensional image of all water-containing tissues and organs.

When electromagnetic wave pulses enter hydrogen nuclei that are oriented by magnetism in a target tissue, the hydrogen nuclei cause nuclear magnetic resonance and then return signals as a result of relaxation of protons. On the basis of a slight difference between signals from various tissues, MRI can identify an organ and indicate a potential contrast between a benign tissue and a malignant tissue. MRI is useful for detection of a tumor, an inflammation, bleeding, an edema, and the like.

Note that a "contrast agent for MRI" refers to a drug which enables detection of a lesion area or examination of a blood flow in a blood vessel, a function of each organ, and the like, by (i) changing relaxation times (T₁, T₂) of water in a living organism mainly by shortening the relaxation times (T₁, T₂) and (ii) thus enhancing a contrast between different tissues.

The contrast agent for MRI is expected to have the following properties: that the contrast agent exhibits a contrast effect quickly after administration; that the contrast agent has no adverse effect on a living organism; and that the whole contrast agent is eliminated from the living organism. The contrast agent for MRI can be distributed in blood and extracellular fluid by, for example, intravenous administration. A half-life of the contrast agent in blood is preferably within 3 hours, and the contrast agent is excreted to urine via the kidney more preferably within 2 hours. The contrast agent distributed in the extracellular fluid is in itself not directly imaged by MRI. The contrast agent promotes relaxation of protons in tissues in the area in which the contrast agent has been distributed. This is mainly called a T₁-shortening effect, and allows the contrast agent to exhibit a contrast effect in a T₁-weighted image (signals are enhanced). The contrast agent causes a change in relaxation time of a tissue occupied by the contrast agent.

In a case where a concentration of the contrast agent is increased to a certain level or higher, the signal is then attenuated by T₂- and T₂*-shortening effects. As such, an optimum concentration for allowing signal intensity to be increased varies depending on the purpose of performing the imaging.

Degrees of T₁- and T₂-relaxation shortening effects in a magnetic body, i.e., efficiencies in shortening relaxation times of protons are represented as relaxation rate (R). A relaxation rate R₁ and a relaxation rate R₂ are represented as a reciprocal of a longitudinal relaxation time T₁ and a reciprocal of a transverse relaxation time T₂, respectively, of MRI (R₁ = 1/T₁, R₂ = 1/T₂). A relaxation rate per unit concentration is represented as relaxivity (r). Longitudinal relaxivity is represented as r₁, and transverse relaxivity is represented as r₂. An R₁/R₂ ratio and an r₁/r₂ ratio are each used as a parameter for evaluating a relaxivity of a contrast agent for MRI.

In particular, a contrast agent which utilizes T₁ relaxation and is used for the purpose of enhancing signals on a T₁-weighted image is referred to as a T₁-shortening contrast agent or a positive contrast agent. The positive contrast agent causes a signal increase in tissues occupied by the positive contrast agent. A contrast agent which utilizes T₂ relaxation and is used for the purpose of attenuating signals on a T₂-weighted image is referred to as a T₂-shortening contrast agent or a negative contrast agent. The negative contrast agent causes a signal decrease in tissues occupied by the negative contrast agent. Ti-weighted MRI and T₂-weighted MRI are imaging methods commonly used in medical diagnoses. The positive contrast agent in T₁-weighted MRI is highly useful in diagnosis because, as compared with the negative contrast agent, the positive contrast agent does not cause tissue loss due to signal decrease and can improve the contrast of lesion without loss of normal tissue information, therefore the use of the positive contrast agent in imaging diagnosis is indispensable.

In particular, an r₁/r₂ ratio of a contrast agent is an important value for evaluation of the positive contrast agent. A high r₁/r₂ ratio of a positive contrast agent provides a T₁-weighted MR image with good contrast.

A gadolinium (Gd)-based chelate compound can be clinically used as a positive contrast agent, and exhibits excellent T₁ contrast due to high r₁ and low r₂ (i.e., a high r_{1/}r₂ ratio). However, Gd-based compounds are known to have a severe toxicity to an elderly person and a patient with low excretion ability of the kidney (e.g., a patient with renal failure).

Iron oxide-based compounds, on the other hand, have an extremely low toxicity as compared with the Gd-based compounds. As such, research and development are being conducted on iron oxide-based nanoparticles as an alternative material to Gd, which is the current mainstream in the market (Non-patent Literature 1).

So far, research and development have been conducted on nanoparticles to be applied to medical uses (e.g., for diagnosis, treatment, and the like). As an embodiment of a nanoparticle to be applied to a living organism, there is known a nanoparticle including (i) a core particle consisting of a metal material and (ii) a molecule of various kinds (such as a polymer) with which a surface of the core particle is coated. For example, there have been reported (i) a method for producing iron oxide particles (ESIONs) having a size of 4 nm or less and (ii) a positive contrast agent for MRI which positive contrast agent contains nanoparticles including (a) ESIONs and (b) polyethylene glycol phosphate (PO-PEG) with which the ESIONs are coated (Non-patent Literature 2). There has also been reported a nanoparticle having a structure in which zwitterionic dopamine sulfonate (ZDS) is bound to a surface of an iron oxide nanoparticle serving as a core particle (Non-patent Literature 3 and Patent Literature 1). Properties of such nanoparticles (ZDS-SPIONs) when used as a positive contrast agent have also been reported (Patent Literature 2 and Non-patent Literature 4).

### Citation list

### [Patent Literatures]

[Patent Literature 1]
   International Publication No. WO2013/090601 (Publication Date: June 20, 2013)
[Patent Literature 2]
   International Publication No. WO2016/044068 (Publication Date: March 24, 2016)

### [Non-patent Literatures]

[Non-patent Literature 1]
   Corot et al., Advanced Drug Delivery Reviews, 58, 1471-1504, 2006
[Non-patent Literature 2] Byung Hyo Kim et al., J Am. Chem. Sci., 133, 12624-12631, 2011
[Non-patent Literature 3]
   He Wei et al., Integr. Biol., 5, 108-114, 2013
[Non-patent Literature 4]
   He Wei et al., Proc. Natr. Acad. Sci., 114(9), 2325-2330, 2017

### Summary of Invention

### Technical Problem

There is still a demand for (i) a novel nanoparticle that sufficiently meets the following conditions: exhibiting a behavioral stability in a living organism while having an excellent positive contrast ability (i.e., high r_{1/}r₂); having a low toxicity to a living organism; and having a good storage stability and (ii) a ligand compound for coating the nanoparticle. Further, there is a need for development of a contrast agent for magnetic resonance imaging containing the nanoparticle.

### Solution to Problem

In order to solve the above problem, the present invention includes in its scope any one embodiment below.

Note that, unless otherwise stated, when a symbol in a certain chemical formula in this specification is also used in another chemical formula, the same symbol indicates the same meaning.

### <1>

A nanoparticle including: at least one zwitterionic ligand represented by a formula (I); and a metal particle containing iron oxide, the at least one zwitterionic ligand being coordinately bound to the metal particle: 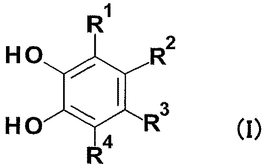 where
one of R¹ and R² is a group represented by a formula (a) or a formula (b), and the other of R¹ and R² is H, lower alkyl, -O- lower alkyl, or halogen, 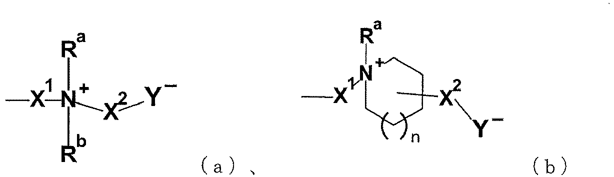
X¹ is a bond or methylene, or X¹ is optionally ethylene when R¹ is a group represented by the formula (a),
X² is C₁₋₅ alkylene that is optionally substituted with OH or is -C₁₋₂ alkylene-O-C₁₋₃ alkylene-, or X² is optionally a bond when R¹ is a group represented by the formula (b),
R^{a} and R^{b} are the same as or different from each other and represent C₁₋₃ alkyl or -C₁₋₃ alkylene-O-C₁₋₂ alkyl, or R^{a} and R^{b} form a 5- or 6-membered nitrogen-containing saturated heterocycle together with a quaternary nitrogen atom to which R^{a} and R^{b} are bound,
Y⁻ is SO₃⁻, HPO₃⁻, or CO₂⁻,
R³ and R⁴ are the same as or different from each other and represent H, C₁₋₃ alkyl, -O-C₁₋₃ alkyl, or halogen, n is an integer of 0 to 2,
   and,
   i) when R¹ is a group represented by the formula (a) and X¹ is methylene, R² optionally forms ethylene together with R^{a} or R^{b},
   ii) when R¹ is a group represented by the formula (a) and X¹ is ethylene, R² optionally forms methylene together with R^{a} or R^{b}, and
   iii) when R² is a group represented by the formula (a) and X¹ is methylene, R³ optionally forms ethylene together with R^{a} or R^{b},
provided that, when R² is a group represented by the formula (a), R^{a} and R^{b} are methyl, X¹ is a bond, X² is C₁₋₄ alkylene, and R¹, R³ and R⁴ are H, Y⁻ is HPO₃⁻ or CO₂⁻.

### <2>

A compound represented by the following formula (I) or a salt thereof: 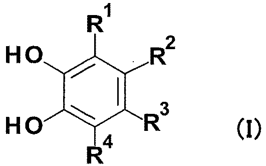 where
one of R¹ and R² is a group represented by a formula (a) or a formula (b) below, and the other of R¹ and R² is H, lower alkyl, -O- lower alkyl, or halogen, 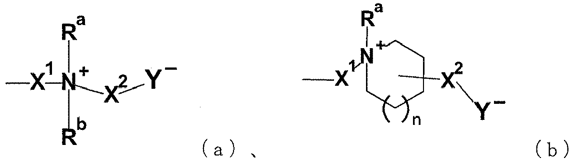
X¹ is a bond or methylene, or X¹ is optionally ethylene when R¹ is a group represented by the formula (a),
X² is C₁₋₅ alkylene that is optionally substituted with OH or is -C₁₋₂ alkylene-O-C₁₋₃ alkylene-, or X² is optionally a bond when R¹ is a group represented by the formula (b),
R^{a} and R^{b} are the same as or different from each other and represent C₁₋₃ alkyl or -C₁₋₃ alkylene-O-C₁₋₂ alkyl, or R^{a} and R^{b} form a 5- or 6-membered nitrogen-containing saturated heterocycle together with a quaternary nitrogen atom to which R^{a} and R^{b} are bound,
Y⁻ is SO₃⁻, HPO₃⁻, or CO₂⁻,
R³ and R⁴ are the same as or different from each other and represent H, C₁₋₃ alkyl, -O-C₁₋₃ alkyl, or halogen,
n is an integer of 0 to 2,
   and,
   i) when R¹ is a group represented by the formula (a) and X¹ is methylene, R² optionally forms ethylene together with R^{a} or R^{b},
   ii) when R¹ is a group represented by the formula (a) and X¹ is ethylene, R² optionally forms methylene together with R^{a} or R^{b}, and
   iii) when R² is a group represented by the formula (a) and X¹ is methylene, R³ optionally forms ethylene together with R^{a} or R^{b},
provided that, when R² is a group represented by the formula (a), R^{a} and R^{b} are methyl, X¹ is a bond, X² is C₁₋₄ alkylene, and R¹, R³ and R⁴ are H, Y⁻ is HPO₃⁻ or CO₂⁻.

### Advantageous Effects of Invention

The present invention is expected to bring about an effect of providing a novel nanoparticle having good positive contrast ability and no cytotoxicity and an effect of providing a contrast agent for magnetic resonance imaging containing the nanoparticle.

### Brief Description of Drawings

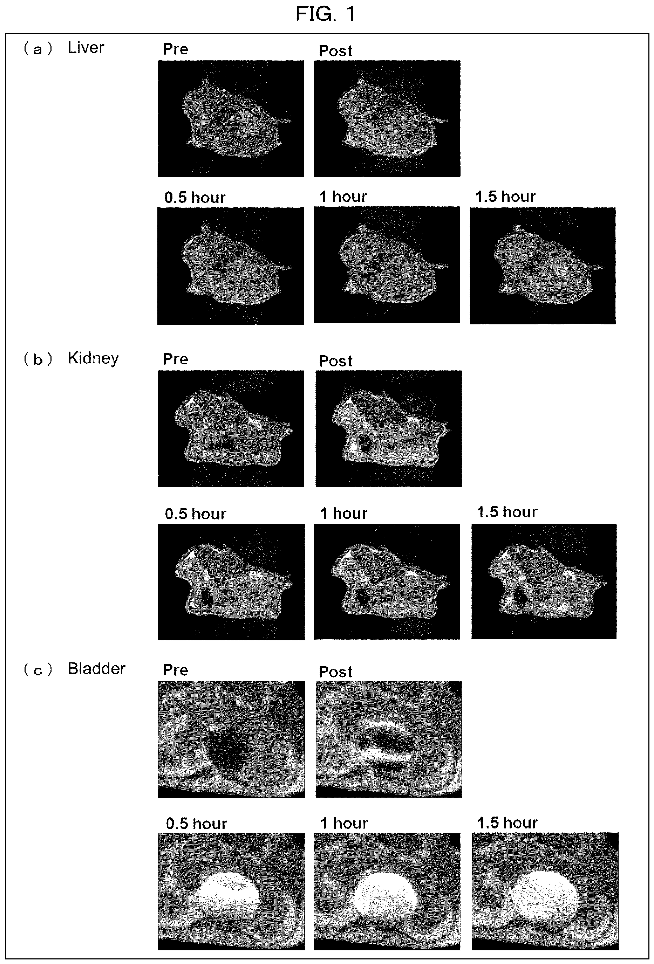

(a) of Fig. 1 shows images of a liver of a mouse to which a contrast agent containing 3K purified particles of Example 6 was administered, the images being obtained as a result of T₁-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).
(b) of Fig. 1 shows images of a kidney of a mouse to which the contrast agent containing 3K purified particles of Example 6 was administered, the images being obtained as a result of Ti-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).
(c) of Fig. 1 shows images of a bladder of a mouse to which the contrast agent containing 3K purified particles of Example 6 was administered, the images being obtained as a result of Ti-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).

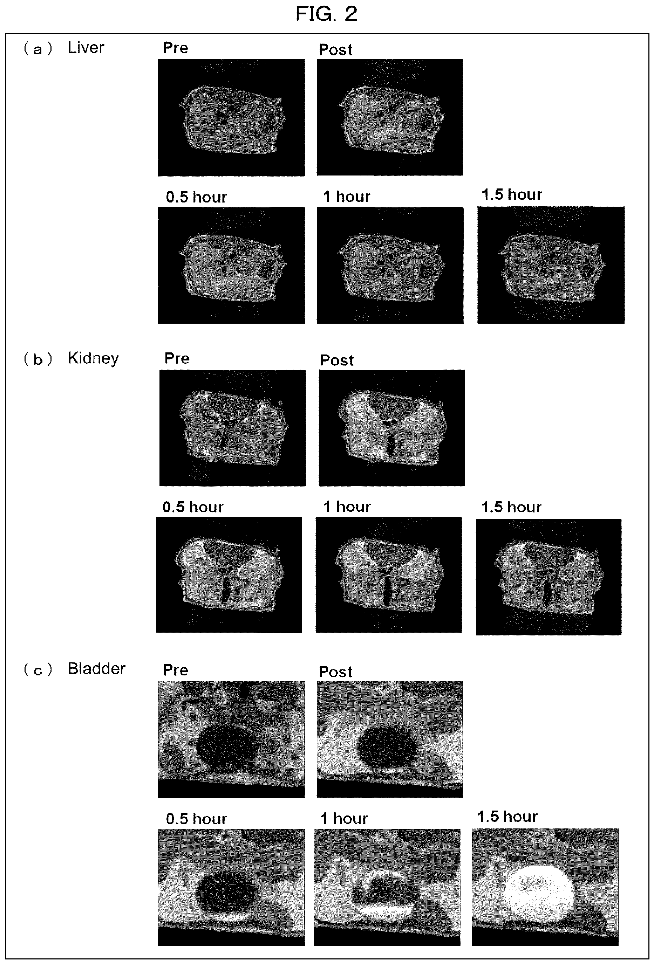

(a) of Fig. 2 shows images of a liver of a mouse to which a contrast agent containing 10K purified particles of Example 6 was administered, the images being obtained as a result of T₁-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).
(b) of Fig. 2 shows images of a kidney of a mouse to which the contrast agent containing 10K purified particles of Example 6 was administered, the images being obtained as a result of Ti-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).
(c) of Fig. 2 shows images of a bladder of a mouse to which the contrast agent containing 10K purified particles of Example 6 was administered, the images being obtained as a result of MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).

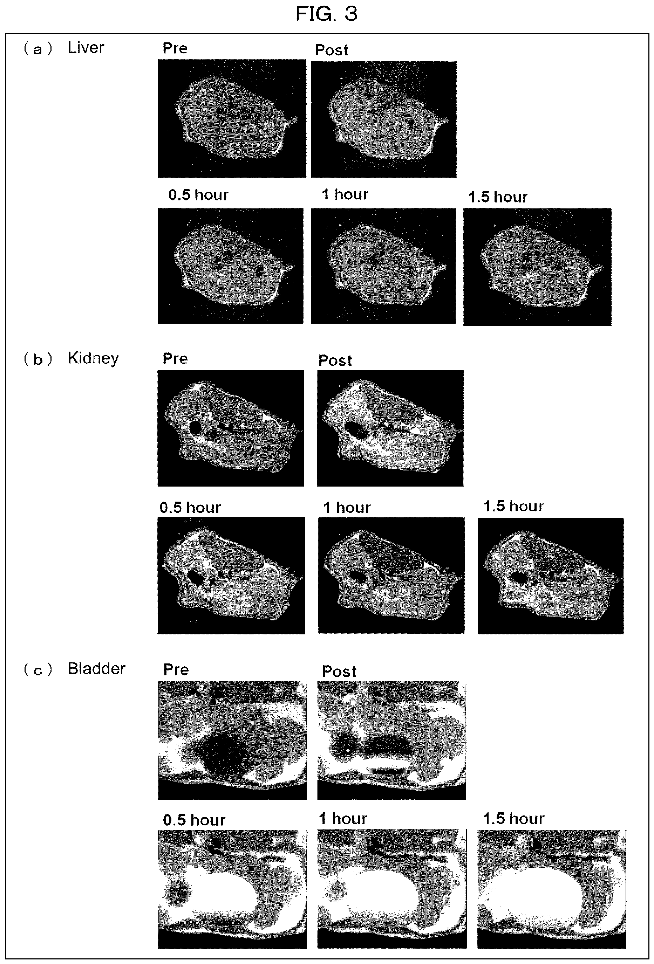

(a) of Fig. 3 shows images of a liver of a mouse to which a contrast agent containing 3K purified particles of Example 7 was administered, the images being obtained as a result of T₁-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).
(b) of Fig. 3 shows images of a kidney of a mouse to which the contrast agent containing 3K purified particles of Example 7 was administered, the images being obtained as a result of Ti-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).
(c) of Fig. 3 shows images of a bladder of a mouse to which the contrast agent containing 3K purified particles of Example 7 was administered, the images being obtained as a result of Ti-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).

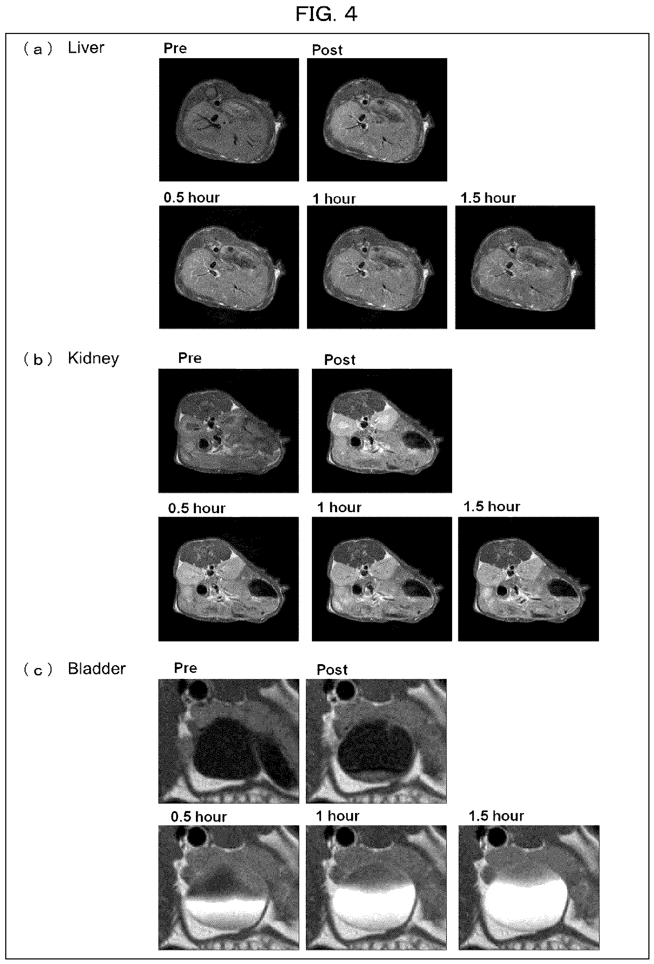

(a) of Fig. 4 shows images of a li17.9ver of a mouse to which a contrast agent containing 10K purified particles of Example 7 was administered, the images being obtained as a result of T₁-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).
(b) of Fig. 4 shows images of a kidney of a mouse to which the contrast agent containing 10K purified particles of Example 7 was administered, the images being obtained as a result of Ti-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).
(c) of Fig. 4 shows images of a bladder of a mouse to which the contrast agent containing 10K purified particles of Example 7 was administered, the images being obtained as a result of Ti-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).

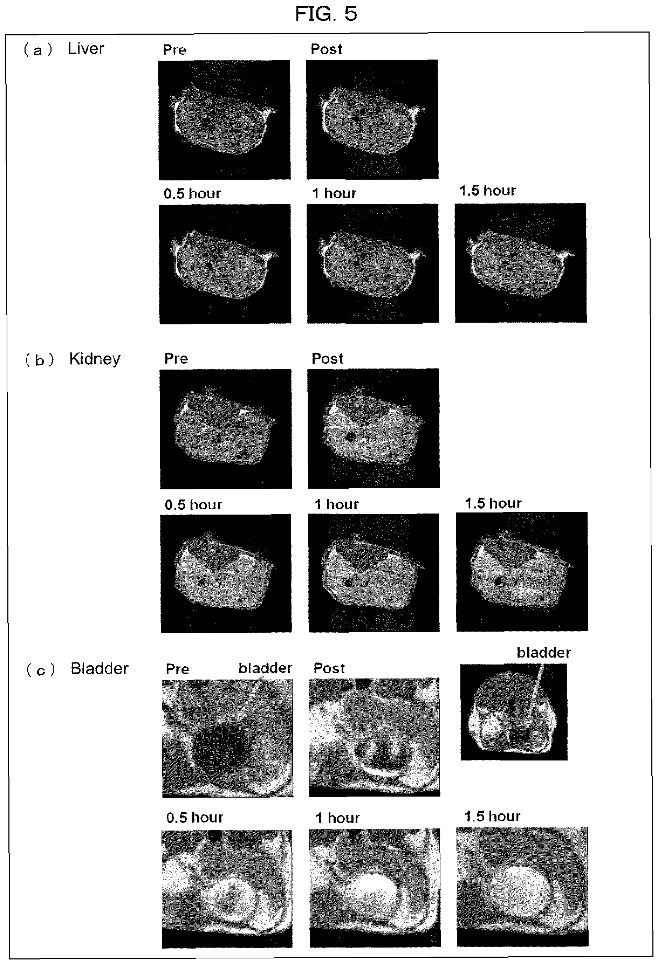

(a) of Fig. 5 shows images of a liver of a mouse to which a contrast agent containing 3K purified particles of Example 25 was administered, the images being obtained as a result of Ti-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).
(b) of Fig. 5 shows images of a kidney of a mouse to which the contrast agent containing 3K purified particles of Example 25 was administered, the images being obtained as a result of Ti-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).
(c) of Fig. 5 shows images of a bladder of a mouse to which the contrast agent containing 3K purified particles of Example 25 was administered, the images being obtained as a result of T₁-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).

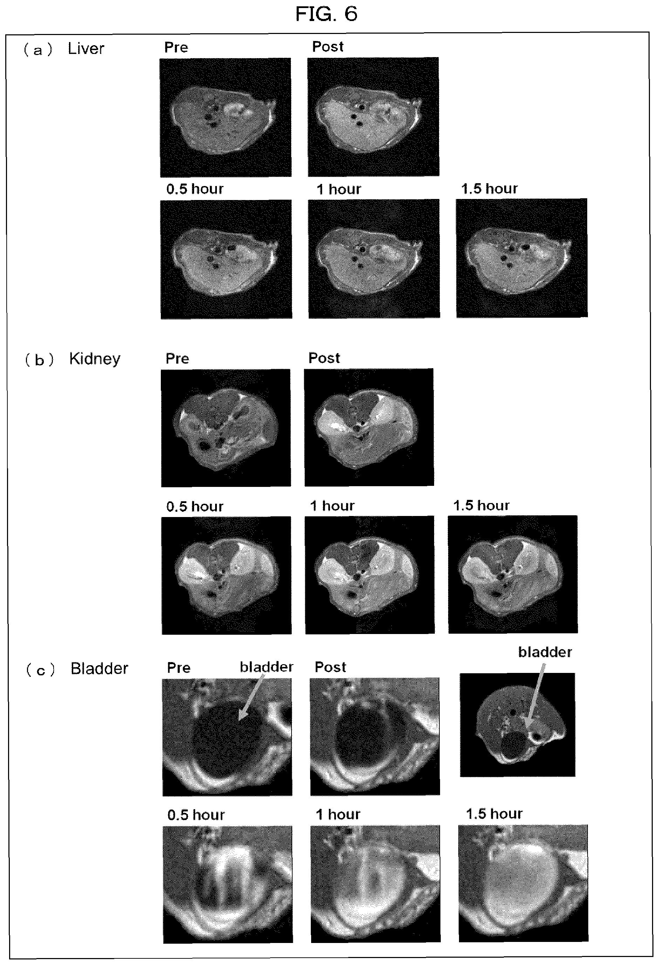

(a) of Fig. 6 shows images of a liver of a mouse to which a contrast agent containing 10K purified particles of Example 25 was administered, the images being obtained as a result of T₁-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).
(b) of Fig. 6 shows images of a kidney of a mouse to which the contrast agent containing 10K purified particles of Example 25 was administered, the images being obtained as a result of T₁-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).
(c) of Fig. 6 shows images of a bladder of a mouse to which the contrast agent containing 10K purified particles of Example 25 was administered, the images being obtained as a result of T₁-weighted MRI measured over time, respectively at the following timings: prior to the administration (pre), immediately after the administration (post), 0.5 hours after the administration (0.5 hour), 1 hour after the administration (1 hour), and 1.5 hours after the administration (1.5 hour).

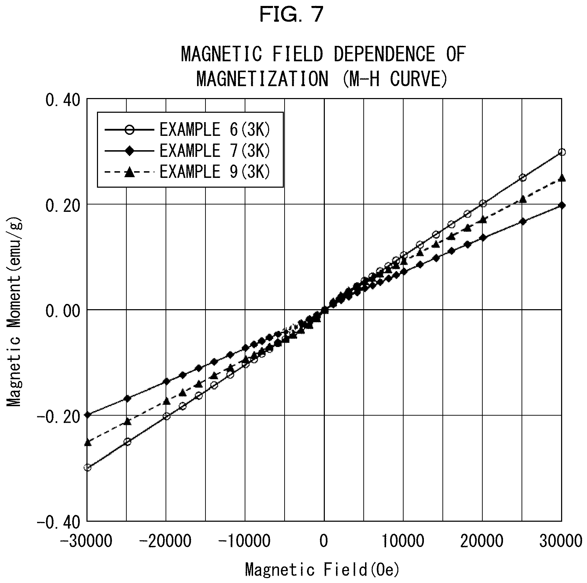

Fig. 7 shows magnetic field dependencies at 300K magnetization of 3K purified particles of Examples 6, 7 and 9. This graph is a plot where the horizontal axis indicates an applied magnetic field and the vertical axis indicates magnetization per weight.

### Description of Embodiments

The description below deals with an embodiment of the present invention in detail.

### [Definitions of Terms]

The term "lower alkyl" refers to alkyls having 1 to 6 linear or branched carbons (hereinafter abbreviated as "C₁₋₆"), such as methyl, ethyl, n-propyl, isopropyl, n-butyl, isobutyl, sec-butyl, tert-butyl, n-pentyl, and n-hexyl and the like. As another embodiment, the lower alkyl is C₁₋₄ alkyl, and as still another embodiment, the lower alkyl is C₁₋₃ alkyl, and as still another embodiment, the lower alkyl is methyl, ethyl, or n-propyl, and as still another embodiment, the lower alkyl is methyl. As one embodiment, "C₁₋₃ alkyl" is methyl, ethyl or n-propyl, and as one embodiment, "C₁₋₃ alkyl" is methyl.

"C₁₋₅ alkylene" is linear or branched C₁₋₅ alkylene, such as methylene, ethylene, trimethylene, tetramethylene, pentamethylene, propylene, butylene, methylmethylene, ethylethylene, 1,1-dimethylethylene, 2,2-dimethylethylene, 1,2-dimethylethylene, or 1-methylbutylene, and the like. As one embodiment, C₁₋₅ alkylene is C₁₋₃ alkylene, as another embodiment, C₁₋₅ alkylene is C₁₋₂ alkylene, and as still another embodiment, C₁₋₅ alkylene is methylene, ethylene, trimethylene, propylene or butylene. Each of "C₁₋₅ alkylene" and "C₁₋₄ alkylene" is C₁₋₃ or C₁₋₂ alkylene and is, as one embodiment, methylene or ethylene.

A "5- or 6-membered nitrogen-containing saturated heterocycle", which is formed by R^{a} and R^{b} together with a quaternary nitrogen atom to which R^{a} and R^{b} are bound, is a non-aromatic heterocycle having 5 or 6 ring members and containing a quaternary nitrogen atom as a ring constituent atom. That is, the "5- or 6-membered nitrogen-containing saturated heterocycle" is a pyrrolidine ring or a piperidine ring. As one embodiment, the 5- or 6-membered nitrogen-containing saturated heterocycle is a pyrrolidine ring which contains a quaternary nitrogen atom as a ring constituent atom.

The term "halogen" means F, C1, Br, and I. As another embodiment, halogen is F and C1, as still another embodiment, halogen is F, and as still another embodiment, halogen is C1.

In this specification, the term "nanoparticle" refers to a particle having a particle diameter in an order of nanometers or smaller. The term "nanoparticle" refers to a particle having a particle diameter of less than 100 nm, as another embodiment, less than 10 nm, as still another embodiment, less than 5 nm, as still another embodiment, less than 3 nm. As still another embodiment, the term "nanoparticle" refers to a particle having a particle diameter of less than 1 nm. Details of the particle diameter will be discussed later in a section of particle diameter.

In this specification, the term "cluster" refers to an aggregate in which a plurality of identical or different particles are collected and formed into a single lump. As another embodiment, the term "cluster" refers to an aggregate of zwitterionic ligands and metal fine particles to which the zwitterionic ligands are coordinately bound.

The term "zwitterionic ligand" or "zwitterionic ligand compound" refers to a compound which (i) has, in its molecule, a group carrying both a positive charge and a negative charge, (ii) has another group capable of forming a coordinate bond with a metal atom on a surface of a metal particle and (iii) is used as a modifier on the surface of the metal particle for allowing the metal particle to be stably dispersed in water. As used herein, the term "zwitterionic ligand" or "zwitterionic ligand compound" refers to (i) a case in which the compound has not been coordinately bound to a surface of a metal particle and/or (ii) a case in which the compound has a molecular structure in which the compound has been coordinately bound to a surface of a metal particle.

As used herein, the term "subject" refers to a given organism to which a contrast agent for MRI, a nanoparticle, or a composition containing the nanoparticle of the present invention can be administered for the purpose of, for example, experiment, diagnosis, and/or treatment. As an example, the subject is a human.

The following description will discuss a nanoparticle, a contrast agent for MRI, and a zwitterionic ligand compound in accordance with the present invention.

### [1. Nanoparticle]

The nanoparticle in accordance with the present invention is a particle containing a metal particle containing iron oxide that the at least one zwitterionic ligand which is represented by the above formula (I) is being coordinately bound to the metal particle. As another embodiment, the zwitterionic ligand which is coordinately bound will be described in the following sections.

According to an embodiment, the nanoparticle of the present invention is a particle that at least one zwitterionic ligand compound is coordinately bound to an outer surface of the metal particle containing iron oxide, and the metal particle is coated with the at least one zwitterionic ligand compound.

According to another embodiment, the nanoparticle in accordance with the present invention is a particle which includes a metal particle in a center part (core) of the particle and has a core-shell structure in which one or more zwitterionic ligand compounds are coordinately bound to an outer surface of the metal particle so as to coat the metal particle.

According to another embodiment, the nanoparticle of the present invention is a composite including (i) at least one metal particle containing iron oxide, at least one zwitterionic ligand being coordinately bound to the at least one metal particle, and (ii) at least one zwitterionic ligand compound.

According to another embodiment, the nanoparticle of the present invention is a cluster including (i) two or more zwitterionic ligand compounds and (ii) two or more metal particles, each of the two or more metal particles containing iron oxide, and at least one zwitterionic ligand compound being coordinately bound to each of the two or more metal particles.

According to another embodiment, the nanoparticle of the present invention is a cluster in which two or more zwitterionic ligand compounds are irregularly bound to two or more metal particles containing iron oxide that at least one zwitterionic ligand compound being coordinately bound to each of the two or more metal particles.

The nanoparticle to which the zwitterionic ligand compound of the present invention is coordinately bound enables prevention of agglomeration of nanoparticles, and exhibits stable particle properties even in, for example, a solution containing the nanoparticles at a high concentration. Such a nanoparticle can be expected to both (i) ensure low saturation magnetization and thus make it possible to obtain a T₁-weighted image with clear contrast and (ii) facilitate renal excretion and thus enable good renal clearance.

### (Metal particle)

The metal particle contains iron oxide. In one embodiment, the metal particle is an iron oxide particle containing only iron oxide. In another example, the metal particle is a metal particle containing iron in addition to iron oxide. The term "metal particle" in this specification encompasses an "iron oxide nanoparticle" in a raw material which is an "iron oxide nanoparticle in which a hydrophobic ligand is coordinately bound to a surface of the nanoparticle", and encompasses a "metal particle containing iron oxide" in which some sort of change has occurred from an iron oxide nanoparticle which is the raw material, as a result of carrying out a production method in which the zwitterionic ligand of the present invention is coordinately bound to a metal particle (for example, an MEAA method described later). Here, the some sort of change includes, but is not limited to, a structural change from a core-shell structure to a composite or a cluster, a change in particle diameter, a change in composition, and the like. That is, the term "metal particle" in this specification at least encompasses all metal particles containing iron oxide, which are obtained by the MEAA method, a TMA(OH) method (described later), or a phase transfer catalyst method (described later), in which the zwitterionic ligand shown in Formula (I) described in this specification is coordinated with a metal particle.

In an embodiment of the present invention, the metal particle containing iron oxide can further contain at least one metal derivative other than iron oxide. Further, the metal particle can contain at least one metal element other than iron (Fe). As the other metal element, the metal particle can further contain, as necessary, at least one selected from the group consisting of gadolinium (Gd), manganese (Mn), cobalt (Co), nickel (Ni), and zinc (Zn).

In still another embodiment of the present invention, the metal particle can consist of iron oxide alone or can contain ferrite derived from iron oxide. Ferrite is an oxide represented by formula: MFe₂O₄ where M is preferably a transition metal selected from Zn, Co, Mn, and Ni.

A material known as super paramagnetic iron oxide (SPIO) can be also suitably used. Such a material is represented by general formula: [Fe₂O₃]ₓ[Fe₂O₃(M²⁺O)]₁₋ₓ (where x = 0 or 1). M can be, for example, Fe, Mn, Ni, Co, Zn, magnesium (Mg), copper (Cu), or a combination thereof. Note that the material is magnetite (Fe₃O₄) in a case where the metal ion (M²⁺) is a ferrous iron (Fe²⁺) and x = 0, and the material is maghemite (γ-Fe₂O₃) in a case where x = 1.

In an embodiment of the present invention, iron oxide is magnetic oxide of iron, and can be magnetite (Fe₃O₄), maghemite (γ-Fe₂O₃), or a mixture thereof. A metal particle of the magnetic iron oxide is a super paramagnetic nanoparticle.

In still another embodiment of the present invention, in a case where the iron oxide particle contains derivative(s) of one or more metal elements other than iron, the derivative(s) of the respective metal element(s) can differ in kind. That is, the iron oxide particle can contain an oxide, a nitride, and the like. In another embodiment of the present invention, a core particle can contain a derivative (e.g., FePt and FeB) of iron other than iron oxide which derivative has an iron element other than iron oxide.

A metal particle in accordance with an embodiment of the present invention can be a metal particle produced by a well-known method such as a method disclosed in Patent Literature 1, Non-patent Literature 2, Non-patent Literature 3, or the like, or can be a commercially available metal particle. For example, the metal particle can be an iron oxide particle produced by a coprecipitation method or a reduction method.

### (Particle diameter of metal particle)

As used herein, the term "particle diameter" refers to an "average particle diameter" unless otherwise noted.

The term "particle diameter" of a metal particle means, for example, a diameter of a maximum inscribed circle of a two-dimensional shape of a particle observed with use of a transmission electron microscope (TEM). For example, in a case where the two-dimensional shape of the particle is substantially a circle, the "particle diameter" means a diameter of the circle. In a case where the two-dimensional shape of the particle is substantially an ellipse, the "particle diameter" means a minor axis of the ellipse. In a case where the two-dimensional shape of the particle is substantially a square, the "particle diameter" means a length of a side of the square. In a case where the two-dimensional shape of the particle is substantially a rectangle, the "particle diameter" means a length of a short side of the rectangle.

Examples of a method for confirming whether a value of an average particle diameter is in a predetermined range include a method of observing 100 particles with use of a transmission electron microscope (TEM) to measure the particle diameter of each particle and find an average value of the particle diameters of the 100 particles.

According to an embodiment of the present invention, a particle diameter of the metal particle measured with TEM (including an average diameter of a cluster or a composite containing the metal particle) is preferably 5 nm or less, more preferably 4 nm or less, more preferably 3 nm or less, further preferably 2 nm or less, most preferably 1 nm or less. Having a particle diameter of 2 nm or less makes the metal particle more useful as a positive contrast agent for high-magnetic field MRI of 3 tesla (T) or more.

Further, a metal particle having a particle diameter of 2 nm or less, preferably 1 nm or less, enables achieving a higher signal-to-noise ratio when used for high-magnetic field MRI of 7 T or more. This can enable measurement with a higher spatial resolution and in a shorter period of time.

In an embodiment of the present invention, properties of nanoparticles contained as a group in the contrast agent for MRI are preferably as uniform as possible among the individual nanoparticles. Accordingly, it is preferable that the metal particles serving as cores of the respective nanoparticles be uniform in size and shape. As an example, the metal particles have uniformity within a range of ±1 nm of the average particle diameter thereof. As another example, the metal particles have uniformity within a range of ±0.5 nm of the average particle diameter thereof.

In another embodiment of the present invention, as the metal particles to be contained, small particles are preferably contained as many as possible in the nanoparticles contained in the contrast agent for MRI. As an example, a ratio of the number of metal particles having a particle size of 3 nm or more to the number of all the metal particles is 30% or less, preferably 10% or less, more preferably 5% or less. As another example, a ratio of the number of metal particles having a particle size of 2 nm or more to the number of all the metal particles is 30% or less, preferably 10% or less, more preferably 5% or less. As yet another example, a ratio of the number of metal particles having a particle size of 1 nm or more to the number of all the metal particles is 30% or less, preferably 10% or less, more preferably 5% or less.

In yet another embodiment, a group of nanoparticles contained in the contrast agent for MRI can be heterogeneous in properties of particles, so that metal particles with which the zwitterionic ligands are coordinated can be nonuniform in size and in shape. As an example, the metal particle can encompass particles that differ in size from an average particle diameter by 1 nm or more.

### (Particle diameter of nanoparticle)

It is inferred that the particle diameter of the nanoparticle increases as a thickness of the zwitterionic ligand which is bound, by a coordinate bond, to the surface of the metal particle increases. In general, a hydrodynamic diameter (HD) of the nanoparticle as measured in a solution of the nanoparticle is employed as an index for the size of the nanoparticle. As an example, the nanoparticles have an average HD of 10 nm or less, preferably 8 nm or less. As another example, the nanoparticles have an average HD of 5 nm or less, preferably 4 nm or less, preferably 3 nm or less, preferably 2 nm or less, further preferably 1 nm or less.

The HD of nanoparticle can be measured, for example, by observing particles by a small angle X-ray scattering (SAXS) technique and averaging the particle diameters.

In the measurement by SAXS, a commercially available instrument can be used, and it is preferable to use a radiation facility such as SPring-8 (BL19B2) or Aichi Synchrotron Radiation Center. For example, when SPring-8 (BL19B2) is used, a camera length is set to 3 m, a sample is irradiated with 18KeV X-rays, and a wave number q is observed in a range approximately from 0.06 nm⁻¹ to 3 nm⁻¹.

In a case of a dispersion solution sample, the dispersion solution sample is placed in a capillary having a diameter of 2 mm, an exposure time is appropriately set to such an extent that scattered radiation is not saturated, and scattering data is obtained. The scattering data can be subjected to fitting with use of Guinier analysis or appropriate SAXS analysis software to obtain an average particle diameter.

For example, size exclusion chromatography (SEC) can be used as a method for measuring a relative size of nanoparticle.

SEC is an analysis technique in which (i) a sample is caused to flow through a column filled with a carrier having pores and (ii) a size of the sample is estimated on the basis of a time taken for the sample to be discharged from the column. Large aggregates do not enter the pores of the carrier, and therefore are quickly discharged from the column. Small nanoparticles pass through the pores of the carrier, and therefore are slowly discharged from the column due to following of a longer route before being discharged from the column. It is thus possible to measure a relative size of nanoparticle with use of standard particles.

### [2. Zwitterionic ligand compound]

The zwitterionic ligand compound in accordance with the present invention is a compound represented by the following formula (I) or a salt thereof: 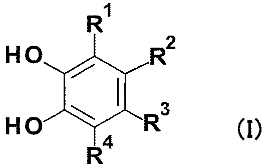 where
one of R¹ and R² is a group represented by a formula (a) or a formula (b), and the other of R¹ and R² is H, lower alkyl, -O- lower alkyl, or halogen, 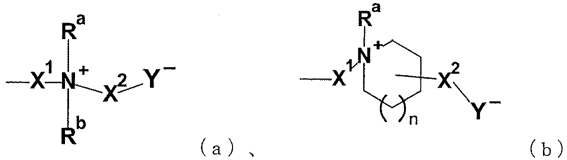
X¹ is a bond or methylene, or X¹ is optionally ethylene when R¹ is a group represented by the formula (a),
X² is C₁₋₅ alkylene that is optionally substituted with OH or is -C₁₋₂ alkylene-O-C₁₋₃ alkylene-, or X² is optionally a bond when R¹ is a group represented by the formula (b),
R^{a} and R^{b} are the same as or different from each other and represent C₁₋₃ alkyl or -C₁₋₃ alkylene-O-C₁₋₂ alkyl, or R^{a} and R^{b} form a 5- or 6-membered nitrogen-containing saturated heterocycle together with a quaternary nitrogen atom to which R^{a} and R^{b} are bound,
Y⁻ is SO₃⁻, HPO₃⁻, or CO₂⁻,
R³ and R⁴ are the same as or different from each other and represent H, C₁₋₃ alkyl, -O-C₁₋₃ alkyl, or halogen,
n is an integer of 0 to 2,
   and,
   i) when R¹ is a group represented by the formula (a) and X¹ is methylene, R² optionally forms ethylene together with R^{a} or R^{b},
   ii) when R¹ is a group represented by the formula (a) and X¹ is ethylene, R² optionally forms methylene together with Ra or R^{b}, and
   iii) when R² is a group represented by the formula (a) and X¹ is methylene, R³ optionally forms ethylene together with R^{a} or R^{b},
provided that, when R² is a group represented by the formula (a), R^{a} and R^{b} are methyl, X¹ is a bond, X² is C₁₋₄ alkylene, and R¹, R³ and R⁴ are H, Y⁻ is HPO₃⁻ or CO₂⁻.

According to another embodiment, the zwitterionic ligand compound is a zwitterionic ligand in which one of R¹ and R² is a group represented by the formula (a), and the other of R¹ and R² is H, lower alkyl, -O-lower alkyl, or halogen.

According to another embodiment, the zwitterionic ligand compound in accordance with the present invention is a compound represented by the following formula (o): 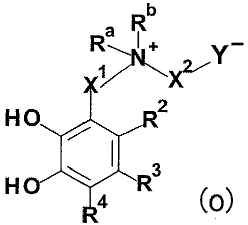 (where the signs are similar to those in the formula (I)).

According to an embodiment, the compound represented by the formula (o) is a zwitterionic ligand in which R² is H, lower alkyl, -O-lower alkyl, or halogen. According to another embodiment, the zwitterionic ligand compound is a zwitterionic ligand in which R² is H or halogen, X¹ is a bond, methylene or ethylene, or R² optionally forms ethylene together with R^{a} or R^{b} when X¹ is methylene, X² is C₂₋₄ alkylene, R^{a} and R^{b} are methyl, and R³ and R⁴ are the same as or different from each other and represent H, C₁₋₃ alkyl or halogen. According to still another embodiment, the zwitterionic ligand compound is a zwitterionic ligand in which R² is H or halogen, X¹ is a bond or methylene, or R² optionally forms ethylene together with R^{a} or R^{b} when X¹ is methylene, X² is C₂₋₄ alkylene, R^{a} and R^{b} are methyl, and R³ and R⁴ are the same as or different from each other and represent H, C₁₋₃ alkyl or halogen. According to still another embodiment, the zwitterionic ligand compound is a zwitterionic ligand in which R² is H or F, X¹ is a bond, methylene or ethylene, X² is ethylene or propylene, R^{a} and R^{b} are methyl, and R³ and R⁴ are H. According to still another embodiment, the zwitterionic ligand compound is a zwitterionic ligand in which R² is H, X¹ is ethylene, X² is ethylene or propylene, R^{a} and R^{b} are methyl, and R³ and R⁴ are H. According to still another embodiment, the zwitterionic ligand compound is a zwitterionic ligand in which R² is H or F, X¹ is a bond or ethylene, X² is an ethylene group or a propylene group, R^{a} and R^{b} are methyl, R³ and R⁴ are H, and Y⁻ is SO₃ ⁻ or CO₂⁻. According to still another embodiment, the zwitterionic ligand compound is a zwitterionic ligand in which R² is H or F, X¹ is methylene, X² is a propylene group or a butylene group, R^{a} and R^{b} are methyl, R³ and R⁴ are H, and Y⁻ is SO₃⁻, HPO₃⁻, or CO₂⁻. According to still another embodiment, the zwitterionic ligand compound is a zwitterionic ligand in which R² is H or F, X¹ is methylene, X² is a propylene group or a butylene group, R^{a} and R^{b} are methyl, R³ and R⁴ are H, and Y⁻ is SO₃⁻.

According to a certain embodiment, the zwitterionic ligand compound is a zwitterionic ligand represented by the following formula (1): 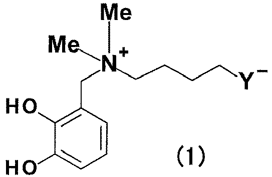 (where the sign Y⁻ is similar to that in the formula (I)).

According to another embodiment, the zwitterionic ligand compound is a zwitterionic ligand represented by the following formula (2): 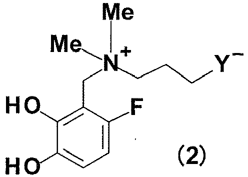 (where the sign Y⁻ is similar to that in the formula (I)).

According to another embodiment, the zwitterionic ligand compound is a zwitterionic ligand represented by the following formula (3): 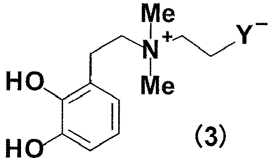 (where the sign Y⁻ is similar to that in the formula (I)).

According to another embodiment, the zwitterionic ligand compound is a zwitterionic ligand represented by the following formula (4): 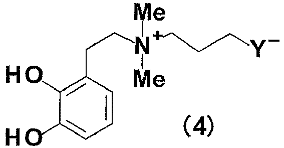 (where the sign Y⁻ is similar to that in the formula (I)).

According to another embodiment, the zwitterionic ligand compound is a zwitterionic ligand represented by the following formula (5): 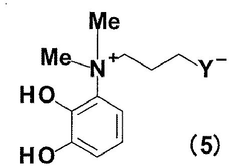 (where the sign Y⁻ is similar to that in the formula (I)).

Moreover, according to another embodiment, the zwitterionic ligand compound in accordance with the present invention is a compound represented by the following formula (6): 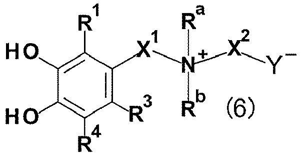 (where the signs are similar to those in the formula (I)).

According to a certain embodiment, the zwitterionic ligand compound is a zwitterionic ligand represented by the following formula (7): 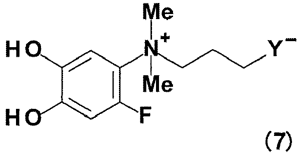 (where the sign Y⁻ is similar to that in the formula (I)).

According to an embodiment, the zwitterionic ligand compound is a zwitterionic ligand in which, in the above formula (I), one of R¹ and R² is a group represented by the formula (b-1) below, and the other of R¹ and R² is H, lower alkyl, -O-lower alkyl, or halogen: 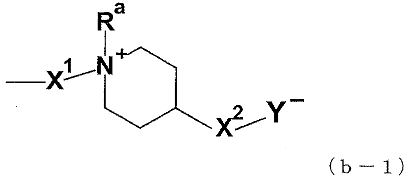 (where the signs are similar to those in the formula (I)).

According to an embodiment, the zwitterionic ligand compound in accordance with the present invention is a compound represented by the following formula (8): 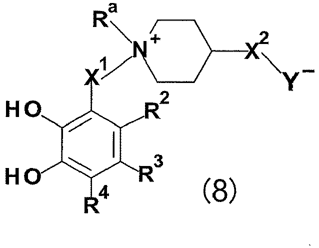 (where the signs are similar to those in the formula (I)).

According to an embodiment, the compound represented by the formula (8) is a zwitterionic ligand in which R² is H, lower alkyl, -O-lower alkyl, or halogen. According to another embodiment, the zwitterionic ligand compound is a zwitterionic ligand in which R² is H or halogen, X¹ is a bond or methylene, X² is a bond or C₁₋₃ alkylene, R^{a} is methyl, and R³ and R⁴ are the same as or different from each other and represent H, C₁₋₃ alkyl or halogen. According to still another embodiment, the zwitterionic ligand compound is a zwitterionic ligand in which R² is H or F, X¹ is methylene, X² is a bond or methylene, R^{a} is methyl, R³ and R⁴ are H, and Y⁻ is SO₃⁻, HPO₃⁻, or CO₂⁻. According to still another embodiment, the zwitterionic ligand compound is a zwitterionic ligand in which R² is H or F, X¹ is methylene, X² is a bond or methylene, R^{a} is methyl, R³ and R⁴ are H, and Y⁻ is CO₂⁻. Moreover, according to still another embodiment, the zwitterionic ligand compound is a zwitterionic ligand in which R² is H or halogen, X¹ is a bond or methylene, X² is C₁₋₅ alkylene or a bond, R^{a} is methyl, R³ and R⁴ are the same as or different from each other and represent H, C₁₋₃ alkyl, or halogen, and Y⁻ is SO₃⁻ or CO₂⁻.

According to another embodiment, the zwitterionic ligand compound is a zwitterionic ligand represented by the following formula (9): 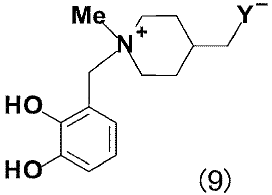 (where the sign Y⁻ is similar to that in the formula (I)).

According to another embodiment, the zwitterionic ligand compound is a zwitterionic ligand represented by the following formula (10): 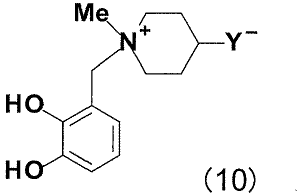 (where the sign Y⁻ is similar to that in the formula (I)).

The nanoparticle in accordance with the present invention is a nanoparticle containing at least one zwitterionic ligand represented by the above formula (I) and a metal particle containing iron oxide, the at least one zwitterionic ligand being coordinately bound to the metal particle. An embodiment of the nanoparticle in accordance with the present invention includes a nanoparticle containing (i) the zwitterionic ligand compound of each of the embodiments described in [2. Zwitterionic ligand compound] and (ii) a metal particle containing iron oxide, the zwitterionic ligand compound being coordinately bound to the metal particle. Note that, in a case where the zwitterionic ligand is bound, by a coordinate bond, to a metal particle containing iron or iron oxide, oxygens of two hydroxyl groups of the zwitterionic ligand compound are bound to the metal atom on a surface of the metal particle by a coordinate bond to form the nanoparticle in accordance with the present invention.

In addition, the present invention also encompasses use of the zwitterionic ligand compound for producing the nanoparticle in accordance with the present invention, as well as the zwitterionic ligand compound itself. The above embodiments described in [2. Zwitterionic ligand compound] are also embodiments of the zwitterionic ligand compound used in those features.

In the zwitterionic ligand in accordance with the present invention, a trisubstituted amino group is substituted with catechol directly or via an alkylene group to form an ammonium cation. The zwitterionic ligand of the present invention has a molecular chain shorter than that of a conventionally known ligand, and accordingly a ligand layer can be thinner. Further, the zwitterionic ligand of the present invention is characterized by having a positive charge on a metal particle side and a negative charge on an outer surface side. As such, it can be expected that the nanoparticles of the present invention are less likely to undergo agglomeration in body fluid and thus are highly stable. Further, thinness of the ligand layer reduces a distance from the metal atom. It can be accordingly expected that the nanoparticle of the present invention exhibits an excellent contrast ability resulting from an increase in the number of water molecules affected by the metal particle, and the like.

The number of zwitterionic ligand molecules (the number of zwitterionic ligands) coordinated on the surface of the metal particle varies depending on a size, surface area, and the like of the metal particle. The number of zwitterionic ligands per metal particle is 2 to 200 in an embodiment, 5 to 50 in another embodiment, and 5 to 20 in still another embodiment.

### (Compound bound to metal particle other than zwitterionic ligand)

The nanoparticle of the present invention can contain a component other than the zwitterionic ligand of the present invention. In an embodiment of the present invention, the nanoparticle can be (i) a nanoparticle in which a metal particle itself has a fluorescent property or (ii) a nanoparticle which further contains a molecule such as a fluorescent molecule or a dye molecule bound to a surface of the metal particle. In a case where the metal particle itself has a fluorescent property or in a case where a fluorescent molecule or a dye molecule is introduced in the nanoparticle, the nanoparticle can be used not only as a contrast agent for MRI but also as a contrast agent for an optical image. In another embodiment of the present invention, it is possible to employ a ligand in which a fluorescent molecule or a dye molecule is covalently bound to the zwitterionic ligand of the present invention, wherein the molecule is linked to the iron oxide particle via the zwitterionic ligand. After the nanoparticle is injected into a body, the fluorescent molecule is present on the surface of the iron oxide particle. The fluorescent molecule can thus be utilized for microscopic imaging and examination of localization of the nanoparticle. Examples of the fluorescent molecule and the dye molecule include rhodamine, fluorescein, nitrobenzoxadiazole (NBD), cyanine, green fluorescence protein (GFP), coumarin, and a derivative thereof.

In another embodiment of the present invention, the nanoparticle of the present invention can include at least one substance bound to the surface of the metal particle. Examples of such a substance include, but are not limited to, a peptide, a nucleic acid, a small molecule, and the like. For example, in a case where a peptide having a property of bringing about a therapeutic effect specifically to a tumor is bound to the nanoparticle of the present invention, the nanoparticle can have the therapeutic effect on the tumor.

Alternatively, a ligand other than the zwitterionic ligand of the present invention can be bound to the surface of the metal particle. For example, in a case where a ligand having a property of being accumulated specifically to a tumor is bound to the metal particle of the present invention, the nanoparticle can have a tumor-selective binding property.

Imparting such a tissue specificity to the contrast agent is preferable in order to (i) enhance a signal at a portion that is a subject of MRI measurement and (ii) thereby obtain information of a specific pathological condition or the like. A distribution of the contrast agent in a living organism depends on particle diameter, charge, surface chemistry, route of administration, and route of elimination.

In addition, the nanoparticle in accordance with the present invention is expected to have a lower toxicity to a living organism because the nanoparticle contains iron oxide as a metal particle. Accordingly, the nanoparticle is expected to be highly safe and have few restrictions on various uses.

### [3. Method for producing zwitterionic ligand]

A method for producing the zwitterionic ligand represented by the formula (I) of the present invention is not particularly limited. The zwitterionic ligand can be produced easily from a well-known raw material compound by a reaction well known to a person skilled in the art. For example, the zwitterionic ligand can be produced with reference to a method disclosed in Wei H. et al., Nano Lett. 12, 22-25, 2012.

As an example, a synthesis method described in Production Examples can be suitably employed.

### [4. Method for producing nanoparticle]

The following description will discuss a method for producing the nanoparticle.

### (Production of metal particle to which hydrophobic ligand or hydrophilic ligand as raw material is coordinately bound)

The metal particle to which a hydrophobic ligand or a hydrophilic ligand, which is a raw material for producing nanoparticles, is coordinately bound can be produced with use of a known method. For example, the metal particle can be produced with reference to the methods disclosed in Byung Hyo Kim et al., J Am. Chem. Soc. 2011, 133, 12624-12631 and Byung Hyo Kim et al., J Am. Chem. Soc. 2013, 135, 2407-2410.

For example, a metal particle having a surface coated with a hydrophobic ligand can be synthesized by (a) causing a metal salt to react with an alkali metal salt of a fatty acid to form a metal-fatty acid complex; and (b) heating the complex together with a surfactant rapidly to a high temperature of 200°C or more and, optionally, causing a reaction at the high temperature for a certain period of time. Further, (c) ligand substitution can be carried out in the metal particle coated with the hydrophobic ligand to form a metal particle coated with [2-(2-methoxyethoxy)ethoxy]acetic acid (MEAA) to obtain a metal particle coated with MEAA capable of being dispersed in a highly polar solvent.

The following describes each step in detail.

### (Step (a))

A metal salt and an alkali metal salt of a fatty acid are dispersed in a solvent. Examples of the metal salt include iron(III) chloride hexahydrate (FeCl₃·6H₂O), examples of the alkali metal salt of a fatty acid include sodium oleate, and examples of the solvent include ethanol, water, hexane, and a mixture thereof. Subsequently, a resultant solution is stirred while being heated, preferably at 70°C, for 1 hour to 10 hours, preferably for 3 hours to 4 hours, and an organic layer is collected. The organic layer is washed with water once or more, more preferably 3 times to 4 times. Thus, a metal-fatty acid complex is obtained. The organic layer obtained is optionally dried.

### (Step (b))

For example, in an atmosphere of an inert gas selected from argon (Ar) and nitrogen, the following (i) and (ii) are added to the complex obtained in the step (a): (i) at least one surfactant selected from the group consisting of a fatty acid, aliphatic alcohol, and aliphatic amine and (ii) a solvent selected from diphenyl ether and phenyloctyl ether. As an example, the surfactant can be oleic acid, oleyl alcohol, oleylamine, or a mixture thereof, and the solvent can be diphenyl ether. Subsequently, a mixture thus obtained is rapidly heated from room temperature to a temperature of 180°C to 300°C, and then is optionally stirred in this state for 10 minutes to several hours. As an example, the mixture is heated from 30°C to 250°C at a rate of 10°C/min, and is stirred at 250°C for 30 minutes. As another example, the mixture is heated from 30°C to 200°C at a rate of 10°C/min, and is stirred at 200°C for 30 minutes.

A resultant reaction solution is cooled down to room temperature. Then, acetone is added, and a resultant mixture is centrifuged to remove a supernatant. This operation is repeated 2 times to 3 times, preferably 4 times to 5 times. A solution thus obtained is optionally dried. As an example, the operation of adding acetone and performing centrifugation to remove the supernatant is repeated 3 times, and a metal particle is obtained whose surface is coated with a hydrophobic ligand such as oleic acid.

### (Step (c))

In an atmosphere of an inert gas selected from Ar and nitrogen, the nanoparticles coated with the hydrophobic ligand are dispersed in a solvent, and then a reaction is caused by adding MEAA. Methanol is suitably used as the solvent.

A reaction solution thus obtained is stirred at room temperature or while being heated, preferably at 25°C to 80°C for approximately 1 hour to 15 hours, preferably 5 hours to 10 hours. As an example, the reaction is carried out by stirring the reaction solution at 50°C for 7 hours. As another example, the reaction is carried out by stirring the reaction solution at 70°C for 10 hours. As yet another example, the reaction is carried out by stirring the reaction solution at 70°C for 5 hours.

The reaction solution is cooled down to room temperature. Then, a solvent selected from acetone and hexane is added, a resultant mixture is centrifuged to remove a supernatant. This operation can be repeated 2 times to 3 times, preferably 4 times to 5 times. A solution thus obtained can optionally be dried. As an example, the above operation is repeated 3 times, and thus a metal particle whose surface is coated with MEAA is obtained.

### (Method for producing nanoparticle of the present invention)

The "nanoparticle containing metal particle containing iron oxide to which at least one zwitterionic ligand is coordinately bound" in accordance with the present invention can be produced by using a known method through a metal particle having a surface coated with MEAA (MEAA method), a method using TMA(OH) (TMA(OH) method), or a new synthetic method using a phase transfer catalyst.

### A) MEAA method

In this production method, a metal particle having a surface coated with MEAA is caused to react with the zwitterionic ligand compound in accordance with the present invention to obtain the nanoparticle in accordance with the present invention. The metal particle having a surface coated with MEAA is caused to react with the zwitterionic ligand compound in accordance with the present invention by being stirred for 1 hour to several tens of hours in an atmosphere of an inert gas selected from Ar and nitrogen and at room temperature or while being heated. As an example, the above reaction is carried out in an Ar atmosphere. A reaction temperature is 25°C to 80°C as an example, and 50°C to 70°C as another example. A stirring time is 5 hours to 7 hours as an example, and 24 hours as another example. As an example, the stirring is carried out overnight at room temperature. Subsequently, a resultant reaction solution is cooled down to room temperature, and a solvent is added. A resultant mixture is centrifuged to remove a supernatant, and thus a nanoparticle is obtained in which at least one zwitterionic ligand compound of the present invention is coordinately bound. The solvent is not particularly limited, and can be selected from acetone, hexane, and the like. As an example, the solvent is acetone. The operation of adding the solvent and performing centrifugation to remove the supernatant can be repeated a plurality of times. For example, the operation can be repeated 4 times to 5 times. As an example, this operation is repeated 3 times. Subsequently, a resultant solution containing the nanoparticle coated with the zwitterionic ligand compound of the present invention can be concentrated with use of a concentration column or the like of a centrifugal ultrafilter or the like. This concentration operation can be repeated a plurality of times, during which a solution such as PBS can be added at some point, and then the concentration operation can be repeated.

### B) TMA(OH) method

An iron oxide particle (SNP-OA) coated with oleic acid is suspended in a hexane solution. A resultant suspension is mixed with 1.7% tetramethylammonium hydroxide (TMA(OH)) aqueous solution, and is vigorously shaken. A resultant solution is centrifuged to separate an aqueous layer, and acetone is added. A resultant mixture is centrifuged at 8000 rpm to 12000 rpm for 5 minutes to 10 minutes, and a supernatant is removed to obtain a precipitate. 2 mL of 0.1% TMA(OH) solution is added and dispersed in the precipitate, acetone is added again in an amount of 10 mL, and a resultant mixture is left for precipitation. This operation can be repeated a plurality of times, and is repeated preferably 3 times to 4 times. A solution thus obtained is dispersed in 0.1% TMA(OH) solution and stored.

To 0.1% TMA(OH) solution thus prepared in accordance with the above procedure, a solution of the ligand compound, which has been prepared with use of 0.1% to 2% TMA(OH) solution so as to achieve approximately pH 8 to pH 12, is added. A resultant solution is stirred at room temperature for 6 hours to 24 hours, and acetone is added. A resultant mixture is left for precipitation and is centrifuged at 8000 rpm to 12000 rpm for 3 minutes to 10 minutes to remove a supernatant. A precipitate thus obtained is dispersed in a phosphate buffer, and a resultant solution is centrifuged at 7000 rpm to 12000 rpm with use of a concentration column to reduce an amount of the solution. A phosphate buffer is added again, and a resultant mixture is centrifuged at 7000 rpm to 12000 rpm for 10 minutes to 20 minutes for concentration. This operation can be repeated a plurality of times, preferably 3 times to 4 times, more preferably 5 times to 10 times. Thus, a nanoparticle is obtained in which at least one zwitterionic ligand of the present invention is coordinately bound. A solution of the nanoparticle thus obtained can be diluted with PBS and stored.

### C) Phase transfer catalyst method

In this method, a metal particle having a surface to which a hydrophobic ligand (such as oleic acid) is coordinately bound is brought into contact with the zwitterionic ligand compound in accordance with the present invention in the presence of a phase transfer catalyst in a two-layered solvent including an organic layer and an aqueous layer. Thus, a nanoparticle is produced in which at least one zwitterionic ligand of the present invention is coordinately bound.

The "two-layered solvent including an organic layer and an aqueous layer" is a mixed solvent containing an organic solvent and water, which are separated into respective two layers. The organic solvent is an aprotic solvent and, in one embodiment, the organic solvent is selected from the group consisting of 2-methyltetrahydrofuran (2-Me-THF), cyclopentyl methyl ether (CPME), methyl tert-butyl ether (MTBE), chloroform, toluene, xylene, heptane and combinations thereof. In another embodiment, the organic solvent is selected from 2-methyltetrahydrofuran, chloroform and combinations thereof.

The term "phase transfer catalyst" refers to a phase transfer catalyst selected from salts having quaternary ammonium and quaternary phosphonium which are soluble both in an organic solvent and in water. One embodiment of the phase transfer catalyst is a quaternary ammonium salt, and the quaternary ammonium salt is, for example, selected from the group consisting of tetrabutylammonium salt, trioctylmethylammonium salt, and benzyldimethyloctadecylammonium salt. Examples of anions forming salts here include halide ions, hydroxide ions, hydrogen sulfate ions, and the like. Yet another embodiment of the phase transfer catalyst is tetrabutylammonium halide salt, and the tetrabutylammonium halide salt is, for example, selected from tetrabutylammonium bromide (TBAB) and tetrabutylammonium fluoride (TBAF). Yet another embodiment of the phase transfer catalyst is a hydrate of tetrabutylammonium fluoride, e.g., tetrabutylammonium fluoride trihydrate.

Further, optionally, a pH adjusting agent can be added and, for example, sodium hydrogen carbonate, sodium carbonate, potassium hydrogen carbonate, ammonium hydrogen carbonate or dipotassium hydrogen phosphate can be used.

The reaction is carried out by stirring the zwitterionic ligand compound and a metal particle having a surface to which a hydrophobic ligand is coordinately bound. The stirring is carried out in a two-layered solvent including an organic layer and an aqueous layer in the presence of a phase transfer catalyst at room temperature or while being heated in an inert gas atmosphere selected from nitrogen and argon. In one embodiment, the stirring is carried out at room temperature to 80°C. In another embodiment, the stirring is carried out at 30°C to 60°C for one hour or more. In one embodiment, the stirring is carried out for 1 to 20 hours. In another embodiment, the stirring is carried out for 1 to 15 hours. In another embodiment, the stirring is carried out for 1 to 6 hours. The reaction temperature and the reaction time can be appropriately adjusted according to a metal particle used in the reaction and a type of the zwitterionic ligand.

In this reaction, the zwitterionic ligand can be used, relative to the metal particle, in a ratio of 1 to 30 wt (weight ratio), 5 to 20 wt in one embodiment, or 6 to 15 wt in another embodiment. The phase transfer catalyst can be added in the following ratios relative to the metal particle: 0.1 to 10 wt, 0.1 wt to 6 wt in one embodiment, 0.1 wt to 5 wt in another embodiment, 0.5 to 6 wt in another embodiment, 0.5 to 3 wt in another embodiment, and 0.5 wt to 2 wt in yet another embodiment. In a case where the pH adjusting agent is additionally used, the phase transfer catalyst can be added in a ratio of 0.1 wt to 5 wt, or 0.5 wt to 2 wt in one embodiment, relative to the metal particle.

Isolation of a nanoparticle from the reaction solution can be carried out using a known method such as centrifugation, ultrafiltration, or liquid separating operation. For example, the isolation can be carried out by repeating centrifugation or filtration using Amicon (registered trademark) Ultracentrifuge filter (Merck Millipore), Agilent Captiva Premium Syringe Filters (Regenerated Cellulose, 15 mm), YMC Duo-Filter, or the like. A solution of the nanoparticle thus obtained can be diluted with PBS and stored.

In any of the methods in which the zwitterionic ligand in accordance with the present invention is used, in some cases, a nanoparticle is produced in which a hydrophobic ligand on the surface is simply substituted with the zwitterionic ligand, and in other cases, a nanoparticle (e.g., a 3K purified particle shown in Examples described later) is produced in which a metal particle in the nanoparticle is smaller than the metal particle used as the raw material. In many cases, both of those types are obtained. This seems to be because the zwitterionic ligand in accordance with the present invention has the property of changing a metal particle when the zwitterionic ligand is coordinately bound to the metal particle. The type of obtained nanoparticles varies depending on the zwitterionic ligand. The type of obtained nanoparticles can also vary depending on the reaction conditions and purification conditions.

By adjusting the type of the zwitterionic ligand to be used, the reaction conditions and the isolation conditions, a nanoparticle having a core-shell structure and/or a nanoparticle (cluster, composite, or the like) having a metal fine particle can be obtained.

According to an embodiment, a metal particle which is coated with the at least one zwitterionic ligand compound is produced in which at least one zwitterionic ligand compound is coordinately bound to the outer surface of the metal particle containing iron oxide.

According to an embodiment, a fine particle is produced as a composite including at least one zwitterionic ligand compound and at least one metal particle containing iron oxide, at least one zwitterionic ligand compound being coordinately bound to each of the at least one metal particle.

According to an embodiment, a cluster consisting of two or more zwitterionic ligand compounds and two or more "metal particles containing iron oxide in which at least one zwitterionic ligand compounds are coordinately bound" is produced.

In any of the embodiments, the nanoparticle of the present invention can be used as a contrast agent for magnetic resonance imaging.

An embodiment is a method later described in Examples below.

### [5. Contrast agent for magnetic resonance imaging (contrast agent for MRI)]

The present invention also provides a contrast agent for magnetic resonance imaging which contrast agent includes the above-described nanoparticle.

The following description will discuss the contrast agent for MRI in detail.

### (Various components contained in contrast agent for MRI)

### i) Nanoparticle

In an embodiment of the present invention, the contrast agent for MRI of the present invention is characterized by containing at least one kind of the above-described nanoparticle. In another embodiment of the present invention, the contrast agent for MRI of the present invention can include a combination of two or more kinds of the above-described nanoparticle.

Further, the contrast agent for MRI can contain, if necessary, a solvent and a pharmacologically acceptable additive in addition to the nanoparticle. In an embodiment of the contrast agent for MRI of the present invention, the contrast agent can further contain a suitable solvent and/or at least one selected from additives such as a carrier, a vehicle, and a complex.

### ii) Solvent

Examples of the solvent contained in the contrast agent for MRI include water, a buffer solution, and the like. Further, examples of the buffer solution include physiological saline, phosphate buffer, tris buffer, boric acid buffer, Ringer's solution, and the like. In a case where a dosage form is an injection, examples of a preferable solvent include water, Ringer's solution, physiological saline, and the like.

That is, the contrast agent for MRI in accordance with the present invention can be a solution obtained by suspending the nanoparticle in accordance with the present invention in a solution having a desired composition. Specifically, the contrast agent can be in the form of a buffer solution such as phosphate buffer, tris buffer, or boric acid buffer in which the nanoparticle is suspended.

### iii) Additive

Examples of the additive such as a carrier, a complex, and a vehicle contained in the contrast agent for MRI include a carrier, a vehicle, and the like which are generally used in the fields of pharmaceuticals and biotechnology. Examples of the carrier include a polymer such as polyethylene glycol, a metal fine particle, and the like. Examples of the complex include diethylenetriaminepentaacetic acid (DTPA), 1,4,7,10-tetraazacyclododecane-1,4,7,10-tetraacetic acid (DOTA), and the like. Examples of the vehicle include lime, soda ash, sodium silicate, starch, glue, gelatin, tannin, quebracho, and the like.

The contrast agent for MRI of the present invention can further contain an excipient, a lubricant, a wetting agent, an emulsifier, a suspension, a preservative, a pH adjusting agent, an osmotic pressure controlling agent, and the like.

### (Dosage form)

A dosage form of the contrast agent for MRI of the present invention is not particularly limited, and can be liquid, solid or semisolid, or semiliquid. These dosage forms can be produced easily in accordance with a method well known to a person skilled in the art. In a case where the dosage form is a liquid, the liquid can be one which is obtained by dispersing, suspending, or dissolving the nanoparticle in accordance with the present invention in, for example, an aqueous solvent so that the liquid contains the nanoparticle. Further, the contrast agent can be in the form of a lyophilized agent, and be dispersed, suspended, or dissolved when used.

### (Concentration of nanoparticle)

A concentration of the nanoparticle in the contrast agent for MRI is determined as appropriate in accordance with a purpose, a tissue to be imaged, and the like. For example, a concentration is selected such that the selected concentration is in a range within which (i) an adequate contrast ability is exhibited and (ii) a degree of influence on a living organism is tolerable.

The nanoparticle of the present invention, even when contained at a high concentration, is less likely to agglomerate and thus is capable of maintaining the stability. Accordingly, the nanoparticle of the present invention is expected to maintain, stably and for a long period of time, a higher MRI contrast ability than a well-known nanoparticle.

For example, in a case where the contrast agent for MRI is a liquid that is an aqueous solution, examples of a concentration of the nanoparticle in the liquid when, for example, the liquid is used as a general injection include 0.1 mM Fe to 1000 mM Fe, preferably 1.0 mM Fe to 500 mM Fe, further preferably 5.0 mM Fe to 100 mM Fe, and, in an embodiment, 10 mM Fe to 500 mM Fe, and, in another embodiment, 5.0 mM Fe to 50 mM Fe.

### (Administration target)

An administration target to which the contrast agent in accordance with the present invention is administered can be, for example, a given organism that is not a human, or a human. Examples of the organism that is not a human include, but not limited to, mammals (e.g., rodents such as mice, rats, and rabbits, primates such as monkeys, dogs, cats, sheep, cows, horses, pigs, and the like), birds, reptiles, amphibians, fish, insects, and plants. In an embodiment, the animal can be a transgenic animal, a genetically-engineered animal, or a clone animal. Further, the administration target can be one that is not a living organism, for example, a tissue sample or a biological material which includes a cell.

### (Uses to which contrast agent for MRI is applied)

As described above, there are two types of contrast agents for MRI, namely, a positive contrast agent and a negative contrast agent.

In an embodiment of the present invention, the contrast agent for MRI of the present invention is a positive contrast agent. In another embodiment, the contrast agent is a negative contrast agent.

The present invention also encompasses an MRI contrast imaging method using the above MRI contrast agent. In addition, the present invention also involves contrast imaging of various organs of a subject by an MRI apparatus using the above described contrast agent for MRI. Examples of the contrast imaging include contrast imaging of a kidney, a liver, and a cerebral vessel. The present invention also involves a method for diagnosing, for example, the presence or absence of a lesion or tumor in various organs in a subject using the above described contrast agent for MRI. For example, the contrast agent for MRI can be suitably used in a method for diagnosing a kidney function, a method for diagnosing a liver tumor, and the like. Furthermore, the present invention also involves a method of visualizing various organs of a subject by an MRI apparatus using the above contrast agent for MRI. For example, the contrast agent for MRI can be suitably used in visualization of a kidney, a liver, a cerebral vessel, and the like. Note that the MRI apparatus can be any apparatus, and a well-known MRI apparatus can be used. A magnetic field to be applied can be, for example, 1 T, 1.5 T, 3 T, or 7 T. The diagnosis method or the visualization method using the contrast agent of the present invention includes the steps of: administering a positive contrast agent to a living subject such as a human; and subsequently obtaining an MRI image of an intended organ of the subject with use of an MRI apparatus.

Paramagnetism occurs as follows: when an external magnetic field is applied to a magnetic body, a dipole moment in a certain orientation is turned to an orientation identical with that of the applied magnetic field, and thus the magnetic body is magnetized in the same direction as the external magnetic field. Such a substance brings about a T₁-shortening effect through dipole-dipole interaction. A super paramagnetic body also generates a net magnetic moment with a similar mechanism, and has a magnetic susceptibility greater than that of a paramagnetic body and brings bout a greater T₂-shortening effect. The contrast agent of the present invention is considered to be in a boundary between paramagnetism and super paramagnetism or to exhibit paramagnetism. The relaxation mechanisms of both paramagnetism and super paramagnetism are inferred to exert influence according to the magnetic field strength, and T₁ relaxation, T₂ relaxation, and T₂* relaxation are brought about. In particular, the T₁-shortening effect in the practical magnetic field region is expected to result in a higher positive contrast effect. It is possible to confirm that the contrast agent is in the boundary between paramagnetism and super paramagnetism or exhibits paramagnetism by measuring a magnetic field dependence of magnetization with use of a superconducting quantum interference device (SQUID). Fig. 7 shows measurement examples at 300K. The magnetic susceptibility is substantially in proportion to the magnetic field. The property as a super paramagnetic substance seems to be low, and the contrast agent, even in the form of nanoparticle, has the paramagnetic property, and is expected to have an excellent T₁-shortening effect in the practical magnetic field region.

In an embodiment of the present invention, the contrast agent in accordance with the present invention has a contrast ability represented by an r₂ relaxivity of 2.8 mM⁻¹s⁻¹ to 6.2 mM⁻¹s⁻¹ and an r₁ relaxivity of 2.5 mM⁻¹s⁻¹ to 4.4 mM⁻¹s⁻¹, at 37°C and with a magnetic field of 1.5 T. In another embodiment of the present invention, the contrast agent in accordance with the present invention has a contrast ability represented by an r₂ relaxivity of 3.0 mM⁻¹s⁻¹ to 4.2 mM⁻¹s⁻¹ and an r₁ relaxivity of 2.7 mM⁻¹s⁻¹ to 3.9 mM⁻¹s⁻¹, at 37°C and with a magnetic field of 1.5 T.

The relaxivity depends on various factors such as (i) a particle diameter of the metal particle in the nanoparticle of the contrast agent for MRI, (ii) a composition of the metal particle, (iii) a charge and properties of the surface of the particle, (iv) particle stability, and (v) agglomeration and a binding property to tissues in a living organism. A relaxivity ratio r₁/r₂ is generally used for quantification of a type of a contrast generated in MRI, and can serve as an index for performance of the contrast agent.

An r₁/r₂ value of the positive contrast agent for MRI in accordance with the present invention preferably as high as possible for obtaining a higher positive contrast effect to improve diagnosability. For example, the r₁/r₂ value in a case where the magnetic field is 1.5 T is preferably 0.6 or more, more preferably 0.7 or more, even more preferably 0.8 or more. In a case where the r₁/r₂ value is 0.7 or more, the positive contrast agent exhibits an excellent T₁ (positive) effect and, even in MRI measurement with a higher magnetic field, exhibits a high contrast effect with a high resolution. From the viewpoint of significantly increasing the contrast effect and reducing an amount of the positive contrast agent for MRI to be administered, the r₁/r₂ value is preferably 0.8 or more.

In the nanoparticle in accordance with the present invention, a molecular chain length of the zwitterionic ligand is shorter than that of a publicly known ligand. This reduces a distance between the metal particle and an outside water molecule, and allows the relaxivity to be efficiently exhibited.

The contrast agent for MRI in accordance with the present invention encompasses a contrast agent for MRI containing a nanoparticle having a metal particle whose particle diameter (including an average diameter of a cluster or a composite containing the metal particles) is 2 nm or less (e.g., 1 nm or less). Such a contrast agent for MRI can be used as a positive contrast agent in a T₁-weighted image taken by an MRI apparatus of 7 T or more. As an example, the contrast agent for MRI of the present invention encompasses a positive contrast agent for MRI to be used with an MRI apparatus of 7 T or less. As an example, the contrast agent for MRI of the present invention encompasses a positive contrast agent for MRI to be used with an MRI apparatus of 3 T or less.

### (Toxicity and stability)

The contrast agent for MRI of the present invention exhibits a high stability of the nanoparticle. It is possible to confirm a degree of agglomeration with a method described in Test Example 3 (described later), and the contrast agent for MRI is expected to be stored in a solution for a long period of time at room temperature or at 4°C without undergoing agglomeration. Further, the contrast agent has a low toxicity to organisms. From this, long-term and continuous application of the contrast agent to a living organism is expected.

### [6. Examples of specific embodiments in accordance with the present invention]

In order to attain the object, the present invention includes in its scope any one embodiment below.

Note that, unless otherwise stated, when a symbol in a certain chemical formula in this specification is also used in another chemical formula, the same symbol indicates the same meaning.

### <1>

A nanoparticle comprising: at least one zwitterionic ligand represented by a formula (I); and a metal particle containing iron oxide, the at least one zwitterionic ligand being coordinately bound to the metal particle: 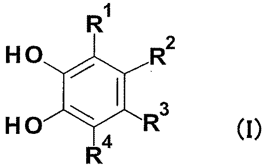 where
one of R¹ and R² is a group represented by a formula (a) or a formula (b), and the other of R¹ and R² is H, lower alkyl, -O- lower alkyl, or halogen, 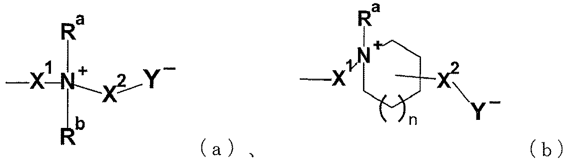
X¹ is a bond or methylene, or X¹ is optionally ethylene when R¹ is a group represented by the formula (a),
X² is C₁₋₅ alkylene that is optionally substituted with OH or is -C₁₋₂ alkylene-O-C₁₋₃ alkylene-, or X² is optionally a bond when R¹ is a group represented by the formula (b),
R^{a} and R^{b} are the same as or different from each other and represent C₁₋₃ alkyl or -C₁₋₃ alkylene-O-C₁₋₂ alkyl, or R^{a} and R^{b} form a 5- or 6-membered nitrogen-containing saturated heterocycle together with a quaternary nitrogen atom to which R^{a} and R^{b} are bound,
Y⁻ is SO₃⁻, HPO₃⁻, or CO₂⁻,
R³ and R⁴ are the same as or different from each other and represent H, C₁₋₃ alkyl, -O-C₁₋₃ alkyl, or halogen,
n is an integer of 0 to 2,
   and,
   i) when R¹ is a group represented by the formula (a) and X¹ is methylene, R² optionally forms ethylene together with R^{a} or R^{b},
   ii) when R¹ is a group represented by the formula (a) and X¹ is ethylene, R² optionally forms methylene together with R^{a} or R^{b}, and
   iii) when R² is a group represented by the formula (a) and X¹ is methylene, R³ optionally forms ethylene together with R^{a} or R^{b},
provided that, when R² is a group represented by the formula (a), R^{a} and R^{b} are methyl, X¹ is a bond, X² is C₁₋₄ alkylene, and R¹, R³ and R⁴ are H, Y⁻ is HPO₃⁻ or CO₂⁻.

### <2>

The nanoparticle described in <1>, in which:
in the at least one zwitterionic ligand,
one of R¹ and R² is a group represented by the formula (a) or the formula (b), and the other of R¹ and R² is H, lower alkyl, or halogen,
X¹ is a bond or methylene, or X¹ is optionally ethylene when R¹ is a group represented by the formula (a),
X² is C₁₋₅ alkylene that is optionally substituted with OH or is -C₁₋₂ alkylene-O-C₁₋₃ alkylene-, or X² is optionally a bond when R¹ is a group represented by the formula (b),
R^{a} and R^{b} are the same as or different from each other and represent C₁₋₃ alkyl or -C₁₋₃ alkylene-O-C₁₋₂ alkyl, or R^{a} and R^{b} form a pyrrolidine ring together with a quaternary nitrogen atom to which R^{a} and R^{b} are bound,
Y⁻ is SO₃⁻, HPO₃⁻, or CO₂⁻,
R³ and R⁴ are the same as or different from each other and represent H, C₁₋₃ alkyl, or halogen,
n is 1,
   and,
   i) when R¹ is a group represented by the formula (a) and X¹ is methylene, R² optionally forms ethylene together with R^{a} or R^{b}.

### <3>

The nanoparticle described in <2>, in which:
in the at least one zwitterionic ligand,
R¹ is a group represented by the formula (a) or the formula (b), and R² is H or halogen,
X¹ is a bond or methylene, or X¹ is optionally ethylene when R¹ is a group represented by the formula (a),
X² is C₁₋₅ alkylene, or X² is optionally a bond when R¹ is a group represented by the formula (b),
R^{a} and R^{b} are methyl, and
Y⁻ is SO₃⁻ or CO₂⁻.

### <4>

The nanoparticle described in <1>, in which, in the at least one zwitterionic ligand, one of R¹ and R² is a group represented by the formula (a), and the other of R¹ and R² is H, lower alkyl, -O-lower alkyl, or halogen.

### <5>

The nanoparticle described in <4>, in which:
in the at least one zwitterionic ligand,
1) R¹ is a group represented by the formula (a), and R² is H, lower alkyl, -O-lower alkyl, or halogen, or
2) R¹ is H, R² is a group represented by the formula (a), R³ is C₁₋₃ alkyl or halogen, and R⁴ is H.

### <6>

The nanoparticle described in <5>, in which, in the at least one zwitterionic ligand, R¹ is a group represented by the formula (a), and R² is H, lower alkyl, -O-lower alkyl, or halogen.

### <7>

The nanoparticle described in <6>, in which:
in the at least one zwitterionic ligand,
R² is H or halogen,
X¹ is a bond, methylene, or ethylene,
X² is C₂₋₄ alkylene,
R^{a} and R^{b} are methyl,
R³ and R⁴ are the same as or different from each other and represent H, C₁₋₃ alkyl, or halogen,
and, when X¹ is methylene, R² optionally forms ethylene together with R^{a} or R^{b}.

### <8>

The nanoparticle described in <7>, in which:
in the at least one zwitterionic ligand,
R2 is H or F,
X² is ethylene or propylene, and
R³ and R⁴ are H.

### <9>

The nanoparticle described in <8>, in which:
in the at least one zwitterionic ligand,
R2 is H, and
X¹ is a bond or ethylene.

### <10>

The nanoparticle described in any one of <4> through <9>, in which:
in the at least one zwitterionic ligand,
Y⁻ is SO₃⁻ or CO₂⁻.

### <11>

The nanoparticle described in <3>, in which:
in the at least one zwitterionic ligand,
R¹ is a group represented by the following formula (b-1), 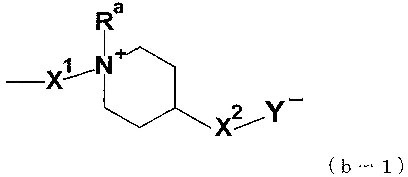
R² is H or halogen,
X¹ is a bond or methylene,
X² is C₁₋₅ alkylene or a bond,
R^{a} is methyl, and
Y⁻ is SO₃⁻ or CO₂⁻.

### <12>

The nanoparticle described in any one of <1> through <11>, in which the metal particle contains only iron oxide.

### <13>

The nanoparticle described in any one of <1> through <12>, in which: the at least one zwitterionic ligand is coordinately bound to an outer surface of the metal particle containing iron oxide; and the metal particle is coated with the at least one zwitterionic ligand.

### <14>

The nanoparticle described in any one of <1> through <12>, in which the nanoparticle is a composite containing the at least one zwitterionic ligand and the metal particle containing iron oxide, the at least one zwitterionic ligand being coordinately bound to the metal particle.

### <15>

The nanoparticle described in any one of <1> through <12>, in which the nanoparticle is a cluster containing two or more zwitterionic ligand compounds and two or more metal particles, each of the two or more metal particles containing iron oxide, and at least one zwitterionic ligand compound being coordinately bound to each of the two or more metal particles.

### <16>

A contrast agent for magnetic resonance imaging, containing a nanoparticle described in any one of <1> through <15>.

### <17>

The contrast agent described in <16>, in which the contrast agent is a positive contrast agent.

### <18>

Use of a zwitterionic ligand compound represented by the following formula (I) for producing the nanoparticle described in <1>: 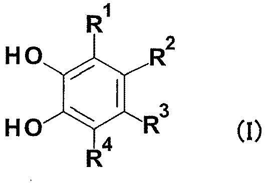 where
one of R¹ and R² is a group represented by a formula (a) or a formula (b) below, and the other of R¹ and R² is H, lower alkyl, -O- lower alkyl, or halogen, 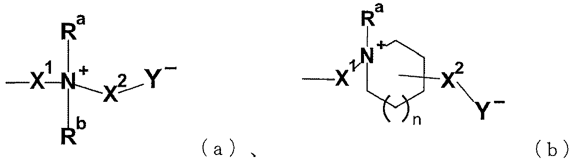
X¹ is a bond or methylene, or X¹ is optionally ethylene when R¹ is a group represented by the formula (a),
X² is C₁₋₅ alkylene that is optionally substituted with OH or is -C₁₋₂ alkylene-O-C₁₋₃ alkylene-, or X² is optionally a bond when R¹ is a group represented by the formula (b),
R^{a} and R^{b} are the same as or different from each other and represent C₁₋₃ alkyl or -C₁₋₃ alkylene-O-C₁₋₂ alkyl, or R^{a} and R^{b} form a 5- or 6-membered nitrogen-containing saturated heterocycle together with a quaternary nitrogen atom to which R^{a} and R^{b} are bound,
Y⁻ is SO₃⁻, HPO₃⁻, or CO₂⁻,
R³ and R⁴ are the same as or different from each other and represent H, C₁₋₃ alkyl, -O-C₁₋₃ alkyl, or halogen,
n is an integer of 0 to 2,
   and,
   i) when R¹ is a group represented by the formula (a) and X¹ is methylene, R² optionally forms ethylene together with R^{a} or R^{b},
   ii) when R¹ is a group represented by the formula (a) and X¹ is ethylene, R² optionally forms methylene together with R^{a} or R^{b}, and
   iii) when R² is a group represented by the formula (a) and X¹ is methylene, R³ optionally forms ethylene together with R^{a} or R^{b},
provided that, when R² is a group represented by the formula (a), R^{a} and R^{b} are methyl, X¹ is a bond, X² is C₁₋₄ alkylene, and R¹, R³ and R⁴ are H, Y⁻ is HPO₃⁻ or CO₂⁻.

### <19>

The use described in <18>, in which, in the zwitterionic ligand compound, one of R¹ and R² is a group represented by the formula (a), and the other of R¹ and R² is H, lower alkyl, -O-lower alkyl, or halogen.

### <20>

A compound represented by the following formula (I) or a salt thereof: 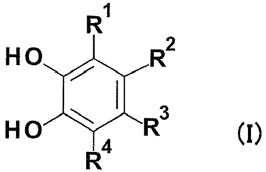 where
one of R¹ and R² is a group represented by a formula (a) or a formula (b) below, and the other of R¹ and R² is H, lower alkyl, -O- lower alkyl, or halogen, 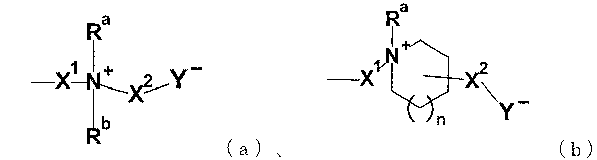
X¹ is a bond or methylene, or X¹ is optionally ethylene when R¹ is a group represented by the formula (a),
X² is C₁₋₅ alkylene that is optionally substituted with OH or is -C₁₋₂ alkylene-O-C₁₋₃ alkylene-, or X² is optionally a bond when R¹ is a group represented by the formula (b),
R^{a} and R^{b} are the same as or different from each other and represent C₁₋₃ alkyl or -C₁₋₃ alkylene-O-C₁₋₂ alkyl, or R^{a} and R^{b} form a 5- or 6-membered nitrogen-containing saturated heterocycle together with a quaternary nitrogen atom to which R^{a} and R^{b} are bound,
Y⁻ is SO₃⁻, HPO₃⁻, or CO₂⁻,
R³ and R⁴ are the same as or different from each other and represent H, C₁₋₃ alkyl, -O-C₁₋₃ alkyl, or halogen,
n is an integer of 0 to 2,
   and,
   i) when R¹ is a group represented by the formula (a) and X¹ is methylene, R² optionally forms ethylene together with R^{a} or R^{b},
   ii) when R¹ is a group represented by the formula (a) and X¹ is ethylene, R² optionally forms methylene together with R^{a} or R^{b}, and
   iii) when R² is a group represented by the formula (a) and X¹ is methylene, R³ optionally forms ethylene together with R^{a} or R^{b},
provided that, when R² is a group represented by the formula (a), R^{a} and R^{b} are methyl, X¹ is a bond, X² is C₁₋₄ alkylene, and R¹, R³ and R⁴ are H, Y⁻ is HPO₃⁻ or CO₂⁻.

### <21>

The compound described in <20> or a salt thereof, in which:
one of R¹ and R² is a group represented by the formula (a) or the formula (b), and the other of R¹ and R² is H, lower alkyl, or halogen,
X¹ is a bond or methylene, or X¹ is optionally ethylene when R¹ is a group represented by the formula (a),
X² is C₁₋₅ alkylene that is optionally substituted with OH or is -C₁₋₂ alkylene-O-C₁₋₃ alkylene-, or X² is optionally a bond when R¹ is a group represented by the formula (b),
R^{a} and R^{b} are the same as or different from each other and represent C₁₋₃ alkyl or -C₁₋₃ alkylene-O-C₁₋₂ alkyl, or R^{a} and R^{b} form a pyrrolidine ring together with a quaternary nitrogen atom to which R^{a} and R^{b} are bound,
Y⁻ is SO₃⁻, HPO₃⁻, or CO₂⁻,
R³ and R⁴ are the same as or different from each other and represent H, C₁₋₃ alkyl, or halogen, n is 1,
and, i) when R¹ is a group represented by the formula (a) and X¹ is methylene, R² optionally forms ethylene together with R^{a} or R^{b}.

### <22>

The compound described in <21> or a salt thereof, in which:
R¹ is a group represented by the formula (a) or the formula (b), and R² is H or halogen,
X¹ is a bond or methylene, or X¹ is optionally ethylene when R¹ is a group represented by the formula (a),
X² is C₁₋₅ alkylene, or X² is optionally a bond when R¹ is a group represented by the formula (b),
R^{a} and R^{b} are methyl, and
Y⁻ is SO₃⁻ or CO₂⁻.

### <23>

The compound described in <20> or a salt thereof, in which: one of R¹ and R² is a group represented by the formula (a), and the other of R¹ and R² is H, lower alkyl, - O-lower alkyl, or halogen.

### <24>

The compound described in <20> or a salt thereof, which is selected from the group consisting of:
4-{[(2,3-dihydroxyphenyl)methyl](dimethyl)azaniumyl}butane-1-sulfonate,
3-{[(6-fluoro-2,3-dihydroxyphenyl)methyl](dimethyl)azaniumyl}propane-1-sulfonate,
hydrogen (3-{[(2,3-dihydroxyphenyl)methyl](dimethyl)azaniumyl}propyl)phosph onate,
5-{[(2,3-dihydroxyphenyl)methyl](dimethyl)azaniumyl}pentanoate,
{1-[(2,3-dihydroxyphenyl)methyl]-1-methylpiperidin-1-ium-4-yl}acetate,
1-[(2,3-dihydroxyphenyl)methyl]-1-methylpiperidin-1-ium-4-carboxylate,
4-{[2-(2,3-dihydroxyphenyl)ethyl](dimethyl)azaniumyl}butanoate,
2-{[2-(2,3-dihydroxyphenyl)ethyl](dimethyl)azaniumyl}ethane-1-sulfonate, and
3-[(2,3-dihydroxyphenyl)(dimethyl)azaniumyl]propane-1-sulfonate.

### <25>

The compound described in <24> or a salt thereof, which is selected from the group consisting of:
{1-[(2,3-dihydroxyphenyl)methyl]-1-methylpiperidin-1-ium-4-yl}acetate, and
2-{[2-(2,3-dihydroxyphenyl)ethyl](dimethyl)azaniumyl}ethane-1-sulfonate.

### <26>

The compound described in <24> or a salt thereof, which is selected from the group consisting of:
4-{[(2,3-dihydroxyphenyl)methyl](dimethyl)azaniumyl}butane-1-sulfonate, and
3-{[(6-fluoro-2,3-dihydroxyphenyl)methyl](dimethyl)azaniumyl}propane-1-sulfonate.

The present invention is not limited to each above embodiments, but can be altered by a skilled person in the art within the scope of the claims. The present invention also encompasses, in its technical scope, any embodiment derived by combining technical means disclosed in differing embodiments. Further, it is possible to form a new technical feature by combining the technical means disclosed in the respective embodiments.

### Examples

The following will provide Production Examples and Examples to describe the present invention in further detail.

In Examples, Production Examples, and Tables below, the following abbreviations are sometimes used.

PEx: Production Example number; Ex: Example number; PSyn: Production Example number produced with a similar method; ESyn: Example number produced with a similar method; Str: Chemical structural formula; Me: Methyl group; Et: Ethyl group; Data1: Physicochemical data in Production Example; NMR-D: Characteristic peak δ (ppm) in 1H-NMR in DMSO-d6; ESI+: m/z value in mass-spectrometric values (ionization method ESI; indicating (M+H)+ unless otherwise noted, or indicating (M+)+ for ESI(M+)+ in Tables below); APCI/ESI(M+)+: m/z value in mass-spectrometric values (ionization methods APCI and ESI) (note that Production Example 27 indicates Mass data of an oleic acid portion excluding iron ions, and ESI thereof indicates (M-)-); Data2: Physicochemical data of Example; SEC(min): Flow-out time of nanoparticle under conditions of Test Example 2; 3K: 3K purified particles which have been purified with a filter described below; 10K: 10K purified particles which have been purified with a filter described below; THF: Tetrahydrofuran; DMF: N,N-dimethylformamide; OA: Oleic acid; MEAA: [2-(2-methoxyethoxy)ethoxy]acetic acid; TBAF trihydrate: tetrabutylammonium fluoride trihydrate; PBS: Phosphate buffered saline; SNP-OA: Iron oxide nanoparticle to which OA is coordinately bound; SNP-MEAA: Iron oxide nanoparticle to which MEAA is coordinately bound; Br⁻ (in structural formula): Bromide ion; and I⁻ (in structural formula): Iodide ion. In reversed phase column chromatography, a column was used which was filled with silica gel whose surface was modified with ODS (octadecylsilyl group).

An Amicon Ultracentrifuge 3K filter (Merck Millipore) used in purification of an iron oxide nanoparticle is referred to as "Amicon 3K filter". Furthermore, similar filters for different molecular weight cutoffs 10K, 30K, 50K, and 100K are referred to as "Amicon 10K filter", "Amicon 30K filter", "Amicon 50K filter", and "Amicon 100K filter", respectively. Particles purified by ultrafiltration at the molecular weight cutoffs of 30K, 10K, and 3K are referred to as "30K purified particles", "10K purified particles", and "3K purified particles", respectively.

Filtering operation of particles with use of Agilent Captiva Premium Syringe Filters (Regenerated Cellulose, 15 mm, pore size: 0.2 µm) or YMC Duo-Filter (XQUO15, pore size: 0.2 µm) is referred to as "filtered with a membrane (0.2 µm)".

The dashed line in Tables of Examples below represents a coordinate bond with the metal atom on the surface of the metal particle.

Compounds of Production Examples and nanoparticles of Examples shown in Tables below were produced as in Production Examples and Examples below or in manners similar to those Production Examples and Examples.

Production Examples show examples of producing a zwitterionic ligand compound, an iron-oleic acid complex, and an iron oxide nanoparticle coated with oleic acid (SNP-OA). Examples show examples of producing a nanoparticle which was derived directly from SNP-OA or derived via SNP-MEAA and to which the zwitterionic ligand compound is coordinately bound.

### Production Example 1

A 9.5 mol/L dimethylamine aqueous solution (7.1 mL) was added to 6-fluoro-2,3-dimethoxybenzaldehyde (2.50 g), and a resultant mixture was stirred for 15 hours at room temperature. Sodium borohydride (514 mg) was added to the mixture in a water bath, and a resultant mixture was stirred for 2 hours at room temperature. In an ice bath, concentrated hydrochloric acid was added (pH 1-2). An aqueous layer was washed twice with dichloromethane. A 1 mol/L sodium hydroxide aqueous solution was added to the aqueous layer (pH>11). A resultant mixture was subjected to extraction three times with dichloromethane, and an extracted substance was dried with anhydrous sodium sulfate. After filtration, a resultant filtrate was concentrated, and thus 1-(6-fluoro-2,3-dimethoxyphenyl)-N,N-dimethylmethanamine (2.46 g) was obtained.

### Production Example 2

Sodium triacetoxyborohydride (3.74 g) was added to a mixture of 4-fluoro-2,3-dimethoxybenzaldehyde (2.50 g), dichloromethane (75 mL), and a 2 mol/L dimethylamine THF solution (13.6 mL) in a water bath, and a resultant mixture was stirred for 1 hour at room temperature. Basic silica gel was added, and a resultant mixture was concentrated under reduced pressure. Purification was carried out by basic silica gel column chromatography (developing solvent: hexane-chloroform), and thus 1-(4-fluoro-2,3-dimethoxyphenyl)-N,N-dimethylmethanamine (2.81 g) was obtained.

### Production Example 3

A mixture of 1-(2,3-dimethoxyphenyl)-N,N-dimethylmethanamine (3.82 g), 1,2λ⁶-oxathiolane-2,2-dione (1.89 mL), and ethyl acetate (38.2 mL) was stirred for 7 days at room temperature. 1,2λ⁶-oxathiolane-2,2-dione (515 µL) was further added and stirred for 4 hours at 50°C. A resultant mixture was cooled down to room temperature, and a resultant solid substance was taken by filtration, washed with ethyl acetate, and dried under reduced pressure. Thus, 3-{[(2,3-dimethoxyphenyl) methyl] (dimethyl) azaniumyl}propane-1-sulfonate (5.41 g) was obtained.

### Production Example 4

A mixture of 1-(2,3-dimethoxyphenyl)-N,N-dimethylmethanamine (3.00 g), sodium carbonate (1.63 g), sodium 2-bromoethane-1-sulfonate (3.24 g), water (6 mL), and ethanol (30 mL) was stirred for 3 days at 75°C. Sodium 2-bromoethane-1-sulfonate (3.24 g) was further added and the mixture was stirred for 2 days at 80°C. Sodium 2-bromoethane-1-sulfonate (3.24 g) was further added and the mixture was stirred for 2 days at 80°C. A resultant mixture was cooled down to room temperature, and then was concentrated under reduced pressure. Water was added, and purification was carried out by reversed phase column chromatography (developing solvent: acetonitrile-water) to obtain 2-{[(2,3-dimethoxyphenyl) methyl] (dimethyl) azaniumyl}ethane-1-sulfonate (3.50 g).

### Production Example 5

A mixture of 1-(2,3-dimethoxyphenyl)-N,N-dimethylmethanamine (2.00 g), 1,2λ⁶-oxathiane-2,2-dione (1.36 mL), and ethyl acetate (20 mL) was stirred for 3 hours at 50°C and then stirred for 24 hours at 70°C. 1,2λ⁶-oxathiane-2,2-dione (1.04 mL) was further added and the mixture was stirred for 24 hours at 70°C. A resultant mixture was cooled down to room temperature, and a resultant solid substance was taken by filtration, washed with ethyl acetate, and dried under reduced pressure. Thus, 4-{[(2,3-dimethoxyphenyl)methyl](dimethyl)azaniumyl}butane-1-sulfonate (2.28 g) was obtained.

### Production Example 6

A mixture of 2-fluoro-4,5-dimethoxyaniline (2.50 g), 1,2λ⁶-oxathiolane-2,2-dione (1.54 mL), and acetonitrile (63 mL) was stirred for 8 hours at 115°C. 1,2λ⁶-oxathiolane-2,2-dione (0.64 mL) was further added and the mixture was stirred for 8 hours at 115°C. A resultant mixture was cooled down to room temperature, and a resultant solid substance was taken by filtration, washed with acetonitrile, and dried at 50°C under reduced pressure. Thus, 3-(2-fluoro-4,5-dimethoxyanilino)propane-1-sulfonic acid (4.00 g) was obtained.

### Production Example 7

3,4-dimethoxyaniline (1.66 g), potassium iodide (1.79 g), and potassium carbonate (2.49 g) were added to a mixture of 3-(2-chloroethoxy)propane-1-sulfonic acid (1.46 g), dioxane (22 mL), and water (11 mL), and the mixture was stirred overnight at 100°C. The reaction liquid was cooled down to room temperature and then concentrated, purified by reversed phase column chromatography (developing solvent: acetonitrile-water), and freeze-dried to obtain 3-[2-(3,4-dimethoxyanilino)ethoxy]propane-1-sulfonic acid (532 mg).

### Production Example 8

A mixture of 2-methoxy-N-(2-methoxyethyl)ethan-1-amine (3.0 mL), 1,2λ⁶-oxathiolane-2,2-dione (2.0 mL), and acetonitrile (27 mL) was stirred for 4 hours at 80°C. The mixture was cooled down to room temperature, and then concentrated. Diethyl ether was added to the mixture and the mixture was stirred for 2 hours at room temperature. Then, a resultant solid substance was taken by filtration and dried under reduced pressure at room temperature to obtain 3-[bis(2-methoxyethyl)amino]propane-1-sulfonic acid (5.00 g).

### Production Example 9

A mixture of 1-(2,3-dimethoxyphenyl)-N,N-dimethylmethanamine (1.70 g), sodium 3-chloro-2-hydroxypropane-1-sulfonate (3.42 g), potassium iodide (1.73 g), ethanol (26 mL), and water (7.7 mL) was stirred overnight at 80°C. The mixture was cooled down to room temperature, and then concentrated, purified by reversed phase column chromatography (developing solvent: acetonitrile-water), and freeze-dried to obtain 3-{[(2,3-dimethoxyphenyl) methyl] (dimethyl) azaniumyl}-2-hydroxypropane-1-sulfonate (2.17 g).

### Production Example 10

A mixture of 7,8-dimethoxy-1,2,3,4-tetrahydroisoquinoline (1.80 g), 1,2λ⁶-oxathiolane-2,2-dione (0.98 mL), potassium carbonate (1.29 g), and acetonitrile (45 mL) was stirred for 8 hours at 100°C. The mixture was cooled down to room temperature, and then water was added. The mixture was concentrated, purified by reversed phase column chromatography (developing solvent: acetonitrile-water), and freeze-dried to obtain 3-(7,8-dimethoxy-3,4-dihydroisoquinolin-2(1H)-yl)propane-1-sulfonic acid (1.79 g).

### Production Example 11

A mixture of 1-(2,3-dimethoxyphenyl)-N,N-dimethylmethanamine (1.30 g), diethyl (3-bromopropyl)phosphonate (1.66 mL), and ethanol (6.50 mL) was stirred for 6 hours at 80°C. The mixture was cooled down to room temperature, and then concentrated and purified by reversed phase column chromatography (developing solvent: acetonitrile-water) to obtain 3-(diethoxyphosphoryl)-N-[(2,3-dimethoxyphenyl)methyl]-N,N-dimethylpropan-1-aminium bromide (2.70 g).

### Production Example 12

A mixture of 3-[bis(2-methoxyethyl)amino]propane-1-sulfonic acid (3.00 g), 1-(chloromethyl)-2,3-dimethoxybenzene (4.39 g), potassium carbonate (1.95 g), and ethanol (45 mL) was stirred overnight at 80°C. The mixture was cooled down to room temperature, and then concentrated, purified by reversed phase column chromatography (developing solvent: acetonitrile-water), and freeze-dried to obtain 3-{[(2,3-dimethoxyphenyl)methyl]bis(2-methoxyethyl)azaniumyl}propane-1-sulfonate (3.09 g).

### Production Example 13

A mixture of diethyl (3-bromopropyl)phosphonate (2.53 g) and 3,4-dimethoxyaniline (3.00 g) was stirred for 6 hours at 95°C in an argon atmosphere. The mixture was cooled down to room temperature. Saturated aqueous sodium hydrogen carbonate solution was added, and a resultant mixture was subjected to extraction once with ethyl acetate. An organic layer thus obtained was washed once with brine, and dried with anhydrous magnesium sulfate. After filtration, a filtrate thus obtained was concentrated and purified by silica gel column chromatography (developing solvent; hexane-ethyl acetate, then ethyl acetate-methanol) to obtain diethyl [3-(3,4-dimethoxyanilino)propyl]phosphonate (1.74 g).

### Production Example 14

A mixture of 1-(2,3-dimethoxyphenyl)-N,N-dimethylmethanamine (2.00 g) and ethyl 4-bromobutanoate (2.60 g) was stirred for 3 hours at 80°C. The mixture was purified by reversed phase column chromatography (developing solvent; water-acetonitrile) to obtain N-[(2,3-dimethoxyphenyl)methyl]-4-ethoxy-N,N-dimethyl-4-oxobutan-1-aminium bromide (3.93 g).

### Production Example 15

A mixture of 1-(6-fluoro-2,3-dimethoxyphenyl)-N,N-dimethylmethanamine (1.20 g), 1,2λ⁶-oxathiolane-2,2-dione (990 µL), and ethyl acetate (12 mL) was stirred for 18 hours at 50°C. The mixture was cooled down to room temperature, and then a resultant solid substance was taken by filtration, washed with ethyl acetate, and dried under reduced pressure to obtain 3-{[(6-fluoro-2,3-dimethoxyphenyl) methyl] (dimethyl) azaniumyl}propane-1-sulfonate (1.79 g).

### Production Example 16

A mixture of 1-(2,3-dimethoxyphenyl)-N,N-dimethylmethanamine (2.00 g) and ethyl 5-bromopentanoate (2.79 g) was stirred for 3 hours at 80°C. The mixture was purified by reversed phase column chromatography (developing solvent; water-acetonitrile) to obtain N-[(2,3-dimethoxyphenyl)methyl]-5-ethoxy-N,N-dimethyl-5-oxopentan-1-aminium bromide (3.91 g).

### Production Example 17

A mixture of 3-(2-fluoro-4,5-dimethoxyanilino)propane-1-sulfonic acid (4.00 g), potassium carbonate (4.52 g), methyl iodide (7.7 mL), and methanol (60 mL) was stirred overnight at 50°C. The mixture was cooled down to room temperature, and then concentrated, purified by reversed phase column chromatography (developing solvent: acetonitrile-water), and freeze-dried to obtain 3-[(2-fluoro-4,5-dimethoxyphenyl)(dimethyl)azaniumyl]propane-1-sulfonate (4.34 g).

### Production Example 18

A mixture of 3-(3,4-dimethoxyanilino)propane-1-sulfonic acid (2.00 g), 1,4-diiodobutane (1.04 mL), potassium carbonate (2.21 g), dioxane (30 mL), and water (15 mL) was stirred overnight at 100°C. The mixture was cooled down to room temperature, and then concentrated, purified by reversed phase column chromatography (developing solvent: acetonitrile-water), and freeze-dried to obtain 3-[1-(3,4-dimethoxyphenyl)pyrrolidin-1-ium-1-yl]propane-1-sulfonate (2.37 g).

### Production Example 19

A mixture of 3-(3,4-dimethoxyanilino)propane-1-sulfonic acid (2.00 g), ethyl iodide (2.94 mL), potassium carbonate (2.41 g), and methanol (30 mL) was stirred overnight at 50°C. Methyl iodide (4.1 mL) was added, and a resultant mixture was then stirred overnight at 50°C. The mixture was cooled down to room temperature, and then concentrated, purified by reversed phase column chromatography (developing solvent: acetonitrile-water), and freeze-dried to obtain 3-[(3,4-dimethoxyphenyl)(ethyl)(methyl)azaniumyl]propane-1-sulfonate (2.14 g).

### Production Example 20

A mixture of 3-{[(2,3-dimethoxyphenyl) methyl] (dimethyl) azaniumyl}propane-1-sulfonate (5.41 g) and 57% hydroiodic acid (24 mL) was stirred for 15 hours at 110°C. After the mixture was cooled down to room temperature, water (30 mL) was added and a resultant mixture was concentrated under reduced pressure. This operation was repeated one more time. To a resultant mixture, water (6 mL) was added to dissolve the mixture, then acetone (100 mL) was added, and the mixture was stirred for 3 minutes in an ice bath. A resultant mixture was left still, and then a supernatant was removed by decantation. Water (6 mL) and acetone (75 mL) were further added, and a similar operation was carried out one more time. Water (6 mL) and acetone (75 mL) were added to a resultant mixture and the mixture was stirred for 3 minutes in an ice bath. Then, a resultant solid substance was taken by filtration, washed with acetone, and dried under reduced pressure to obtain 3-{[(2,3-dihydroxyphenyl)methyl](dimethyl)azaniumyl}propane-1-sulfonate (5.02 g).

### Production Example 21

In an argon atmosphere, a 1 mol/L tribromoborane dichloromethane solution (19.2 mL) was added dropwise into a mixture of 3-{[(2,3-dimethoxyphenyl)methyl]bis(2-methoxyethyl)azaniumyl}propane-1-sulfonate (2.59 g) and dichloromethane (52 mL) under dry ice-acetone bath cooling, and a resultant mixture was slowly heated to room temperature over 3 hours, and stirred for 2 hours at room temperature. Methanol was added under ice cooling, and a resultant mixture was stirred for 30 minutes at room temperature and concentrated under reduced pressure. Methanol was added to the residue and the resultant mixture was concentrated under reduced pressure again. This operation was carried out two more times, and a resultant mixture was purified by reversed phase column chromatography (developing solvent: acetonitrile-water) and freeze-dried to obtain 3-{[(2,3-dihydroxyphenyl) methyl] bis(2-methoxyethyl)azaniumyl}propane-1-sulfonate (674 mg).

### Production Example 22

A mixture of 4-{[(2,3-dimethoxyphenyl)methyl](dimethyl)azaniumyl}butane-1-sulfonate (2.28 g) and 57% hydroiodic acid (9.6 mL) was stirred for 4 hours at 110°C. After the mixture was cooled down to room temperature, water was added and a resultant mixture was concentrated under reduced pressure. This operation was repeated one more time. To a resultant mixture, water (4 mL) was added to dissolve the mixture, and then acetone (80 mL) was added and the mixture was stirred. A resultant mixture was left still, and then a supernatant was removed by decantation. Millipore ultrapure water (4 mL) and acetone (60 mL) were further added, and a similar operation was carried out. Ultrapure water (4 mL) and acetone (60 mL) were added to a resultant mixture and the mixture was stirred. Then, a resultant solid substance was taken by filtration, washed with acetone, and dried under reduced pressure to obtain 4-{[(2,3-dihydroxyphenyl) methyl] (dimethyl) azaniumyl}butane-1-sulfonate (2.45 g).

### Production Example 23

A mixture of 3-{[(6-fluoro-2,3-dimethoxyphenyl) methyl] (dimethyl) azaniumyl}propane-1-sulfonate (1.79 g) and 57% hydroiodic acid (7.5 mL) was stirred for 6 hours at 110°C. After the mixture was cooled down to room temperature, water was added and a resultant mixture was concentrated under reduced pressure. This operation was repeated one more time.

Acetone (70 mL) was added to a resultant mixture and the mixture was stirred under ice cooling. A resultant mixture was left still overnight to precipitate a solid substance, and stirred for 1 hour under ice cooling. A resultant mixture was left still, and then a supernatant was removed by decantation. Acetone was added to the mixture, and then a resultant solid substance was taken by filtration, washed with acetone, and dried under reduced pressure to obtain 3-{[(6-fluoro-2,3-dihydroxyphenyl)methyl](dimethyl)azaniumyl}propane-1-sulfonate (1.58 g).

### Production Example 24

A mixture of 3-(diethoxyphosphoryl)-N-[(2,3-dimethoxyphenyl)methyl]-N,N-dimethylpropan-1-aminium bromide (2.80 g) and 57% hydroiodic acid (8 mL) was stirred for 18 hours at 100°C. After the mixture was cooled down to room temperature, water and acetone were added and a resultant mixture was concentrated under reduced pressure. Water was added to a resultant mixture and the mixture was concentrated under reduced pressure. Water was added to a resultant mixture, an insoluble matter was filtered off, and the filtrate was concentrated under reduced pressure. Acetone was added to a resultant mixture, and a resultant solid substance was filtered. The solid substance was washed with acetone and dried under reduced pressure to obtain N-[(2,3-dihydroxyphenyl)methyl]-N,N-dimethyl-3-phosphonopropan-1-aminium iodide (571 mg).

### Production Example 25

A mixture of N-[(2,3-dimethoxyphenyl)methyl]-4-ethoxy-N,N-dimethyl-4-oxobutan-1-aminium bromide (3.91 g) and 57% hydroiodic acid (22.5 g) was stirred for 15 hours at 110°C. The mixture was concentrated, and water was added to a resultant residue, and a resultant mixture was concentrated under reduced pressure. This operation was repeated one more time. Acetone was added to the mixture and the mixture was cooled in an ice bath, and a supernatant was removed. Acetone was added to the mixture and the mixture was cooled in an ice bath, and a resultant solid substance was filtered. The solid substance was washed with acetone to obtain 3-carboxy-N-[(2,3-dihydroxyphenyl)methyl]-N,N-dimethylpropan-1-aminium iodide (2.13 g).

### Production Example 26

A mixture of N-[(2,3-dimethoxyphenyl)methyl]-5-ethoxy-N,N-dimethyl-5-oxopentan-1-aminium bromide (3.90 g) and 57% hydroiodic acid (22.0 g) was stirred for 16 hours at 110°C. The mixture was concentrated, and water was added to a resultant residue, and a resultant mixture was concentrated under reduced pressure. This operation was repeated one more time. Acetone was added to the mixture and cooled in an ice bath, and a supernatant was removed. Acetone was added to the mixture and the mixture was cooled in an ice bath, and a resultant solid substance was filtered. The solid substance was washed with acetone to obtain 4-carboxy-N-[(2,3-dihydroxyphenyl)methyl]-N,N-dimethylbutan-1-aminium iodide (1.33 g). The entire filtrate was concentrated, and a resultant residue was purified by reversed phase column chromatography (developing solvent; water-acetonitrile). Acetone was added to the solid substance generated by concentration, and the solid substance was filtered. The solid substance was washed with acetone to obtain 4-carboxy-N-[(2,3-dihydroxyphenyl)methyl]-N,N-dimethylbutan-1-aminium iodide (1.29 g).

### Production Example 27

Iron(III) chloride hexahydrate (5.80 g), sodium oleate (19.5 g), ethanol (43 mL), water (33 mL), and hexane (75 mL) were mixed together, and the mixture was heated to reflux for 4 hours at 70°C in an argon atmosphere. After cooling, the mixture was put into a separatory funnel to remove an aqueous layer. 50 mL of water was added, and an organic layer was washed and collected. This operation was repeated two more times (in the second time, 50% methanol water was used). A resultant organic layer was dried with sodium sulfate and concentrated under reduced pressure to obtain an oleic acid-iron complex (FeOA₃, 19.2 g).

### Production Example 28

A mixture of FeOA₃ (6.53 g), oleyl alcohol (11.7 g), and diphenyl ether (36.4 g) was stirred for 2 hours at 90°C under reduced pressure. Then, the pressure was changed to normal atmospheric pressure with use of argon, and the mixture was heated to a bath temperature of 213°C over a period of 16 minutes and was stirred for 30 minutes after an internal temperature exceeded 200°C. After the mixture was cooled down to room temperature, hexane (5 mL) and acetone (150 mL) were added. A resultant mixture was centrifuged at 8000 rpm for 10 minutes at 10°C, and a supernatant was removed. Hexane (24 mL) was added to a resultant precipitate, and acetone (150 mL) was further added, and then a resultant mixture was centrifuged at 8000 rpm for 10 minutes at 10°C, and a supernatant was removed. This operation was repeated one more time, and a resultant precipitate was dried under reduced pressure to obtain an iron oxide nanoparticle (SNP-OA, 992 mg) having a surface to which oleic acid is coordinately bound.

### Production Example 33

Sodium carbonate (15.6 g) and sodium 2-bromoethane-1-sulfonate (23.3 g) were added to a mixture of 2-(2,3-dimethoxyphenyl)-N,N-dimethylethan-1-amine (7.71 g), water (15.4 mL), and ethanol (77 mL), and a resultant mixture was stirred for 18 hours at 80°C. Sodium 2-bromoethane-1-sulfonate (11.7 g), sodium carbonate (7.81 g), ethanol (20 mL), and water (4 mL) were added and the mixture was stirred for 1 day at 80°C. A resultant mixture was concentrated and then purified by reversed phase column chromatography (developing solvent: water-acetonitrile) to obtain 2-{[2-(2,3-dimethoxyphenyl)ethyl](dimethyl)azaniumyl}ethane-1-sulfonate (9.00 g).

### Production Example 35

A mixture of 2,3-dimethoxyaniline (5.61 g), 1,2λ⁶-oxathiolane-2,2-dione (5.83 g), and acetonitrile (140 mL) was refluxed for 8 hours. The mixture was cooled down to room temperature, and then stirred in an ice bath. A resultant solid substance was taken by filtration and washed with cooled acetonitrile to obtain 3-(2,3-dimethoxyanilino)propane-1-sulfonic acid (5.59 g).

### Production Example 38

A mixture of 3-(2,3-dimethoxyanilino)propane-1-sulfonic acid (5.58 g), potassium carbonate (6.72 g), methyl iodide (11.4 mL), and methanol (85 mL) was stirred for 8 hours at 50°C. Methyl iodide (11.4 mL) was added and the mixture was stirred for 24 hours at 50°C. An insoluble matter was filtered and then the filtrate was concentrated and purified with SEPABEADS (registered trademark) SP207SS. A solid substance obtained by concentration was dissolved in ethanol while being heated. A resultant mixture was cooled down to room temperature and then stirred in an ice bath. A resultant solid substance was filtered and washed with cooled ethanol to obtain 3-[(2,3-dimethoxyphenyl) (dimethyl) azaniumyl]propane- 1-sulfonate (5.40 g).

### Production Example 43

A mixture of 3-[(2,3-dimethoxyphenyl) (dimethyl) azaniumyl]propane-1-sulfonate (5.40 g) and 57% hydroiodic acid (40 g) was refluxed for 8 hours. After the mixture was cooled down to room temperature, water was added and a resultant mixture was concentrated under reduced pressure. This operation was repeated two more times. To a resultant mixture, water (3 mL) was added to dissolve the mixture, then acetone (50 mL) was added, and the mixture was stirred for 30 minutes in an ice bath. A resultant mixture was left still, and then a supernatant was removed by decantation. Water (3 mL) and acetone (40 mL) were further added, and a similar operation was carried out one more time. Water (3 mL) and acetone (40 mL) were added to a resultant mixture and the mixture was stirred for 30 minutes in an ice bath. A resultant solid substance was filtered and washed with acetone to obtain 3-[(2,3-dihydroxyphenyl) (dimethyl) azaniumyl]propane-1-sulfonate (4.35 g).

### Production Example 50

A mixture of 2-{[2-(2,3-dimethoxyphenyl)ethyl](dimethyl)azaniumyl}ethane-1-sulfonate (9.00 g) and 57% hydroiodic acid (40 mL) was stirred for 15 hours at 100°C. The mixture was concentrated, and acetone was added. The mixture was stirred for 5 minutes in an ice bath. A resultant solid substance was filtered and washed with acetone to obtain 2-{[2-(2,3-dihydroxyphenyl)ethyl](dimethyl)azaniumyl}ethane-1-sulfonate (3.40 g).

### Production Example 58

A mixture of methyl (piperidin-4-yl)acetate monohydrochloride (5.00 g), 1-(chloromethyl)-2,3-dimethoxybenzene (5.78 g), potassium carbonate (4.64 g), and acetonitrile (50 mL) was stirred overnight at room temperature. The reaction mixture was filtered, and the filtrate was concentrated and purified by basic silica gel column chromatography (developing solvent: hexane-ethyl acetate) to obtain methyl {1-[(2,3-dimethoxyphenyl)methyl]piperidin-4-yl}acetate (4.90 g).

### Production Example 59

A mixture of 2-(2,3-dimethoxyphenyl)-N,N-dimethylethan-1-amine (10.9 g) and ethyl 4-bromobutanoate (8.28 mL) was stirred for 3 hours at 80°C. The mixture was purified by reversed phase column chromatography (developing solvent; water-acetonitrile) to obtain N-[2-(2,3-dimethoxyphenyl)ethyl]-4-ethoxy-N,N-dimethyl-4-oxobutan-1-aminium bromide (15.7 g).

### Production Example 60

A mixture of 2,3-dimethoxyaniline (5.00 g) and 1,2λ⁶-oxathiane-2,2-dione (5.78 g) was stirred for 24 hours at 95°C. The mixture was purified by reversed phase column chromatography (developing solvent: acetonitrile-water). A solid substance obtained by concentration was washed with acetonitrile to obtain 4-(2,3-dimethoxyanilino)butane-1-sulfonic acid (4.78 g).

### Production Example 61

A mixture of 2,3-dimethoxyaniline (5.00 g), ethyl 5-bromopentanoate (8.19 g), and triethylamine (3.96 g) was stirred for 5 days at room temperature. Water was added and a resultant mixture was subjected to extraction once with ethyl acetate. An organic layer was washed once with brine, and dried with anhydrous magnesium sulfate. After filtration, a resultant filtrate was concentrated and purified by silica gel column chromatography (developing solvent; first time: hexane-ethyl acetate, second time: chloroform-ethyl acetate) to obtain ethyl 5-(2,3-dimethoxyanilino)pentanoate(6.26 g).

### Production Example 62

A mixture of methyl {1-[(2,3-dimethoxyphenyl)methyl]piperidin-4-yl}acetate (4.90 g), methyl iodide (5.0 mL), and methanol (74 mL) was stirred for 4 hours at 50°C. The mixture was cooled down to room temperature, and then concentrated and purified by reversed phase silica gel column chromatography (developing solvent: water-acetonitrile) to obtain 1-[(2,3-dimethoxyphenyl)methyl]-4-(2-methoxy-2-oxoethyl)-1-methylpiperidin-1-ium iodide (6.54 g).

### Production Example 63

A mixture of ethyl 1-[(2,3-dimethoxyphenyl)methyl]piperidin-4-carboxylate (18.8 g), methyl iodide (19.1 mL), and ethanol (188 mL) was stirred for 4 hours at 50°C. The mixture was cooled down to room temperature, and then concentrated and purified by reversed phase silica gel column chromatography (developing solvent: water-acetonitrile) to obtain 1-[(2,3-dimethoxyphenyl)methyl]-4-(ethoxycarbonyl)-1-methylpiperidin-1-ium iodide (25.9 g).

### Production Example 64

A mixture of 1-[(2,3-dimethoxyphenyl)methyl]-4-(2-methoxy-2-oxoethyl)-1-methylpiperidin-1-ium iodide (6.54 g) and 57% hydroiodic acid (19 mL) was stirred for 6 hours at 100°C. After the mixture was cooled down to room temperature, water was added and a resultant mixture was concentrated under reduced pressure. This operation was repeated two more times. Acetone (30 mL) was added and the mixture was stirred at room temperature, and then cooled in an ice bath and left still, and a supernatant was removed by decantation. Acetone was further added, and a similar operation was carried out two more times. Acetone (30 mL) was added and the mixture was stirred at room temperature, and then cooled in an ice bath, and a resultant solid substance was taken by filtration to obtain 4-(carboxymethyl)-1-[(2,3-dihydroxyphenyl)methyl]-1-methylpiperidin-1-ium iodide (4.49 g).

### Production Example 65

A mixture of 1-[(2,3-dimethoxyphenyl)methyl]-4-(ethoxycarbonyl)-1-methylpiperidin-1-ium iodide (25.9 g) and 57% hydroiodic acid (76 mL) was stirred overnight at 100°C. After the mixture was cooled down to room temperature, water was added and a resultant mixture was concentrated under reduced pressure. This operation was repeated two more times. Acetone was added and the mixture was stirred at room temperature, and then a resultant mixture was cooled in an ice bath and left still, and a supernatant was removed by decantation. Acetone was further added, and a similar operation was carried out one more time. Acetone was added and the mixture was stirred at room temperature, and then cooled in an ice bath, and a resultant solid substance was taken by filtration to obtain 4-carboxy-1-[(2,3-dihydroxyphenyl) methyl]-1-methylpiperidin-1-ium iodide (10.8 g). A resultant filtrate was concentrated and purified by reversed phase silica gel column chromatography (developing solvent: water-acetonitrile) to obtain 4-carboxy-1-[(2,3-dihydroxyphenyl)methyl]-1-methylpiperidin-1-ium iodide (12.0 g).

### Production Example 66

A mixture of N-[2-(2,3-dimethoxyphenyl)ethyl]-4-ethoxy-N,N-dimethyl-4-oxobutan-1-aminium bromide (15.7 g) and 57% hydroiodic acid (52 mL) was stirred for 18 hours at 100°C. The mixture was concentrated, and water was added to a resultant residue, and a resultant mixture was concentrated under reduced pressure. Acetonitrile was added to the mixture. A resultant mixture was cooled in an ice bath, and a resultant solid substance was precipitated and then the mixture was concentrated. Acetone was added to this and the mixture was stirred for 10 minutes at room temperature, and then a resultant solid substance was filtered to obtain 3-carboxy-N-[2-(2,3-dihydroxyphenyl)ethyl]-N,N-dimethylpropan-1-aminium iodide (14.8 g).

### Example 1

A mixture of SNP-OA (100 mg), MEAA (2.5 mL), and methanol (7.5 mL) was stirred for 5 hours at 70°C in an argon atmosphere. The mixture was cooled down to room temperature, and then was concentrated under reduced pressure. Acetone (24 mL) and hexane (96 mL) were added, and a resultant mixture was divided into six portions, and each of the six portions was centrifuged at 7000 rpm for 10 minutes at 10°C to remove a supernatant. This operation was repeated one more time to obtain SNP-MEAA.

A mixture of 3-{[(2,3-dihydroxyphenyl)methyl](dimethyl)azaniumyl}propane-1-sulfonate (1.19 g), DMF (25 mL), and water (17 mL) was dissolved while being heated, and sodium hydrogen carbonate (700 mg) was added to the mixture. A DMF (8 mL) solution of the above SNP-MEAA was added to the mixture, and the mixture was stirred for 16 hours at room temperature in an argon atmosphere. The reaction mixture was divided into six portions with use of water (3 mL), acetone (30 mL) was added to each of the six portions, and each of the six portions was centrifuged at 7000 rpm for 10 minutes at 10°C to remove a supernatant. A resultant precipitate was dispersed in PBS, and the mixture was centrifuged at 5800 rpm for 30 minutes at 10°C with use of an Amicon 30K filter. A resultant filtrate was centrifuged at 5800 rpm for 30 minutes at 10°C with use of an Amicon 10K filter. The series of operations was carried out three more times. Water was added to the concentrated liquid on the Amicon 30K filter, and a resultant mixture was centrifuged at 5800 rpm for 30 minutes at 10°C. A resultant filtrate was centrifuged at 5800 rpm for 30 minutes at 10°C with use of an Amicon 10K filter. The series of operations was carried out two more times. Water was added to the concentrated liquid on the Amicon 10K filter, and a resultant mixture was centrifuged at 5800 rpm for 30 minutes at 10°C. This operation was carried out seven more times. The concentrated liquid was filtered with a membrane (0.2 µm), and freeze-dried to obtain 10K purified particles (21.2 mg). A filtrate by washing with the Amicon 10K filter was centrifuged at 5800 rpm for 60 minutes at 10°C with use of an Amicon 3K filter. Water was further added to this and a resultant mixture was centrifuged at 5800 rpm for 60 minutes at 10°C. The concentrated liquid was filtered with a membrane (0.2 µm), and freeze-dried to obtain 3K purified particles (41.3 mg).

### Example 2

A mixture of SNP-OA (20 mg), MEAA (0.5 mL), and methanol (1.5 mL) was stirred for 6 hours at 70°C in an argon atmosphere.

After the mixture was cooled down to room temperature, acetone (4 mL) and hexane (16 mL) were added, and a resultant mixture was centrifuged at 7800 rpm for 10 minutes at 10°C, and a supernatant was removed. This operation was repeated three times with use of acetone (1 mL) and hexane (4 mL), and thus SNP-MEAA was obtained.

Sodium hydrogen carbonate (53 mg) was added to a mixture of 3-[(2-fluoro-4,5-dihydroxyphenyl) (dimethyl) azaniumyl]propane-1-sulfonate (266 mg) and water (3.3 mL). A DMF (6.6 mL) solution of the above SNP-MEAA was added to the mixture, and the mixture was stirred for 15 hours at room temperature in an argon atmosphere. The reaction mixture was divided into two portions with use of water (1.5 mL), acetone (30 mL) was added to each of the two portions, and each of the two portions was centrifuged at 7800 rpm for 10 minutes at 10°C to remove a supernatant.

A resultant precipitate was dispersed in PBS, and the mixture was centrifuged at 5800 rpm for 30 minutes at 10°C with use of an Amicon 100K filter. A resultant filtrate was centrifuged at 5800 rpm for 30 minutes at 10°C with use of an Amicon 10K filter. The series of operations was repeated three more times. Water was added to the concentrated liquid on the Amicon 10K filter, and a resultant mixture was centrifuged at 5800 rpm for 30 minutes at 10°C. This operation was carried out two more times. The concentrated liquid was filtered with a membrane (0.2 µm), and freeze-dried to obtain 10K purified particles (9.9 mg). A filtrate obtained through washing carried out first three times with the Amicon 10K filter was centrifuged at 5800 rpm for 1 hour at 10°C with use of an Amicon 3K filter. Water was further added to this and a resultant mixture was centrifuged at 5800 rpm for 1 hour at 10°C. This operation was carried out seven more times. The concentrated liquid was filtered with a membrane (0.2 µm), and freeze-dried to obtain 3K purified particles (3.2 mg).

### Example 3

A mixture of SNP-OA (20 mg), MEAA (0.5 mL), and methanol (1.5 mL) was stirred for 6 hours at 70°C in an argon atmosphere.

After the mixture was cooled down to room temperature, acetone (4 mL) and hexane (16 mL) were added, and a resultant mixture was centrifuged at 7800 rpm for 10 minutes at 10°C, and a supernatant was removed. This operation was repeated three times with use of acetone (1 mL) and hexane (4 mL), and thus SNP-MEAA was obtained.

Sodium hydrogen carbonate (53 mg) was added to a mixture of 3-(7,8-dihydroxy-2-methyl-3,4-dihydroisoquinolin-2-ium-2(1H)-yl)propane-1-sulfonate (274 mg) and water (6.6 mL). A DMF (13.2 mL) solution of the above SNP-MEAA was added to the mixture, and the mixture was stirred for 17 hours at 50°C in an argon atmosphere.

The reaction mixture was divided into four portions with use of water (3 mL), acetone (30 mL) was added to each of the four portions, and each of the four portions was centrifuged at 7800 rpm for 10 minutes at 10°C to remove a supernatant.

A resultant precipitate was dispersed in PBS, and the mixture was centrifuged at 5800 rpm for 30 minutes at 10°C with use of an Amicon 100K filter. A resultant filtrate was centrifuged at 5800 rpm for 30 minutes at 10°C with use of an Amicon 10K filter. The series of operations was repeated three more times. Water was added to the concentrated liquid on the Amicon 10K filter, and a resultant mixture was centrifuged at 5800 rpm for 30 minutes at 10°C. This operation was carried out two more times. The concentrated liquid was filtered with a membrane (0.2 µm), and freeze-dried to obtain 10K purified particles (17.3 mg). A filtrate obtained through washing carried out first three times with the Amicon 10K filter was centrifuged at 5800 rpm for 1 hour at 10°C with use of an Amicon 3K filter. Water was further added and a resultant mixture was centrifuged at 5800 rpm for 1 hour at 10°C. This operation was carried out seven more times. The concentrated liquid was filtered with a membrane (0.2 µm), and freeze-dried to obtain 3K purified particles (11.1 mg).

### Example 4

A mixture of SNP-OA (40 mg), MEAA (1 mL), and methanol (3 mL) was stirred for 6 hours at 70°C in an argon atmosphere. The mixture was cooled down to room temperature, and then was concentrated under reduced pressure. Acetone (8 mL) and hexane (32 mL) were added, and a resultant mixture was divided into two portions, and each of the two portions was centrifuged at 7000 rpm for 10 minutes at 10°C to remove a supernatant. Acetone (6 mL) and hexane (24 mL) were added to this, and this operation was repeated one more time to obtain SNP-MEAA.

A mixture of 3-[(2,3-dihydroxyphenyl) (dimethyl) azaniumyl]propane-1-sulfonate (500 mg) and water (14.7 mL) was dissolved while being heated, and sodium hydrogen carbonate (130 mg) was added to the mixture. A DMF (2 mL) solution of the above SNP-MEAA was added to the mixture, and the mixture was stirred for 16 hours at room temperature in an argon atmosphere. The reaction mixture was divided into four portions with use of water (2 mL), acetone (30 mL) was added to each of the four portions, and each of the four portions was centrifuged at 7000 rpm for 3 minutes at 10°C to remove a supernatant. A resultant precipitate was dispersed in PBS, and the mixture was centrifuged at 5800 rpm for 10 minutes at 10°C with use of an Amicon 50K filter. A resultant filtrate was centrifuged at 5800 rpm for 30 minutes at 10°C with use of an Amicon 30K filter. The series of operations was carried out two more times. Water was added to the concentrated liquid on the Amicon 30K filter, and a resultant mixture was centrifuged at 5800 rpm for 30 minutes at 10°C. This operation was repeated one more time. The concentrated liquid was filtered with a membrane (0.2 µm), and freeze-dried to obtain 30K purified particles (2.1 mg). A filtrate obtained through washing with the Amicon 30K filter was centrifuged at 5800 rpm for 30 minutes at 10°C with use of an Amicon 10K filter. Water was further added to this and a resultant mixture was centrifuged at 5800 rpm for 30 minutes at 10°C. This operation was carried out six more times. The concentrated liquid was filtered with a membrane (0.2 µm), and freeze-dried to obtain 10K purified particles (1.9 mg). A filtrate obtained through washing with the Amicon 10K filter was centrifuged at 5800 rpm for 60 minutes at 10°C with use of an Amicon 3K filter. Water was further added to this and a resultant mixture was centrifuged at 5800 rpm for 60 minutes at 10°C. This operation was carried out five more times. The concentrated liquid was filtered with a membrane (0.2 µm), and freeze-dried to obtain 3K purified particles (5.0 mg).

### Example 5

A mixture of SNP-OA (20 mg), MEAA (0.5 mL), and methanol (1.5 mL) was stirred for 6 hours at 70°C in an argon atmosphere. After the mixture was cooled down to room temperature, acetone (8 mL) and hexane (32 mL) were added, and a resultant mixture was divided into two portions, and each of the two portions was centrifuged at 7300 rpm for 5 minutes at 10°C to remove a supernatant. This operation was repeated two times with use of acetone (6 mL) and hexane (24 mL), and thus SNP-MEAA was obtained.

A solution of 3-{[(3,4-dihydroxyphenyl)methyl](dimethyl)azaniumyl}propane-1-sulfonate (250 mg), DMF (5 mL), and water (3.3 mL) was dissolved while being heated, and sodium hydrogen carbonate (50 mg) was added to the solution. A DMF (1.7 mL) solution of the above SNP-MEAA was added to the solution, and the mixture was stirred for 21.5 hours at room temperature in an argon atmosphere. The reaction mixture was divided into two portions with use of water (3 mL), acetone (30 mL) was added to each of the two portions, and each of the two portions was centrifuged at 7300 rpm for 10 minutes at 10°C to remove a supernatant. A resultant precipitate was dispersed in PBS, and the mixture was centrifuged at 5800 rpm for 10 minutes at 10°C with use of an Amicon 50K filter. A resultant filtrate was centrifuged at 5800 rpm for 30 minutes at 10°C with use of an Amicon 10K filter. PBS was added to this and a resultant mixture was centrifuged at 5800 rpm for 30 minutes at 10°C. Water was added to this and a resultant mixture was centrifuged at 5800 rpm for 30 minutes at 10°C. This operation was carried out seven more times. The concentrated liquid was filtered with a membrane (0.2 µm), and freeze-dried to obtain 10K purified particles (9.3 mg). A filtrate obtained through washing carried out first three times with the Amicon 10K filter was centrifuged at 5800 rpm for 30 minutes at 10°C with use of an Amicon 3K filter. Water was further added to this and a resultant mixture was centrifuged at 5800 rpm for 30 minutes at 10°C. This operation was carried out five more times. Water was further added to this and a resultant mixture was centrifuged at 5800 rpm for 60 minutes at 10°C. The concentrated liquid was filtered with a membrane (0.2 µm), and freeze-dried to obtain 3K purified particles (2.6 mg).

### Example 6

A mixture of SNP-OA (100 mg), MEAA (2.5 mL), and methanol (7.5 mL) was stirred for 5 hours at 70°C in an argon atmosphere. The mixture was cooled down to room temperature, and then was concentrated under reduced pressure. Acetone (24 mL) and hexane (96 mL) were added, and a resultant mixture was divided into six portions, and each of the six portions was centrifuged at 7000 rpm for 10 minutes at 10°C to remove a supernatant. Thus, SNP-MEAA was obtained.

Sodium hydrogen carbonate (900 mg) was added to a mixture of 4-{[(2,3-dihydroxyphenyl)methyl](dimethyl)azaniumyl}butane-1-sulfonate (1.31 g) and water (40 mL). A DMF (8 mL) solution of the above SNP-MEAA was added to the mixture, and the mixture was stirred for 16 hours at room temperature in an argon atmosphere. The reaction mixture was divided into six portions with use of water (3 mL), acetone (30 mL) was added to each of the six portions, and each of the six portions was centrifuged at 7000 rpm for 10 minutes at 10°C to remove a supernatant. A resultant precipitate was dispersed in PBS, and the mixture was centrifuged at 5800 rpm for 30 minutes at 10°C with use of an Amicon 30K filter. A resultant filtrate was centrifuged at 5800 rpm for 30 minutes at 10°C with use of an Amicon 10K filter. The series of operations was carried out three more times. Water was added to the concentrated liquid on the Amicon 30K filter, and a resultant mixture was centrifuged at 5800 rpm for 30 minutes at 10°C. A resultant filtrate was centrifuged at 5800 rpm for 30 minutes at 10°C with use of an Amicon 10K filter. The series of operations was carried out six more times. Water was added to the concentrated liquid on the Amicon 10K filter, and a resultant mixture was centrifuged at 5800 rpm for 30 minutes at 10°C. This operation was carried out seven more times. The concentrated liquid was filtered with a membrane (0.2 µm), and freeze-dried to obtain 10K purified particles (117.7 mg). Filtrates obtained through washing with the Amicon 10K filter were sequentially centrifuged at 5800 rpm for 60 minutes at 10°C with use of an Amicon 3K filter. Water was further added and a resultant mixture was centrifuged at 5800 rpm for 60 minutes at 10°C. The concentrated liquid was filtered with a membrane (0.2 µm), and freeze-dried to obtain 3K purified particles (53.2 mg). Note that, as a result of subjecting the 3K purified particles to acid hydrolysis with use of hydrochloric acid and analyzing the acid hydrolysate with HPLC, the presence of a zwitterionic ligand, i.e., 4-{[(2,3-dihydroxyphenyl) methyl] (dimethyl) azaniumyl}butane-1-sulfonate was confirmed. As such, it was confirmed that the zwitterionic ligand was bound to the 3K purified particle by a coordinate bond.

HPLC conditions were as follows.
Column: YMC Triart C18
Eluent: 10 mM dipotassium hydrogen phosphate (pH 6.0)/acetonitrile (98 : 2)

### Example 7

A mixture of SNP-OA (100 mg), MEAA (2.5 mL), and methanol (7.5 mL) was stirred for 5 hours at 70°C in an argon atmosphere. The mixture was cooled down to room temperature, and then was concentrated under reduced pressure. Acetone (24 mL) and hexane (96 mL) were added, and a resultant mixture was divided into six portions, and each of the six portions was centrifuged at 7000 rpm for 10 minutes at 10°C to remove a supernatant. Thus, SNP-MEAA was obtained.

Sodium hydrogen carbonate (650 mg) was added to a mixture of 3-{[(6-fluoro-2,3-dihydroxyphenyl)methyl](dimethyl)azaniumyl}propane-1-sulfonate (1.32 g) and water (40 mL). A DMF (8 mL) solution of the above SNP-MEAA was added to the mixture, and the mixture was stirred for 16 hours at room temperature in an argon atmosphere. The reaction mixture was divided into six portions with use of water (3 mL), acetone (30 mL) was added to each of the six portions, and each of the six portions was centrifuged at 7000 rpm for 10 minutes at 10°C to remove a supernatant. A resultant precipitate was dispersed in PBS, and the mixture was centrifuged at 5800 rpm for 30 minutes at 10°C with use of an Amicon 30K filter. A resultant filtrate was centrifuged at 5800 rpm for 30 minutes at 10°C with use of an Amicon 10K filter. The series of operations was carried out three more times. Water was added to the concentrated liquid on the Amicon 30K filter, and a resultant mixture was centrifuged at 5800 rpm for 30 minutes at 10°C. A resultant filtrate was centrifuged at 5800 rpm for 30 minutes at 10°C with use of an Amicon 10K filter. The series of operations was carried out seven more times. Water was added to the concentrated liquid on the Amicon 10K filter, and a resultant mixture was centrifuged at 5800 rpm for 30 minutes at 10°C. This operation was carried out four more times. The concentrated liquid was filtered with a membrane (0.2 µm), and freeze-dried to obtain 10K purified particles (102.4 mg). Filtrates obtained through washing with the Amicon 10K filter were sequentially centrifuged at 5800 rpm for 60 minutes at 10°C with use of an Amicon 3K filter. Water was further added and a resultant mixture was centrifuged at 5800 rpm for 60 minutes at 10°C. The concentrated liquid was filtered with a membrane (0.2 µm), and freeze-dried to obtain 3K purified particles (41.2 mg).

### Example 8

A mixture of SNP-OA (20 mg), MEAA (0.5 mL), and methanol (1.5 mL) was stirred for 5 hours at 70°C in an argon atmosphere. The mixture was cooled down to room temperature, and then was concentrated under reduced pressure. Acetone (8 mL) and hexane (32 mL) were added, and a resultant mixture was divided into two portions, and each of the two portions was centrifuged at 7000 rpm for 3 minutes at 10°C to remove a supernatant. This operation was repeated one more time to obtain SNP-MEAA.

Sodium hydrogen carbonate (50 mg) was added to a mixture of N-[(2,3-dihydroxyphenyl)methyl]-N,N-dimethyl-3-phosphonopropan-1-aminium iodide (263 mg) and water (9.5 mL). A DMF (0.5 mL) solution of the above SNP-MEAA was added to the solution, and the mixture was stirred for 18 hours at room temperature in an argon atmosphere. The reaction mixture was dispersed in PBS, and the mixture was centrifuged at 5800 rpm for 30 minutes at 10°C with use of an Amicon 30K filter. A resultant filtrate was centrifuged at 5800 rpm for 60 minutes at 10°C with use of an Amicon 10K filter. The series of operations was carried out three more times. Water was added to the concentrated liquid on the Amicon 10K filter, and a resultant mixture was centrifuged at 5800 rpm for 60 minutes at 10°C. This operation was carried out two more times. The concentrated liquid was filtered with a membrane (0.2 µm), and freeze-dried to obtain 10K purified particles (3.0 mg). A filtrate obtained through washing with the Amicon 10K filter was centrifuged at 5800 rpm for 60 minutes at 10°C with use of an Amicon 3K filter. Water was further added to this and a resultant mixture was centrifuged at 5800 rpm for 60 minutes at 10°C. This operation was carried out two more times. The concentrated liquid was filtered with a membrane (0.2 µm), and freeze-dried to obtain 3K purified particles (6.0 mg).

### Example 9

A mixture of SNP-OA (20 mg), MEAA (0.5 mL), and methanol (1.5 mL) was stirred for 6 hours at 70°C in an argon atmosphere. After the mixture was cooled down to room temperature, acetone (8 mL) and hexane (32 mL) were added, and a resultant mixture was divided into two portions, and each of the two portions was centrifuged at 7000 rpm for 10 minutes at 10°C to remove a supernatant. This operation was repeated one time with use of acetone (6 mL) and hexane (24 mL), and thus SNP-MEAA was obtained.

Sodium hydrogen carbonate (122 mg) was added to a solution of 3-carboxy-N-[(2,3-dihydroxyphenyl)methyl]-N,N-dimethylpropan-1-aminium iodide (347 mg) and water (8 mL). A DMF (2 mL) solution of the above SNP-MEAA was added to the mixture, and the mixture was stirred for 17 hours at room temperature in an argon atmosphere. The reaction mixture was divided into two portions with use of water (1 mL), acetone (30 mL) was added to each of the two portions, and each of the two portions was centrifuged at 7000 rpm for 10 minutes at 10°C to remove a supernatant. A resultant precipitate was dispersed in PBS, and the mixture was centrifuged at 5800 rpm for 30 minutes at 10°C with use of an Amicon 30K filter. PBS was added to this and a resultant mixture was centrifuged at 5800 rpm for 30 minutes at 10°C. This operation was carried out one more time. Filtrates obtained through the Amicon 30K filter were sequentially centrifuged at 5800 rpm for 30-60 minutes at 10°C with use of an Amicon 10K filter. Water was further added and a resultant mixture was centrifuged at 5800 rpm for 60 minutes at 10°C. This operation was carried out 14 more times. The concentrated liquid was filtered with a membrane (0.2 µm), and freeze-dried to obtain 10K purified particles (23.2 mg). A filtrate obtained through washing carried out first three times with the Amicon 10K filter was centrifuged at 5800 rpm for 30-60 minutes at 10°C with use of an Amicon 3K filter. Water was further added to this and a resultant mixture was centrifuged at 5800 rpm for 60 minutes at 10°C. This operation was carried out 13 more times. The concentrated liquid was filtered with a membrane (0.2 µm), and freeze-dried to obtain 3K purified particles (7.2 mg).

### Example 10

A mixture of SNP-OA (20 mg), MEAA (0.5 mL), and methanol (1.5 mL) was stirred for 6 hours at 70°C in an argon atmosphere. After the mixture was cooled down to room temperature, acetone (8 mL) and hexane (32 mL) were added, and a resultant mixture was divided into two portions, and each of the two portions was centrifuged at 7000 rpm for 10 minutes at 10°C to remove a supernatant. This operation was repeated one time with use of acetone (6 mL) and hexane (24 mL), and thus SNP-MEAA was obtained.

Sodium hydrogen carbonate (123 mg) was added to a solution of 4-carboxy-N-[(2,3-dihydroxyphenyl)methyl]-N,N-dimethylbutan-1-aminium iodide (359 mg) and water (8 mL). A DMF (2 mL) solution of the above SNP-MEAA was added to the mixture, and the mixture was stirred for 20 hours at room temperature in an argon atmosphere. The reaction mixture was divided into two portions with use of water (1 mL), acetone (30 mL) was added to each of the two portions, and each of the two portions was centrifuged at 7000 rpm for 10 minutes at 10°C to remove a supernatant. A resultant precipitate was dispersed in PBS, and the mixture was centrifuged at 5800 rpm for 30 minutes at 10°C with use of an Amicon 30K filter. PBS was further added to this and a resultant mixture was centrifuged at 5800 rpm for 30 minutes at 10°C. This operation was carried out one more time. Filtrates obtained through the Amicon 30K filter were sequentially centrifuged at 5800 rpm for 30-60 minutes at 10°C with use of an Amicon 10K filter. Water was further added and a resultant mixture was centrifuged at 5800 rpm for 60 minutes at 10°C. This operation was carried out 13 more times. The concentrated liquid was filtered with a membrane (0.2 µm), and freeze-dried to obtain 10K purified particles (24.2 mg). A filtrate obtained through washing carried out first four times with the Amicon 10K filter was centrifuged at 5800 rpm for 30-60 minutes at 10°C with use of an Amicon 3K filter. Water was further added to this and a resultant mixture was centrifuged at 5800 rpm for 60 minutes at 10°C. This operation was carried out 11 more times. The concentrated liquid was filtered with a membrane (0.2 µm), and freeze-dried to obtain 3K purified particles (8.8 mg).

### Example 11

Sodium hydrogen carbonate (34 mg) was added to a mixture of 4-{[(2,3-dihydroxyphenyl)methyl](dimethyl)azaniumyl}butane-1-sulfonate (275 mg) and water (2.2 mL). This solution was added to a mixture of SNP-OA (20 mg) and chloroform (2.5 mL), and then a mixture of TBAF trihydrate (63 mg) and water (300 µL) was added and the mixture was stirred for 16 hours at room temperature in an argon atmosphere. An aqueous layer was separated, and a chloroform layer was subjected to extraction twice with water. The aqueous layer was collected and dispersed in PBS, put onto an Amicon 30K filter, and the mixture was centrifuged at 5800 rpm for 15 minutes at 10°C. A resultant filtrate was centrifuged at 5800 rpm for 30 minutes at 10°C with use of an Amicon 10K filter. The series of operations was carried out three more times. Water was added to the concentrated liquid on the Amicon 30K filter, and a resultant mixture was centrifuged at 5800 rpm for 15 minutes at 10°C. A resultant filtrate was centrifuged at 5800 rpm for 30 minutes at 10°C with use of an Amicon 10K filter. The series of operations was carried out two more times. Water was added to the concentrated liquid on the Amicon 10K filter, and a resultant mixture was centrifuged at 5800 rpm for 30 minutes at 10°C. This operation was carried out five more times. The concentrated liquid was filtered with a membrane (0.2 µm), and freeze-dried to obtain 10K purified particles (30.3 mg). Filtrates obtained through washing with the Amicon 10K filter were sequentially centrifuged at 5800 rpm for 60 minutes at 10°C with use of an Amicon 3K filter. Water was further added and a resultant mixture was centrifuged at 5800 rpm for 60 minutes at 10°C. The concentrated liquid was filtered with a membrane (0.2 µm), and freeze-dried to obtain 3K purified particles (17.8 mg).

### Example 12

3-{[(6-fluoro-2,3-dihydroxyphenyl)methyl](dimethyl)azaniumyl}propane-1-sulfonate (280 mg) was dissolved in water (2.2 mL), and sodium hydrogen carbonate (38 mg) was added to the solution. This solution was added to a solution of SNP-OA (20 mg) and chloroform (2.5 mL), and a solution of TBAF trihydrate (65 mg) and water (0.3 mL) was further added. A resultant mixture was stirred for 20 hours at room temperature in an argon atmosphere. An insoluble matter was filtered, and an aqueous layer was put onto an Amicon 30K filter and centrifuged at 5800 rpm for 30 minutes at 10°C. PBS was added to this and a resultant mixture was centrifuged at 5800 rpm for 30 minutes at 10°C. A filtrate obtained through washing carried out first two times with the Amicon 30K filter was centrifuged at 5800 rpm for 30 minutes at 10°C with use of an Amicon 10K filter. Water was further added to this and a resultant mixture was centrifuged at 5800 rpm for 30 minutes at 10°C. Water was further added to this and a resultant mixture was centrifuged at 5800 rpm for 60 minutes at 10°C. This operation was carried out 12 more times. The concentrated liquid was filtered with a membrane (0.2 µm), and freeze-dried to obtain 10K purified particles (13.5 mg). A filtrate obtained through washing carried out first five times with the Amicon 10K filter was centrifuged at 5800 rpm for 30-60 minutes at 10°C with use of an Amicon 3K filter. Water was further added to this and a resultant mixture was centrifuged at 5800 rpm for 60 minutes at 10°C. This operation was carried out nine more times. The concentrated liquid was filtered with a membrane (0.2 µm), and freeze-dried to obtain 3K purified particles (7.3 mg).

### Example 13

3,4-dihydroxy-N,N-dimethyl-N-(3-phosphonopropyl)anilinium iodide (265 mg) was dissolved in water (2.4 mL). To this aqueous solution, a solution of sodium hydrogen carbonate (100 mg), TBAF trihydrate (67 mg), and water (0.3 mL) was added. This solution was added to a solution of SNP-OA (20 mg) and chloroform (2.5 mL), and rinsed with water (0.6 mL). A resultant mixture was stirred for 18 hours at room temperature in an argon atmosphere. An insoluble matter was filtered, and an aqueous layer was put onto an Amicon 100K filter and centrifuged at 5800 rpm for 15 minutes at 10°C. A resultant filtrate was centrifuged at 5800 rpm for 30 minutes at 10°C with use of an Amicon 10K filter. Water was further added to this and a resultant mixture was centrifuged at 5800 rpm for 30 minutes at 10°C. Water was further added to this and a resultant mixture was centrifuged at 5800 rpm for 60 minutes at 10°C. This operation was carried out eight more times. The concentrated liquid was filtered with a membrane (0.2 µm), and freeze-dried to obtain 10K purified particles (1.0 mg). A filtrate obtained through washing carried out first two times with the Amicon 10K filter was centrifuged at 5800 rpm for 30-60 minutes at 10°C with use of an Amicon 3K filter. Water was further added to this and a resultant mixture was centrifuged at 5800 rpm for 60 minutes at 10°C. This operation was carried out seven more times. The concentrated liquid was filtered with a membrane (0.2 µm), and freeze-dried to obtain 3K purified particles (1.4 mg).

### Example 18

A mixture of SNP-OA (150 mg), MEAA (3.75 mL), and methanol (11.25 mL) was stirred for 5 hours at 70°C in an argon atmosphere. The mixture was cooled down to room temperature, and then was concentrated under reduced pressure. The mixture was divided into six centrifuge tubes, and acetone (4 mL) and hexane (16 mL) were added to each of the six centrifuge tubes, and each of the six centrifuge tubes was centrifuged at 7000 rpm for 10 minutes at 10°C to remove a supernatant. This operation was repeated one more time to obtain SNP-MEAA.

A mixture of 2-{[2-(2,3-dihydroxyphenyl) ethyl] (dimethyl)azaniumyl}ethane-1-sulfonate (1.97 g), water (25 mL), and sodium hydrogen carbonate (370 mg) was stirred at room temperature. A DMF (12.5 mL) solution of the above SNP-MEAA was added to the mixture, and the mixture was stirred for 16 hours at room temperature in an argon atmosphere. The reaction mixture was divided into six centrifuge tubes with use of water (3 mL), acetone (30 mL) was added to each of the six centrifuge tubes, and each of the six centrifuge tubes was centrifuged at 7000 rpm for 10 minutes at 10°C to remove a supernatant. A resultant precipitate was dispersed in water, and the mixture was centrifuged at 5800 rpm for 30 minutes at 10°C with use of an Amicon 30K filter. Water was added to this and a resultant mixture was centrifuged at 5800 rpm for 60 minutes at 10°C. This operation was carried out two more times. Filtrates obtained through the Amicon 30K filter were sequentially centrifuged at 5800 rpm for 60 minutes at 10°C with use of an Amicon 10K filter. Water was further added and a resultant mixture was centrifuged at 5800 rpm for 60 minutes at 10°C. This operation was carried out eight more times. The concentrated liquid was filtered with a membrane (0.2 µm), and freeze-dried to obtain 10K purified particles (16.1 mg). Filtrates obtained through the Amicon 10K filter were sequentially centrifuged at 5800 rpm for 60 minutes at 10°C with use of an Amicon 3K filter. Water was further added and a resultant mixture was centrifuged at 5800 rpm for 60 minutes at 10°C. The concentrated liquid was filtered with a membrane (0.2 µm), and freeze-dried to obtain 3K purified particles (56.0 mg).

### Example 25

A mixture of SNP-OA (150 mg), MEAA (3.8 mL), and methanol (11.3 mL) was stirred for 6 hours at 70°C in an argon atmosphere. The mixture was cooled down to room temperature, and then was concentrated under reduced pressure. The mixture was divided into four centrifuge tubes with acetone (28 mL), and hexane (28 mL) was added to each of the four centrifuge tubes, and each of the four centrifuge tubes was centrifuged at 7000 rpm for 10 minutes at 10°C to remove a supernatant. Acetone (7 mL) and hexane (28 mL) were added, and each of resultant mixtures was centrifuged at 7000 rpm for 10 minutes at 10°C to remove a supernatant. This operation was repeated one more time to obtain SNP-MEAA.

A mixture of 4-(carboxymethyl)-1-[(2,3-dihydroxyphenyl) methyl]-1-methylpiperidin-1-ium iodide (2.77 g) and water (60 mL) was dissolved while being heated, and sodium hydrogen carbonate (1.39 g) was added to the mixture. A DMF (15 mL) solution of the above SNP-MEAA was added to the mixture, and the mixture was stirred for 42 hours at room temperature in an argon atmosphere. The reaction mixture was divided into 12 centrifuge tubes with use of water (3 mL), acetone (40 mL) was added to each of the 12 centrifuge tubes, and each of the 12 centrifuge tubes was centrifuged at 7000 rpm for 10 minutes at 10°C to remove a supernatant. A resultant precipitate was dispersed in water, and the mixture was centrifuged at 5800 rpm for 30 minutes at 10°C with use of an Amicon 30K filter. Water was added to the concentrated liquid on the Amicon 30K filter, and a resultant mixture was centrifuged at 5800 rpm for 30 minutes at 10°C. This operation was repeated one more time. Water was added to the concentrated liquid on the Amicon 30K filter, and a resultant mixture was centrifuged at 5800 rpm for 60 minutes at 10°C. This operation was repeated four more times. Resultant filtrates were sequentially put onto an Amicon 10K filter, and each of those was centrifuged at 5800 rpm at 10°C for 30 minutes in first two times and then for 60 minutes in subsequent six times. Water was further added to the concentrated liquid on the Amicon 10K filter, and a resultant mixture was centrifuged at 5800 rpm for 60 minutes at 10°C. This operation was repeated 14 more times. The concentrated liquid was filtered with a membrane (0.2 µm), and freeze-dried to obtain 10K purified particles (96.0 mg). Filtrates obtained with use of the Amicon 10K filter were sequentially put onto an Amicon 3K filter and each of those was centrifuged at 5800 rpm at 10°C for 30 minutes in the first time and then for 60 minutes in subsequent 13 times. Water was further added to the concentrated liquid on the Amicon 3K filter, and a resultant mixture was centrifuged at 5800 rpm for 60 minutes at 10°C. This operation was repeated seven more times. The concentrated liquid was filtered with a membrane (0.2 µm), and freeze-dried to obtain 3K purified particles (121 mg).

### Example 26

A mixture of SNP-OA (150 mg), MEAA (3.8 mL), and methanol (11 mL) was stirred for 6 hours at 70°C in an argon atmosphere. The mixture was cooled down to room temperature, and then was concentrated under reduced pressure. The mixture was divided into four centrifuge tubes with acetone (28 mL), and hexane (28 mL) was added to each of the four centrifuge tubes, and each of the four centrifuge tubes was centrifuged at 7000 rpm for 10 minutes at 10°C to remove a supernatant. Acetone (7 mL) and hexane (28 mL) were added to each of these, and each of resultant mixtures was centrifuged at 7000 rpm for 10 minutes at 10°C to remove a supernatant. This operation was repeated one more time to obtain SNP-MEAA.

A mixture of 4-carboxy-1-[(2,3-dihydroxyphenyl) methyl]-1-methylpiperidin-1-ium iodide (2.68 g) and water (60 mL) was dissolved while being heated, and sodium hydrogen carbonate (832 mg) was added to the mixture. A DMF (15 mL) solution of the above SNP-MEAA was added to the mixture, and the mixture was stirred for 42 hours at room temperature in an argon atmosphere. The reaction mixture was divided into 12 centrifuge tubes with use of water (3 mL), acetone (40 mL) was added to each of the 12 centrifuge tubes, and each of the 12 centrifuge tubes was centrifuged at 7000 rpm for 10 minutes at 10°C to remove a supernatant. A resultant precipitate was dispersed in water, and the mixture was centrifuged at 5800 rpm for 30 minutes at 10°C with use of an Amicon 30K filter. Water was added to the concentrated liquid on the Amicon 30K filter, and a resultant mixture was centrifuged at 5800 rpm for 30 minutes at 10°C. This operation was repeated one more time. Water was added to the concentrated liquid on the Amicon 30K filter, and a resultant mixture was centrifuged at 5800 rpm for 60 minutes at 10°C. This operation was repeated four more times. Resultant filtrates were sequentially put onto an Amicon 10K filter, and each of those was centrifuged at 5800 rpm at 10°C for 30 minutes in first two times and then for 60 minutes in subsequent six times. Water was further added to the concentrated liquid on the Amicon 10K filter, and a resultant mixture was centrifuged at 5800 rpm for 60 minutes at 10°C. This operation was repeated 14 more times. The concentrated liquid was filtered with a membrane (0.2 µm), and freeze-dried to obtain 10K purified particles (62.5 mg). Filtrates obtained with use of the Amicon 10K filter were sequentially put onto an Amicon 3K filter and each of those was centrifuged at 5800 rpm at 10°C for 30 minutes in the first time and then for 60 minutes in subsequent 13 times. Water was further added to the concentrated liquid on the Amicon 3K filter, and a resultant mixture was centrifuged at 5800 rpm for 60 minutes at 10°C. This operation was repeated seven more times. The concentrated liquid was filtered with a membrane (0.2 µm), and freeze-dried to obtain 3K purified particles (74.1 mg).

### Example 27

A mixture of SNP-OA (150 mg), MEAA (3.75 mL), and methanol (11.25 mL) was stirred for 6 hours at 70°C in an argon atmosphere. The mixture was cooled down to room temperature, and then was concentrated under reduced pressure. The mixture was divided into six centrifuge tubes, and acetone (4 mL) and hexane (16 mL) were added to each of the six centrifuge tubes, and each of the six centrifuge tubes was centrifuged at 7000 rpm for 10 minutes at 10°C to remove a supernatant. This operation was repeated one more time to obtain SNP-MEAA.

A mixture of 3-carboxy-N-[2-(2,3-dihydroxyphenyl)ethyl]-N,N-dimethylpropan-1-aminium iodide (2.69 g), water (25 mL), and sodium hydrogen carbonate (1.24 g) was stirred for 5 minutes at room temperature. A DMF (12.5 mL) solution of the above SNP-MEAA was added to the mixture, and the mixture was stirred for 41 hours at room temperature in an argon atmosphere. The reaction mixture was divided into six centrifuge tubes with use of water (3 mL), acetone (30 mL) was added to each of the six centrifuge tubes, and each of the six centrifuge tubes was centrifuged at 7000 rpm for 10 minutes at 10°C to remove a supernatant. A resultant precipitate was dispersed in water, and the mixture was centrifuged at 5800 rpm for 30 minutes at 10°C with use of an Amicon 30K filter. Water was added to this and a resultant mixture was centrifuged at 5800 rpm for 60 minutes at 10°C. This operation was carried out two more times. Filtrates obtained through the Amicon 30K filter were sequentially centrifuged at 5800 rpm for 60 minutes at 10°C with use of an Amicon 10K filter. Water was further added and a resultant mixture was centrifuged at 5800 rpm for 60 minutes at 10°C. This operation was carried out 10 more times. The concentrated liquid was filtered with a membrane (0.2 µm), and freeze-dried to obtain 10K purified particles (17.6 mg). Filtrates obtained through the Amicon 10K filter were sequentially centrifuged at 5800 rpm for 60 minutes at 10°C with use of an Amicon 3K filter. Water was further added and a resultant mixture was centrifuged at 5800 rpm for 60 minutes at 10°C. The concentrated liquid was filtered with a membrane (0.2 µm), and freeze-dried to obtain 3K purified particles (116 mg).

Structural formulae and physicochemical data of the compounds of Production Examples and the nanoparticles of Examples above are shown in tables below.

**[Table 1]**

| PEx | PSyn | Str | Data 1 |
|---|---|---|---|
| 1 | P1 | 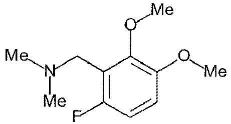 | ESI+:214 |
| 2 | P2 | 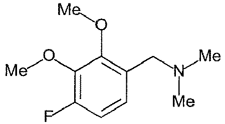 | ESI+: 214 |
| 3 | P3 | 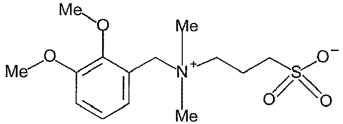 | ESI+:318 |
| 4 | P4 | 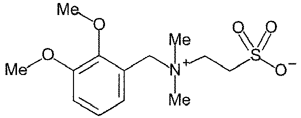 | ESI+:304 |
| 5 | P5 | 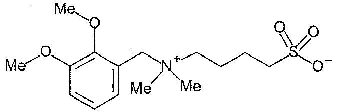 | ESI+:332 |
| 6 | P6 | 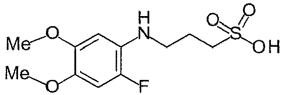 | ESI+:294 |
| 7 | P7 | 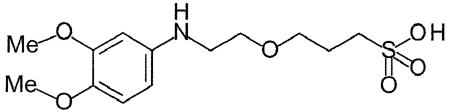 | ESI+:320 |
| 8 | P8 | 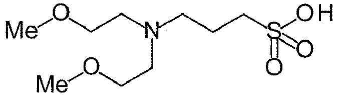 | ESI+:256 |

**[Table 2]**

| PEx | PSyn | Str | Data 1 |
|---|---|---|---|
| 9 | P9 | 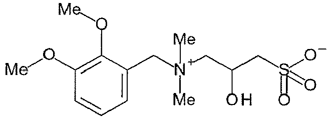 | ESI+: 334 |
| 10 | P10 | 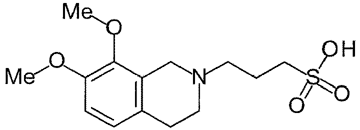 | ESI+:316 |
| 11 | P11 | 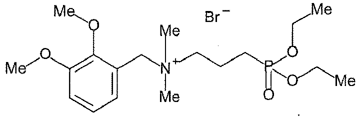 | ESI(M+)+:374 |
| 12 | P12 | 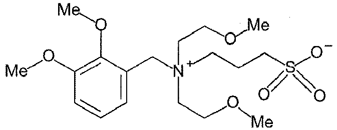 | ESI+:406 |
| 13 | P13 | 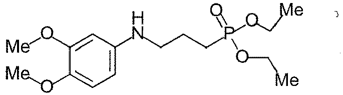 | ESI+:332 |
| 14 | P14 | 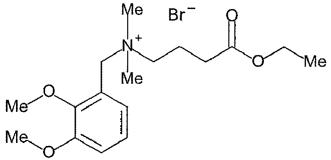 | ESI(M+)+:310 |
| 15 | P15 | 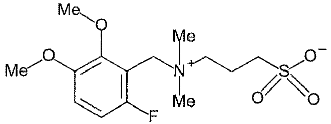 | ESI+:336 |

**[Table 3]**

| PEx | PSyn | Str | Data 1 |
|---|---|---|---|
| 16 | P16 | 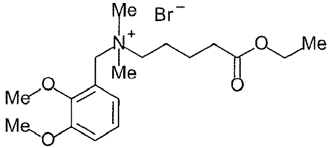 | APCI/ESI(M+)+:32 4 |
| 17 | P17 | 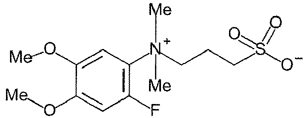 | ESI+:322 |
| 18 | P18 | 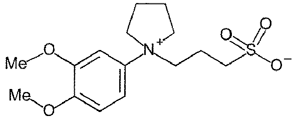 | ESI+: 330 |
| 19 | P19 | 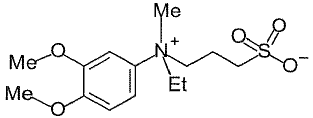 | ESI+:318 |
| 20 | P20 | 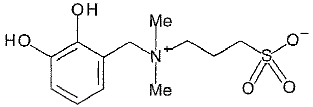 | ESI+:290 |
| | | | NMR-D: 2.04-2.15 (2H, m), 2.44-2.48 (2H, m), 2.92 (6H, s), 3.39-3.45 (2H, m), 4.40 (2H, s), 6.73 (1H, dd, J = 7.6, 7. 6Hz), 6.84 (1H, dd, J = 7.6, 1. 5Hz), 6.92 (1H, dd, J = 7.6, 1.5Hz), 9.22 (1H, br s), 9.77 (1H, br s) |

**[Table 4]**

| PEx | PSyn | Str | Data 1 |
|---|---|---|---|
| 21 | P21 | 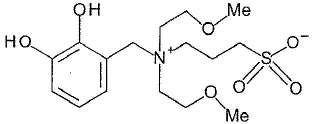 | ESI+:378 |
| 22 | P22 | 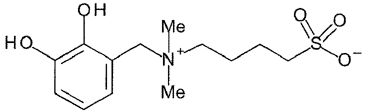 | ESI+:304 |
| | | | NMR-D: 1.56-1.65 (2H, m), 1. 83-1.91 (2H, m), 2.45-2.50 (2H, m), 2.91 (6H, s), 3.24-3.31 (2H, m), 4.39 (2H, s), 6.74 (1H, dd, J = 8.0, 8.0Hz), 6.83 (1H, dd, J = 8.0, 1.5Hz), 6.93 (1H, dd, J = 8.0, 1.5Hz), 9.25 (1H, br s), 9.80 (1H, br s) |
| 23 | P23 | 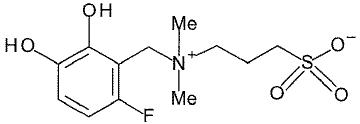 | ESI+:308 |
| | | | NMR-D: 2. 03-2. 13 (2H, m), 2.46 (2H, t, J = 7.1Hz), 2.96 (6H, s), 3.43-3.50 (2H, m), 4.43 (2H, s), 6.61-6.66 (1H, m), 6.92 (1H, dd, J = 8.8, 5.8Hz), 9.77 (1H, s), 9.83 (1H, s) |

**[Table 5]**

| PEx | PSyn | Str | Data 1 |
|---|---|---|---|
| 24 | P24 | 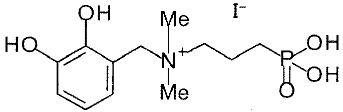 | ESI(M+)+:290 |
| | | | NMR-D: 1.51-1.60 (2H, m), 1.93-2.03 (2H, m), 2.94 (6H, s), 3.32-3.38 (2H, m), 4.42 (2H, s), 6.74 (1H, dd, J = 7.7, 7.7Hz), 6.84 (1H, dd, J = 7.7, 1.5Hz), 6.94 (1H, dd, J = 7.7, 1.5Hz), 9.21 (1H, br s), 9.83 (1H, br s) |
| 25 | P25 | 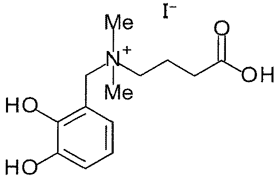 | ESI(M+)+:254 |
| | | | NMR-D: 1.97-2.04 (2H, m), 2.32 (2H, t, J = 7.2Hz), 2.95 (6H, s), 3.25-3.30 (2H, m), 4.42 (2H, s), 6.74 (1H, dd, J = 7.8, 7.8Hz), 6.85 (1H, dd, J = 1.3, 7.8Hz), 6.93 (1H, dd, J = 1.5, 7.8Hz), 9.25 (1H, s), 9.82 (1H, s), 12.33 (1H, s) |

**[Table 6]**

| PEx | PSyn | Str | Data 1 |
|---|---|---|---|
| 26 | P26 | 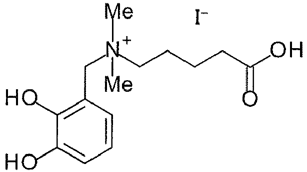 | ESI(M+)+:268 |
| | | | NMR-D: 1.51 (2H, quin, J = 7.5 Hz), 1. 75-1. 85 (2H, m), 2.30 (2H, t, J = 7.3 Hz), 2.93 (6H, s), 3.24-3.29 (2H, m), 4.40 (2H, s), 6.74 (1H, dd, J = 7.8, 7. 8Hz), 6.83 (1H, dd, J = 1.5, 7.8Hz), 6.93 (1H, dd, J = 1.5, 7.8Hz), 9.25 (1H, s), 9.83 (1H, s), 12. 13 (1H, s) |
| 27 | P27 | 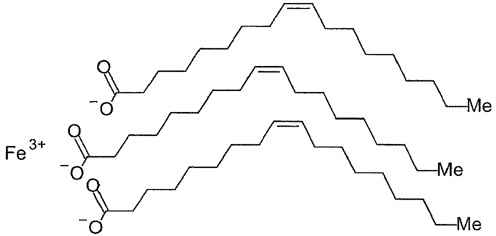 | ESI(M-)-:281 |
| 28 | P28 | 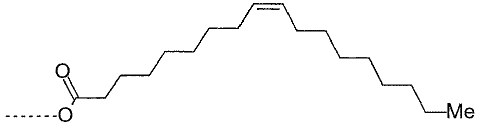 | |
| 29 | P1 | 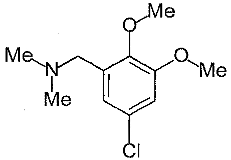 | ESI+:230 |

**[Table 7]**

| PEx | PSyn | Str | Data 1 |
|---|---|---|---|
| 30 | P3 | 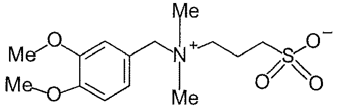 | ESI+:318 |
| 31 | P3 | 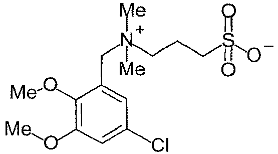 | ESI+:352 |
| 32 | P3 | 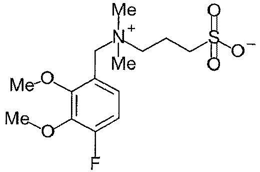 | ESI+:336 |
| 33 | P33 | 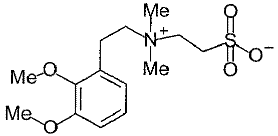 | ESI+: 318 |
| 34 | P5 | 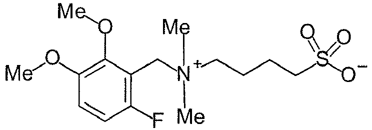 | ESI+:350 |
| 35 | P35 | 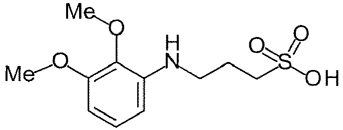 | ESI+:276 |
| 36 | P6 | 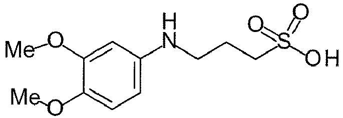 | ESI+:276 |

**[Table 8]**

| PEx | PSyn | Str | Data 1 |
|---|---|---|---|
| 37 | P6 | 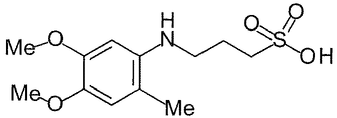 | ESI+:290 |
| 38 | P38 | 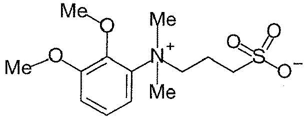 | ESI+:304 |
| 39 | P17 | 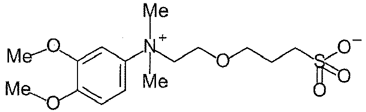 | ESI+:348 |
| 40 | P17 | 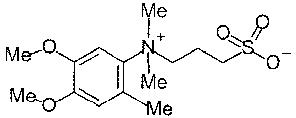 | ESI+:318 |
| 41 | P17 | 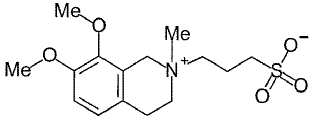 | ESI+: 330 |
| 42 | P17 | 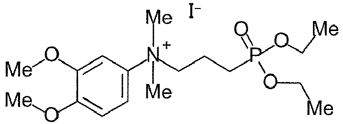 | ESI(M+)+:360 |
| 43 | P43 | 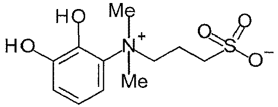 | ESI+:276 |
| | | | NMR-D: 1. 57-1. 63 (2H, m), 2.36 (2H, t, J = 7.5 Hz), 3.59 (6H, s), 4.06-4. 11 (2H, m), 6.84 (1H, dd, J = 8. 4, 8.4 Hz), 7.02-7.08 (2H, m), 10.21 (1H, s), 10.39 (1H, s) |

**[Table 9]**

| PEx | PSyn | Str | Data 1 |
|---|---|---|---|
| 44 | P20 | 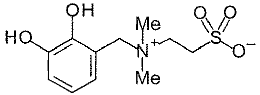 | ESI+: 276 |
| | | | NMR-D: 2.94 (6H, s), 3.01-3.07 (2H, m), 3.53-3.58 (2H, m), 4.44 (2H, s), 6.73 (1H, dd, J = 7.7, 7.7Hz), 6.84 (1H, dd, J = 7.7, 1.5Hz), 6.92 (1H, dd, J = 7.7, 1.5Hz), 9.21 (1H, br s), 9.77 (1H, br s) |
| 45 | P20 | 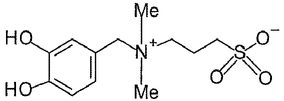 | ESI+:290 |
| 46 | P20 | 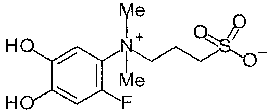 | ESI+:294 |
| 47 | P20 | 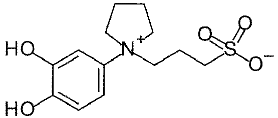 | ESI+: 302 |
| 48 | P20 | 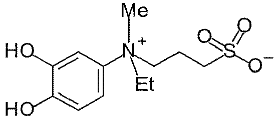 | ESI+:290 |

**[Table 10]**

| PEx | PSyn | Str | Data 1 |
|---|---|---|---|
| 49 | P20 | 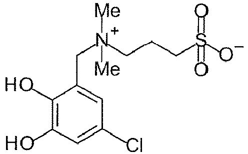 | ESI+: 324 |
| 50 | P50 | 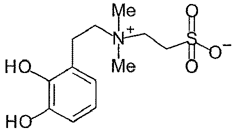 | ESI+:290 |
| | | | NMR-D: 2.90-2.96 (2H, m), 2.96-3.01 (2H, m), 3.10 (6H, s), 3.34-3.39 (2H, m), 3.59-3.65 (2H, m), 6.58-6.64 (2H, m), 6.70 (1H, dd, J = 2.2, 7.2 Hz), 8.57 (1H, br s), 9.33 (1H, br s) |

**[Table 11]**

| PEx | PSyn | Str | Data 1 |
|---|---|---|---|
| 51 | P20 | 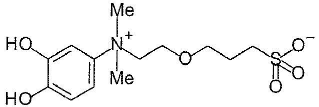 | ESI+:320 |
| 52 | P20 | 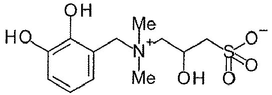 | ESI+: 306 |
| 53 | P20 | 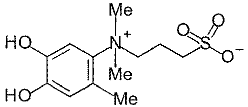 | ESI+:290 |
| 54 | P20 | 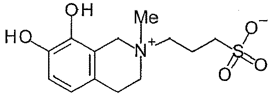 | ESI+: 302 |
| 55 | P20 | 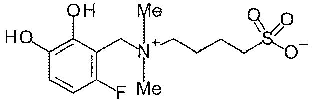 | ESI+: 322 |
| | | | NMR-D: 1.63 (2H, quin, J = 7.5 Hz), 1.82-1.90 (2H, m), 2.45-2.49 (2H, m), 2.94 (6H, s), 3.32-3.37 (2H, m), 4.43 (2H, s), 6.61-6.66 (1H, m), 6.93 (1H, dd, J = 8.8, 6.0Hz), 9.76-9.89 (2H, m) |
| 56 | P20 | 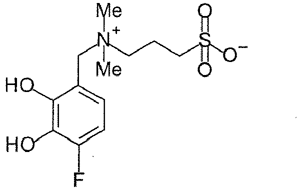 | ESI+:308 |
| 57 | P20 | 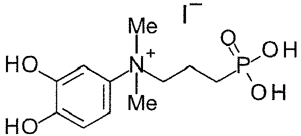 | ESI(M+)+:276 |

**[Table 12]**

| PEx | PSyn | Str | Data 1 |
|---|---|---|---|
| 58 | P58 | 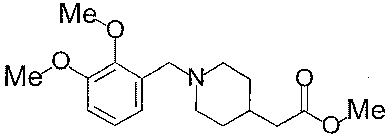 | ESI+:308 |
| 59 | P59 | 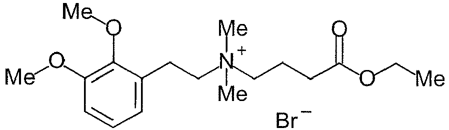 | ESI+:324.5(M+) |
| 60 | P60 | 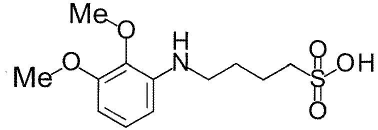 | ESI+:290 |
| 61 | P61 | 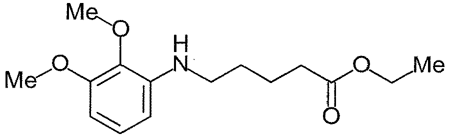 | ESI+:282 |
| 62 | P62 | 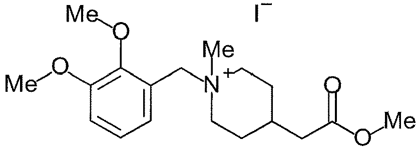 | ESI+:322 (M+) |
| 63 | P63 | 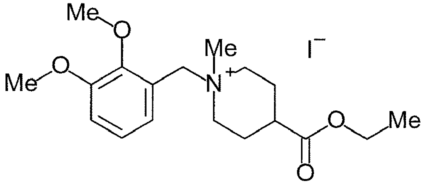 | ESI+:322 (M+) |

**[Table 13]**

| PEx | PSyn | Str | Data 1 |
|---|---|---|---|
| 64 | P64 | 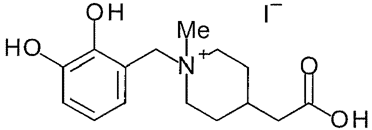 | ESI+:280 (M+) |
| | | | NMR-D: 1.60-1.72 (2H, m), 1.75-1.82 (2H, m), 1.83-1.97 (1H, m), 2.28, 2.36 (2H, d, J = 6.9 Hz), 2.83, 2.93 (3H, s), 3.26-3.48 (4H, m), 4.45, 4.50 (2H, s), 6.74 (1H, dd, J = 7.8, 7. 8 Hz), 6.84 (1H, dd, J = 1.4, 7.8 Hz), 6.93 (1H, dd, J = 1.4, 7.8 Hz), 9.22 (1H, s), 9.81 (1H, s), 12.19 (1H, br) |
| 65 | P65 | 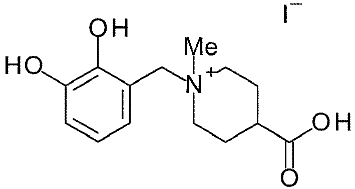 | ESI+:266 (M+) |
| | | | NMR-D: 1.90-2.05 (4H, m), 2.52-2.60 (1H, m), 2.93 (3H, s), 3.34-3.46 (4H, m), 4.46 (2H, s), 6.74 (1H, dd, J = 7.7, 7.7 Hz), 6.83 (1H, dd, J = 7. 7, 1. 5 Hz) 6.93 (1H, dd, J = 7. 7, 1. 5 Hz), 9.25 (1H, s), 9.83 (1H, s), 12.55 (1H, s) |

**[Table 14]**

| PEx | PSyn | Str | Data 1 |
|---|---|---|---|
| 66 | P66 | 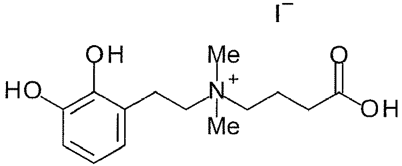 | ESI+:268 (M+) |
| | | | NMR-D: 1.89-1.97 (2H, m), 2.33 (2H, t, J = 7.0 Hz), 2.92-2.97 (2H, m), 3.09 (6H, s), 3.31-3.42 (4H, m), 6.60 (1H, dd, J = 7.7, 7.7 Hz), 6.64 (1H, dd, J = 1.8, 7.7 Hz), 6.71 (1H, dd, J = 1.8, 7.7 Hz), 8.59 (1H, s), 9.39 (1H, s), 12.36 (1H, br) |
| 67 | P17 | 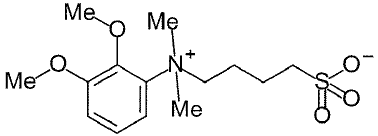 | ESI+: 318 |
| 68 | P20 | 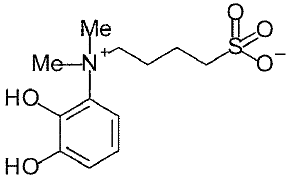 | ESI+: 290 |
| 69 | P17 | 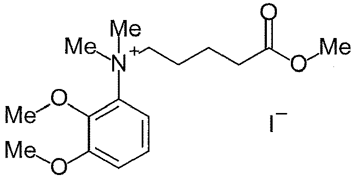 | ESI+:296 (M+) |

**[Table 15]**

| PEx | PSyn | Str | Data 1 |
|---|---|---|---|
| 70 | P25 | 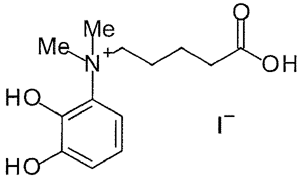 | ESI+:254 (M+) |
| 71 | P14 | 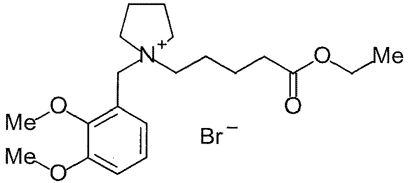 | ESI+:350 (M+) |
| 72 | P25 | 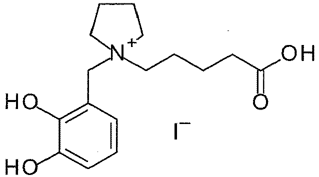 | ESI+:294 (M+) |
| 73 | P14 | 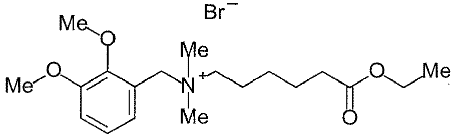 | ESI+:338 (M+) |
| 74 | P25 | 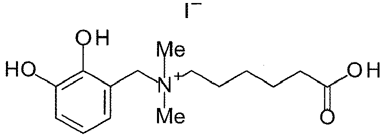 | ESI+:282 (M+) |

**[Table 16]**

| Ex | ESyn | Str | Data 2 |
|---|---|---|---|
| 1 | E1 | 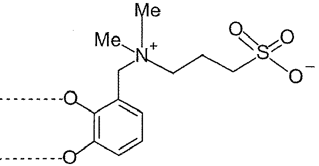 | |
| 2 | E2 | 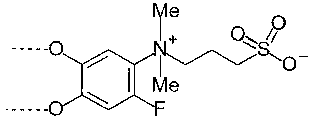 | |
| 3 | E3 | 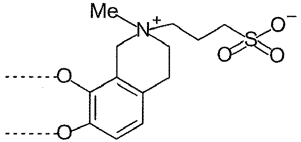 | |
| 4 | E4 | 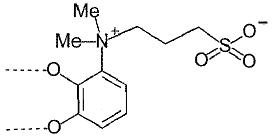 | SEC (min):11.7(3K) |
| | | | SEC(min):11.4(10K) |
| 5 | E5 | 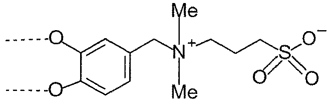 | |
| 6 | E6 | 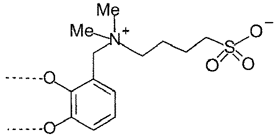 | SEC (min) : 10.8-11.4 (3K) |
| | | | SEC (min) :10.6-11.0 (10K) |
| 7 | E7 | 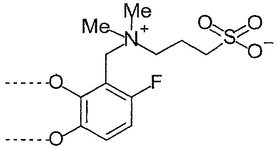 | SEC (min):11.3-11.4 (3K) |
| | | | SEC(min):10.5-10.8 (10K) |

**[Table 17]**

| Ex | ESyn | Str | Data 2 |
|---|---|---|---|
| 8 | E8 | 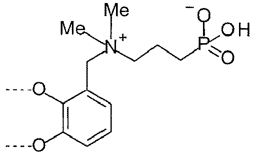 | |
| 9 | E9 | 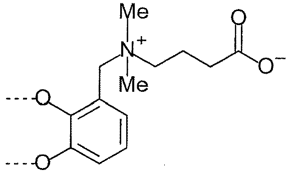 | |
| 10 | E10 | 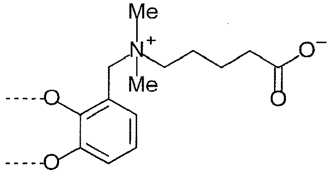 | |
| 11 | E11 | 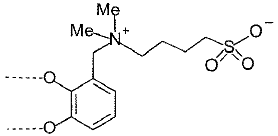 | SEC (min) : 11.2-11. 5 (3K) |
| | | | SEC (min) : 10.7-11.1 (10K) |
| 12 | E12 | 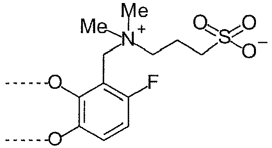 | SEC (min) : 11.2-11.4 (3K) |
| | | | SEC (min):10.8-10.9 (10K) |
| 13 | E13 | 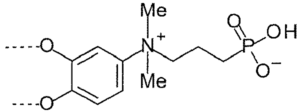 | |
| 14 | E1 | 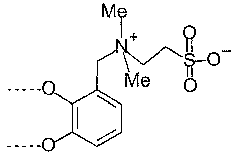 | |

**[Table 18]**

| Ex | ESyn | Str | Data 2 |
|---|---|---|---|
| 15 | E2 | 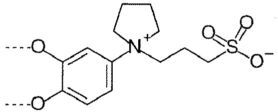 | |
| 16 | E2 | 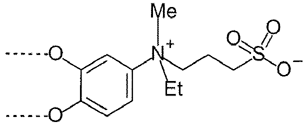 | |
| 17 | E2 | 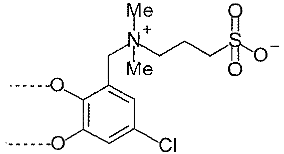 | |
| 18 | E18 | 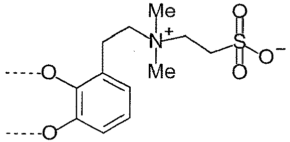 | SEC(min) : 11.0-11.5 (3K) |
| | | | SEC (min) : 10.9 (10K) |
| 19 | E2 | 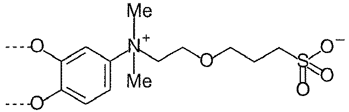 | |
| 20 | E2 | 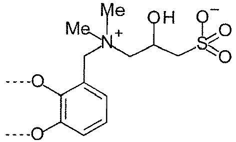 | |
| 21 | E2 | 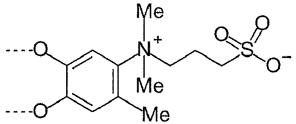 | |

**[Table 19]**

| Ex | ESyn | Str | Data 2 |
|---|---|---|---|
| 22 | E1 | 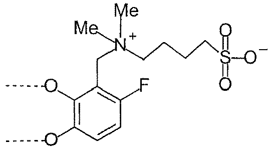 | |
| 23 | E1 | 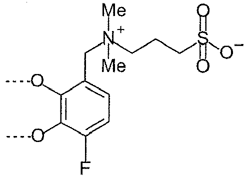 | |
| 24 | E1 | 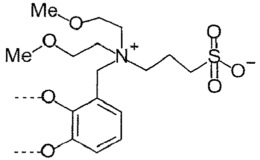 | |
| 25 | E25 | 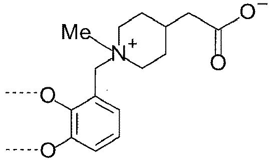 | SEC (min):10.8-11.0 (3K) |
| | | | SEC (min) : 10. 4-10.8 (10K) |
| 26 | E26 | 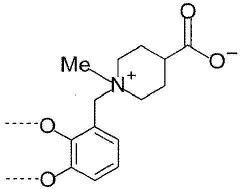 | SEC (min):10.9-11.0 (3K) |
| | | | SEC (min) : 10.3-10.7 (10K) |
| 27 | E27 | 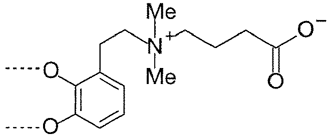 | SEC (min) : 10.8(3K) |
| | | | SEC (min):10.5(10K) |

**[Table 20]**

| Ex | ESyn | Str | Data 2 |
|---|---|---|---|
| 28 | E1 | 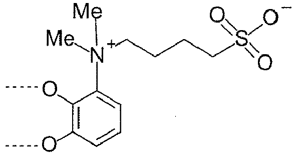 | SEC(min):11.0(3K) |
| | | | SEC(min):10.7(10K) |
| 29 | E1 | 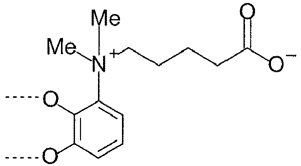 | SEC(min):11.0(3K) |
| | | | SEC (min):10.7(10K) |
| 30 | E1 | 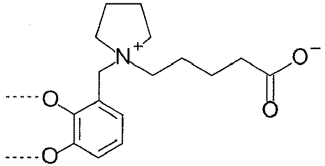 | SEC (min):10.6(3K) |
| | | | SEC (min):10.2(10K) |
| 31 | E1 | 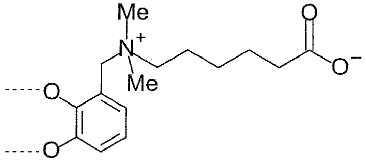 | SEC (min):10.5(3K) |
| | | | SEC (min) : 10.1(10K) |

Then, the nanoparticles of the formula (I) obtained in Examples above were evaluated as follows.

### [Test Example 1. Evaluative measurement of MR relaxivity of nanoparticle]

A relaxivity of 3K purified particles obtained in each Example was evaluated.

First, the concentration of nanoparticles was serially diluted in PBS to prepare test samples. For each sample, a relaxivity was measured by 1.5T-NMR.

T₁ and T₂ were measured under the following conditions.
Measurement magnetic field: 1.5 T; Measurement temperature: 37°C;

### T₁ measurement (inversion recovery)

Recycle Delay (RD): Set to be 5 times or more of T₁ for each sample and for each concentration. The number of obtained data points was 8 or more, an initial time of inversion pulse (inversion time) was fixed to 5 ms, and a last inversion time was set to be identical with RD.

### T₂ measurement (Carr-Purcell-Meiboom-Gill (CPMG))

Recycle Delay (RD): Set to be identical with RD of T₁. τ = 0.5 ms, and the number of obtained data points was set such that the number of τ × 2 × data points became substantially identical with RD.

r₁ and r₂ of each sample were obtained by respectively measuring T₁ and T₂ at different concentrations, and calculating inclinations with the SLOPE function, where X-axis indicates concentration and Y-axis indicates reciprocals of T₁ and T₂.

The table below shows the results. Note that "NT" in the table is an abbreviation for "Not Tested".

**[Table 21]**

| Ex | r₁ /r₂ | Ex | r₁/ r₂ | Ex | r ₁/ r ₂ |
|---|---|---|---|---|---|
| 1 | 0.85 | 13 | 0.89 | 25 | 0.96 - 0.98^{∗} |
| 2 | 0.84 | 14 | 0.92 | 26 | 0.95 - 0.99^{∗} |
| 3 | 0.85 | 15 | NT | 27 | 0.93 |
| 4 | 0.85 | 16 | NT | 28 | 0.93 |
| 5 | 0.87 | 17 | NT | 29 | 0.97 |
| 6 | 0.86 - 0.93^{∗} | 18 | 0.72 - 0.94^{∗} | 30 | 0.98 |
| 7 | 0.88 - 0.90^{∗} | 19 | NT | 31 | 0.97 |
| 8 | 0.91 | 20 | 0.74 | | |
| 9 | 0.93 | 21 | 0.83 | | |
| 10 | 0.93 | 22 | 0.91 | | |
| 11 | 0.90 - 0.95^{∗} | 23 | 0.91 | | |
| 12 | 0.87 - 0.92^{∗} | 24 | NT | | |

The symbol "*" represents that the value is indicated as a range of obtained values because the relaxivity was measured for the nanoparticles which were repeatedly produced a plurality of times with a method similar to that Example.

The r_{1/}r₂ values of the 3K purified particles were 0.86 to 0.93 and 0.90 to 0.95, respectively, in Example 6 and Example 11 which employed the identical zwitterionic ligands that were coordinately bound and employed different methods for producing nanoparticles. The r_{1/}r₂ values of the 3K purified particles were 0.88 to 0.90 and 0.87 to 0.92, respectively, in Example 7 and Example 12 which employed the identical zwitterionic ligands that were coordinately bound and employed different methods for producing nanoparticles. From this result, it was confirmed that the nanoparticles having substantially equivalent good relaxivities can be obtained by any of those production methods.

The 3K purified particles which contained the same zwitterionic ligands and were obtained by a plurality of productions in Example 6 and Example 11 above had relaxivity values r₁ between 2.74 and 3.76, and relaxivity values r₂ between 3.06 and 4.18.

Similarly, the 3K purified particles which contained the same zwitterionic ligands and were obtained by a plurality of productions in Example 7 and Example 12 above had relaxivity values r₁ between 3.02 and 3.85, and relaxivity values r₂ between 3.27 and 4.17.

Moreover, the 10K purified particles which contained the same zwitterionic ligands and were obtained by a plurality of productions in Example 6 and Example 11 had relaxivity values r₁ between 3.19 and 4.15, relaxivity values r₂ between 3.43 and 4.41, and r_{1/}r₂ values between 0.86-0.94.

Similarly, the 10K purified particles which contained the same zwitterionic ligands and were obtained by a plurality of productions in Example 7 and Example 12 had relaxivity values r₁ between 3.38 and 4.84, relaxivity values r₂ between 3.77 and 6.14, and r_{1/}r₂ values between 0.71-0.94.

Moreover, as a result of evaluating a relaxivity of 10K purified particles of Example 18, a relaxivity value r₁ was 2.52, a relaxivity value r₂ was 3.02, and a value of r_{1/}r₂ was 0.83.

Moreover, as a result of evaluating a relaxivity of 10K purified particles of Example 25, relaxivity values r₁ were between 3.72 and 4.04, relaxivity values r₂ were between 4.3 and 4.48, and r_{1/}r₂ values were between 0.83-0.94.

As a result of evaluating a relaxivity of 3K purified particles of Example 18, relaxivity values r₁ were between 2.93 and 2.94, and relaxivity values r₂ were between 3.13 and 4.09.

As a result of evaluating a relaxivity of 3K purified particles of Example 25, relaxivity values r₁ were between 3.18 and 3.43, and relaxivity values r₂ were between 3.30 and 3.52.

Those values are the highest among values obtained with conventionally reported SNPs including an iron oxide particle as a core, after correction of magnetic field strength. This indicates that the nanoparticles are promising nanoparticles to be used as a positive contrast agent.

### [Test Example 2. Evaluative test of particle diameter of nanoparticle]

A relative size of nanoparticle was measured with size exclusion chromatography (SEC).

SEC is an analysis technique in which (i) a sample is caused to flow through a column filled with a carrier having pores and (ii) a size of the sample is estimated on the basis of a time taken for the sample to be discharged from the column. Large aggregates do not enter the pores of the carrier, and therefore are quickly discharged from the column. Small nanoparticles pass through the pores of the carrier, and therefore are slowly discharged from the column due to following of a longer route before being discharged from the column. It is thus possible to measure a relative size by use of standard particles.

The 3K purified nanoparticles and 10K purified nanoparticles produced by the MEAA method of Example 6, the 3K purified nanoparticles and 10K purified nanoparticles of Example 11 which were produced by the phase transfer catalyst method with use of the same zwitterionic ligand as Example 6, the 3K purified nanoparticles and 10K purified nanoparticles produced by the MEAA method of Example 7, and the 3K purified nanoparticles and 10K purified nanoparticles of Example 12 which were produced by the phase transfer catalyst method with use of the same zwitterionic ligand as Example 7 were subjected to measurement under the following SEC conditions. The measurement was carried out twice. Similarly, the 3K purified nanoparticles and 10K purified nanoparticles produced by the MEAA method of Examples 18, 25, and 26 were subjected to measurement under the following SEC conditions. The measurement was carried out twice.

### <SEC conditions>

Flow rate: 0.3 mL/min
Eluent: PBS (pH 7.4)
Column: Shodex KW403-4F (4.6 × 300 mm)
Detector: UV 280 nm

The table below shows the results. Note that the flow-out time of ovalbumin, which is an authentic sample, is 9.4 to 10.2 minutes.

**[Table 22]**

| Ex | Particles | SEC flow-out time of nanoparticles (min.) | Ratio to authentic sample |
|---|---|---|---|
| 6 | 3K purified nanoparticles | 10.8~11. 4 | 1.11~1. 14 |
| | 10K purified nanoparticles | 10. 6~11. 0 | 1. 07~1. 10 |
| 7 | 3K purified nanoparticles | 11. 3~11. 4 | 1. 15~1. 16 |
| | 10K purified nanoparticles | 10.5~10.8 | 1. 07~1. 10 |
| 11 | 3K purified nanoparticles | 11. 2~11. 5 | 1. 12~1. 14 |
| | 10K purified nanoparticles | 10. 7~11. 1 | 1. 06~1. 09 |
| 12 | 3K purified nanoparticles | 11.2~11.4 | 1.12~1. 14 |
| | 10K purified nanoparticles | 10. 8~10. 9 | 1. 08~1. 10 |
| 18 | 3K purified nanoparticles | 11. 0~11. 5 | 1. 13~1. 17 |
| | 10K purified nanoparticles | 10.9 | 1.11 |
| 25 | 3K purified nanoparticles | 10.8~11.0 | 1. 12~1. 14 |
| | 10K purified nanoparticles | 10.4~10.8 | 1. 08~1. 12 |
| 26 | 3K purified nanoparticles | 10. 9~11. 0 | 1. 12~1. 14 |
| | 10K purified nanoparticles | 10. 3~10. 7 | 1. 06~1. 11 |

From the above result, it was confirmed, from the flow-out times of SEC, that the nanoparticles could be obtained which had substantially equivalent particle diameters even with different production methods. From the flow-out time and the ratio to ovalbumin (particle size: 6.1 nm), which is the authentic sample, it was confirmed that the obtained nanoparticles had relatively smaller particle diameters.

### [Test Example 3. Stability evaluation test]

In order for a contrast agent containing nanoparticles to exhibit an expected performance, it is necessary that the nanoparticles be stably dispersed in a solution. It is also desirable that dispersion of the nanoparticles is maintained for a long period of time even in a state where the nanoparticles are contained at a high concentration.

In general, a dispersion stability of nanoparticles can be evaluated by use of size exclusion chromatography (SEC).

In order to confirm the stability of the nanoparticles, nanoparticles obtained in Examples above were freeze-dried and then were dispersed in PBS so as to achieve an Fe ion concentration of approximately 100 mM. A solution thus obtained was used as a test sample. The test samples were left to stand still at -20°C, at 4°C and at room temperature (20°C), respectively. 2 weeks, 1 month, and 3 months later, each of the test samples was subjected to SEC to check a degree of agglomeration. The measurement conditions of SEC were similar to those described in Test Example 2.

### [Test Example 4. MRI contrast imaging using mouse]

i) Contrast agents containing the nanoparticles obtained in Examples were each administered to a mouse, and T₁-weighted images were obtained with use of a 1T MRI apparatus. Measurement conditions were as follows.
   Animal: C57BL/6j jms mouse, male, having a body weight of approximately 25 g
   Concentration of administered nanoparticles: 20 mM
   Administration amount: 100 µL per body weight of 20 g
   Magnetic field strength: 1 T
   Imaging method: T₁-weighted (Figs. 1 through 6), Used apparatus: ICON available from Bruker

### <ICON available from Bruker>

### T₁-weighted image

Pulse sequence: MSME (Multi Slice Multi Echo), Slice Orientation = Axial, TE/TR = 10.464 / 400 msec, Field of view = 40 × 40 mm², matrix size = 256 × 256, Number of Slice = 15, Slice thickness = 1 mm, Slice Gap = 2 mm, Number of averages = 8, Scan Time = 13 min 39 sec

Imaging was carried out before the administration of the contrast agent (pre), and then 20 mM solution of the contrast agent containing nanoparticles was intravenously administered by 100 µL per mouse body weight of 20 g. Imaging was carried out at different elapsed time points to conduct follow-up observation up to 1.5 hours after the administration.

Results are shown in Figs. 1 through 6.

In the mouse to which the contrast agent containing the 3K purified particles of Example 6 in Fig. 1 was administered, increase in signals from both the renal pelvis and the renal cortex and accumulation of urine containing the contrast agent were observed immediately after the administration. Those facts suggested that the contrast agent was excreted as urine via the kidney. Further, observation of these changes in signals suggested that the contrast agent can be potentially used in a renal function test.

In the mouse to which the contrast agent containing the 10K purified particles of Example 6 in Fig. 2 was administered, increase in signals from both the renal pelvis and the renal cortex and accumulation of urine containing the contrast agent were observed immediately after the administration. Those facts suggested that the contrast agent was excreted as urine via the kidney. Further, observation of these changes in signals suggested that the contrast agent can be potentially used in a renal function test.

In the mouse to which the contrast agent containing the 3K purified particles of Example 7 in Fig. 3 was administered, increase in signals from both the renal pelvis and the renal cortex and accumulation of urine containing the contrast agent were observed immediately after the administration. Those facts suggested that the contrast agent was excreted as urine via the kidney. Further, observation of these changes in signals suggested that the contrast agent can be potentially used in a renal function test.

In the mouse to which the contrast agent containing the 10K purified particles of Example 7 in Fig. 4 was administered, increase in signals from both the renal pelvis and the renal cortex and accumulation of urine containing the contrast agent were observed immediately after the administration. Those facts suggested that the contrast agent was excreted as urine via the kidney. Further, observation of these changes in signals suggested that the contrast agent can be potentially used in a renal function test.

In the mouse to which the contrast agent containing the 3K purified particles of Example 25 in Fig. 5 was administered, increase in signals from both the renal pelvis and the renal cortex and accumulation of urine containing the contrast agent were observed immediately after the administration. Those facts suggested that the contrast agent was excreted as urine via the kidney. Further, observation of these changes in signals suggested that the contrast agent can be potentially used in a renal function test.

In the mouse to which the contrast agent containing the 10K purified particles of Example 25 in Fig. 6 was administered, increase in signals from both the renal pelvis and the renal cortex and accumulation of urine containing the contrast agent were observed immediately after the administration. Those facts suggested that the contrast agent was excreted as urine via the kidney. Further, observation of these changes in signals suggested that the contrast agent can be potentially used in a renal function test.

### [Test Example 5. Measurement of magnetic field dependence of magnetization (M-H curve)]

The 3K purified particles obtained in Examples 6, 7 or 9 were put into the SQUID, the applied magnetic field was changed to 3T, -3T, and 3T in this order at intervals of 1000 to 5000 Oe at a temperature of 300K, and magnetization of particles at each point was measured.

The result of measurement is shown in Fig. 7. The result showed the following: the magnetic susceptibility is substantially in proportion to the magnetic field. The property as a super paramagnetic substance seems to be low, and the contrast agent, even in the form of nanoparticles, has the paramagnetic property, and is expected to have an excellent T₁-shortening effect in the practical magnetic field region.

### Industrial Applicability

The contrast agent for MRI of the present invention can be suitably used as a contrast agent for MRI in a medical field. The nanoparticle and the zwitterionic ligand compound of the present invention are applicable to various pharmaceutical compositions and the like, including a contrast agent for MRI, and can be used widely in the fields of pharmaceuticals, biotechnology, and the like, including various diagnosis methods and examination reagents.

## Claims

1. A nanoparticle comprising: at least one zwitterionic ligand represented by a formula (I); and a metal particle containing iron oxide, the at least one zwitterionic ligand being coordinately bound to the metal particle: 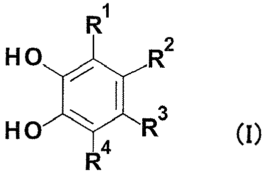 where
one of R¹ and R² is a group represented by a formula (a) or a formula (b), and the other of R¹ and R² is H, lower alkyl, -O- lower alkyl, or halogen, 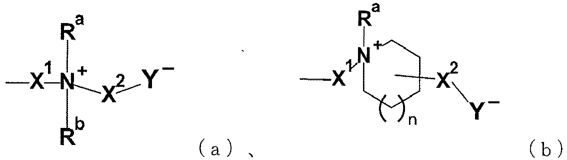
X¹ is a bond or methylene, or X¹ is optionally ethylene when R¹ is a group represented by the formula (a),
X² is C₁₋₅ alkylene that is optionally substituted with OH or is -C₁₋₂ alkylene-O-C₁₋₃ alkylene-, or X² is optionally a bond when R¹ is a group represented by the formula (b),
R^{a} and R^{b} are the same as or different from each other and represent C₁₋₃ alkyl or -C₁₋₃ alkylene-O-C₁₋₂ alkyl, or R^{a} and R^{b} form a 5- or 6-membered nitrogen-containing saturated heterocycle together with a quaternary nitrogen atom to which R^{a} and R^{b} are bound,
Y⁻ is SO₃⁻, HPO₃⁻, or CO₂⁻,
R³ and R⁴ are the same as or different from each other and represent H, C₁₋₃ alkyl, -O-C₁₋₃ alkyl, or halogen,
n is an integer of 0 to 2,
and,
i) when R¹ is a group represented by the formula (a) and X¹ is methylene, R² optionally forms ethylene together with R^{a} or R^{b},
ii) when R¹ is a group represented by the formula (a) and X¹ is ethylene, R² optionally forms methylene together with R^{a} or R^{b}, and
iii) when R² is a group represented by the formula (a) and X¹ is methylene, R³ optionally forms ethylene together with R^{a} or R^{b},
provided that, when R² is a group represented by the formula (a), R^{a} and R^{b} are methyl, X¹ is a bond, X² is C₁₋₄ alkylene, and R¹, R³ and R⁴ are H, Y⁻ is HPO₃⁻ or CO₂⁻.

2. The nanoparticle as set forth in claim 1, wherein:
in the at least one zwitterionic ligand,
one of R¹ and R² is a group represented by the formula (a) or the formula (b), and the other of R¹ and R² is H, lower alkyl, or halogen,
X¹ is a bond or methylene, or X¹ is optionally ethylene when R¹ is a group represented by the formula (a),
X² is C₁₋₅ alkylene that is optionally substituted with OH or is -C₁₋₂ alkylene-O-C₁₋₃ alkylene-, or X² is optionally a bond when R¹ is a group represented by the formula (b),
R^{a} and R^{b} are the same as or different from each other and represent C₁₋₃ alkyl or -C₁₋₃ alkylene-O-C₁₋₂ alkyl, or R^{a} and R^{b} form a pyrrolidine ring together with a quaternary nitrogen atom to which R^{a} and R^{b} are bound,
Y⁻ is SO₃⁻, HPO₃⁻, or CO₂⁻,
R³ and R⁴ are the same as or different from each other and represent H, C₁₋₃ alkyl, or halogen,
n is 1,
and,
i) when R¹ is a group represented by the formula (a) and X¹ is methylene, R² optionally forms ethylene together with R^{a} or R^{b}.

3. The nanoparticle as set forth in claim 2, wherein:
in the at least one zwitterionic ligand,
R¹ is a group represented by the formula (a) or the formula (b), and R² is H or halogen,
X¹ is a bond or methylene, or X¹ is optionally ethylene when R¹ is a group represented by the formula (a),
X² is C₁₋₅ alkylene, or X² is optionally a bond when R¹ is a group represented by the formula (b),
R^{a} and R^{b} are methyl, and
Y⁻ is SO₃⁻ or CO₂⁻.

4. The nanoparticle as set forth in claim 1, wherein:
in the at least one zwitterionic ligand,
one of R¹ and R² is a group represented by the formula (a), and the other of R¹ and R² is H, lower alkyl, - O-lower alkyl, or halogen.

5. The nanoparticle as set forth in claim 4, wherein:
in the at least one zwitterionic ligand,
1) R¹ is a group represented by the formula (a), and R² is H, lower alkyl, -O-lower alkyl, or halogen, or
2) R¹ is H, R² is a group represented by the formula (a), R³ is C₁₋₃ alkyl or halogen, and R⁴ is H.

6. The nanoparticle as set forth in claim 5, wherein:
in the at least one zwitterionic ligand, R¹ is a group represented by the formula (a), and R² is H, lower alkyl, - O-lower alkyl, or halogen.

7. The nanoparticle as set forth in claim 6, wherein:
in the at least one zwitterionic ligand,
R² is H or halogen,
X¹ is a bond, methylene, or ethylene,
X² is C₂₋₄ alkylene,
R^{a} and R^{b} are methyl,
R³ and R⁴ are the same as or different from each other and represent H, C₁₋₃ alkyl, or halogen,
and, when X¹ is methylene, R² optionally forms ethylene together with R^{a} or R^{b}.

8. The nanoparticle as set forth in claim 7, wherein:
in the at least one zwitterionic ligand,
R² is H or F,
X² is ethylene or propylene, and
R³ and R⁴ are H.

9. The nanoparticle as set forth in claim 8, wherein:
in the at least one zwitterionic ligand,
R² is H, and
X¹ is a bond or ethylene.

10. The nanoparticle as set forth in any one of claims 4 through 9, wherein:
in the at least one zwitterionic ligand,
Y⁻ is SO₃⁻ or CO₂⁻.

11. The nanoparticle as set forth in claim 3, wherein:
in the at least one zwitterionic ligand,
R¹ is a group represented by the following formula (b-1), 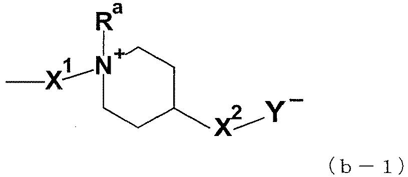
R² is H or halogen,
X¹ is a bond or methylene,
X² is C₁₋₅ alkylene or a bond,
R^{a} is methyl, and
Y⁻ is SO₃⁻ or CO₂⁻.

12. The nanoparticle as set forth in any one of claims 1 through 11, wherein the metal particle contains only iron oxide.

13. The nanoparticle as set forth in any one of claims 1 through 12, wherein: the at least one zwitterionic ligand is coordinately bound to an outer surface of the metal particle containing iron oxide; and the metal particle is coated with the at least one zwitterionic ligand.

14. The nanoparticle as set forth in any one of claims 1 through 12, wherein said nanoparticle is a composite comprising the at least one zwitterionic ligand and the metal particle containing iron oxide, the at least one zwitterionic ligand being coordinately bound to the metal particle.

15. The nanoparticle as set forth in any one of claims 1 through 12, wherein said nanoparticle is a cluster comprising two or more zwitterionic ligand compounds and two or more metal particles, each of the two or more metal particles containing iron oxide, and at least one zwitterionic ligand compound being coordinately bound to each of the two or more metal particles.

16. A contrast agent for magnetic resonance imaging comprising a nanoparticle recited in any one of claims 1 through 15.

17. The contrast agent as set forth in claim 16, wherein said contrast agent is a positive contrast agent.

18. Use of a zwitterionic ligand compound represented by the following formula (I) for producing a nanoparticle recited in claim 1: 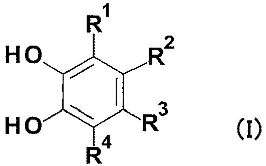 where
one of R¹ and R² is a group represented by a formula (a) or a formula (b) below, and the other of R¹ and R² is H, lower alkyl, -O- lower alkyl, or halogen, 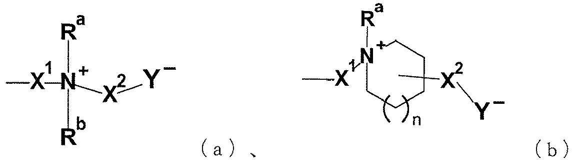
X¹ is a bond or methylene, or X¹ is optionally ethylene when R¹ is a group represented by the formula (a),
X² is C₁₋₅ alkylene that is optionally substituted with OH or is -C₁₋₂ alkylene-O-C₁₋₃ alkylene-, or X² is optionally a bond when R¹ is a group represented by the formula (b),
R^{a} and R^{b} are the same as or different from each other and represent C₁₋₃ alkyl or -C₁₋₃ alkylene-O-C₁₋₂ alkyl, or R^{a} and R^{b} form a 5- or 6-membered nitrogen-containing saturated heterocycle together with a quaternary nitrogen atom to which R^{a} and R^{b} are bound,
Y⁻ is SO₃⁻, HPO₃⁻, or CO₂⁻,
R³ and R⁴ are the same as or different from each other and represent H, C₁₋₃ alkyl, -O-C₁₋₃ alkyl, or halogen,
n is an integer of 0 to 2,
and,
i) when R¹ is a group represented by the formula (a) and X¹ is methylene, R² optionally forms ethylene together with R^{a} or R^{b},
ii) when R¹ is a group represented by the formula (a) and X¹ is ethylene, R² optionally forms methylene together with R^{a} or R^{b}, and
iii) when R² is a group represented by the formula (a) and X¹ is methylene, R³ optionally forms ethylene together with R^{a} or R^{b},
provided that, when R² is a group represented by the formula (a), R^{a} and R^{b} are methyl, X¹ is a bond, X² is C₁₋₄ alkylene, and R¹, R³ and R⁴ are H, Y⁻ is HPO₃⁻ or CO₂⁻.

19. The use as set forth in claim 18, wherein, in the zwitterionic ligand compound, one of R¹ and R² is a group represented by the formula (a), and the other of R¹ and R² is H, lower alkyl, -O-lower alkyl, or halogen.

20. A compound represented by the following formula (I) or a salt thereof: 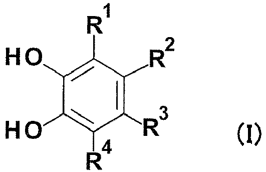 where
one of R¹ and R² is a group represented by a formula (a) or a formula (b) below, and the other of R¹ and R² is H, lower alkyl, -O- lower alkyl, or halogen, 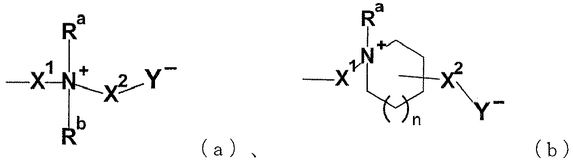
X¹ is a bond or methylene, or X¹ is optionally ethylene when R¹ is a group represented by the formula (a),
X² is C₁₋₅ alkylene that is optionally substituted with OH or is -C₁₋₂ alkylene-O-C₁₋₃ alkylene-, or X² is optionally a bond when R¹ is a group represented by the formula (b),
R^{a} and R^{b} are the same as or different from each other and represent C₁₋₃ alkyl or -C₁₋₃ alkylene-O-C₁₋₂ alkyl, or R^{a} and R^{b} form a 5- or 6-membered nitrogen-containing saturated heterocycle together with a quaternary nitrogen atom to which R^{a} and R^{b} are bound,
Y⁻ is SO₃⁻, HPO₃⁻, or CO₂⁻,
R³ and R⁴ are the same as or different from each other and represent H, C₁₋₃ alkyl, -O-C₁₋₃ alkyl, or halogen,
n is an integer of 0 to 2,
and,
i) when R¹ is a group represented by the formula (a) and X¹ is methylene, R² optionally forms ethylene together with R^{a} or R^{b},
ii) when R¹ is a group represented by the formula (a) and X¹ is ethylene, R² optionally forms methylene together with R^{a} or R^{b}, and
iii) when R² is a group represented by the formula (a) and X¹ is methylene, R³ optionally forms ethylene together with R^{a} or R^{b},
provided that, when R² is a group represented by the formula (a), R^{a} and R^{b} are methyl, X¹ is a bond, X² is C₁₋₄ alkylene, and R¹, R³ and R⁴ are H, Y⁻ is HPO₃⁻ or CO₂⁻.

21. The compound as set forth in claim 20 or a salt thereof, wherein:
one of R¹ and R² is a group represented by the formula (a) or the formula (b), and the other of R¹ and R² is H, lower alkyl, or halogen,
X¹ is a bond or methylene, or X¹ is optionally ethylene when R¹ is a group represented by the formula (a),
X² is C₁₋₅ alkylene that is optionally substituted with OH or is -C₁₋₂ alkylene-O-C₁₋₃ alkylene-, or X² is optionally a bond when R¹ is a group represented by the formula (b),
R^{a} and R^{b} are the same as or different from each other and represent C₁₋₃ alkyl or -C₁₋₃ alkylene-O-C₁₋₂ alkyl, or R^{a} and R^{b} form a pyrrolidine ring together with a quaternary nitrogen atom to which R^{a} and R^{b} are bound,
Y⁻ is SO₃⁻, HPO₃⁻, or CO₂⁻,
R³ and R⁴ are the same as or different from each other and represent H, C₁₋₃ alkyl, or halogen,
n is 1,
and, i) when R¹ is a group represented by the formula (a) and X¹ is methylene, R² optionally forms ethylene together with R^{a} or R^{b}.

22. The compound as set forth in claim 21 or a salt thereof, wherein:
R¹ is a group represented by the formula (a) or the formula (b), and R² is H or halogen,
X¹ is a bond or methylene, or X¹ is optionally ethylene when R¹ is a group represented by the formula (a),
X² is C₁₋₅ alkylene, or X² is optionally a bond when R¹ is a group represented by the formula (b),
R^{a} and R^{b} are methyl, and
Y⁻ is SO₃⁻ or CO₂⁻.

23. The compound as set forth in claim 20 or a salt thereof, wherein: one of R¹ and R² is a group represented by the formula (a), and the other of R¹ and R² is H, lower alkyl, -O-lower alkyl, or halogen.

24. The compound as set forth in claim 20 or a salt thereof, which is selected from the group consisting of:
4-{[(2,3-dihydroxyphenyl)methyl](dimethyl)azaniumyl}butane-1-sulfonate,
3-{[(6-fluoro-2,3-dihydroxyphenyl)methyl](dimethyl)azaniumyl}propane-1-. sulfonate,
Hydrogen (3-{[(2,3-dihydroxyphenyl)methyl](dimethyl)azaniumyl}propyl)phosph onate,
5-{[(2,3-dihydroxyphenyl)methyl](dimethyl)azaniumyl}pentanoate,
{1-[(2,3-dihydroxyphenyl)methyl]-1-methylpiperidin-1-ium-4-yllacetate,
1-[(2,3-dihydroxyphenyl)methyl]-1-methylpiperidin-1-ium-4-carboxylate,
4-{[2-(2,3-dihydroxyphenyl)ethyl](dimethyl)azaniumyllbutanoate,
2-{[2-(2,3-dihydroxyphenyl)ethyl](dimethyl)azaniumyl}ethane-1-sulfonate, and
3-[(2,3-dihydroxyphenyl)(dimethyl)azaniumyl]propane-1-sulfonate.

25. The compound as set forth in claim 24 or a salt thereof, which is selected from the group consisting of:
{1-[(2,3-dihydroxyphenyl)methyl]-1-methylpiperidin-1-ium-4-yl}acetate, and
2-{[2-(2,3-dihydroxyphenyl)ethyl](dimethyl)azaniumyl}ethane-1-sulfonate.

26. The compound as set forth in claim 24 or a salt thereof, which is selected from the group consisting of:
4-{[(2,3-dihydroxyphenyl)methyl](dimethyl)azaniumyl}butane-1-sulfonate, and
3-{[(6-fluoro-2,3-dihydroxyphenyl)methyl](dimethyl)azaniumyl}propane-1-sulfonate.
